# EUROPÄISCHE PATENTSCHRIFT

(11) **EP 1 673 352 B1**
(45) Veröffentlichungstag und Bekanntmachung des Hinweises auf die Patenterteilung: **08.04.2009**
(21) Anmeldenummer: 04790500.5
(22) Anmeldetag: 12.10.2004
(51) Int. Cl.: C07D 239/48, C07D 239/47

(54) **SULFOXIMINSUBSTITUIERTE PYRIMIDINE ALS CDK- UND/ODER VEGF-INHITOREN, DEREN HERSTELLUNG UND VERWENDUNG ALS ARZNEIMITTEL**
SULFOXIMINE-SUBSTITUTED PYRIMIDINES FOR USE AS CD AND/OR VEGF-INHIBITORS, THE PRODUCTION THEREOF AND THEIR USE AS DRUGS
PYRIMIDINES SUBSTITUEES SULFOXIMINE EN TANT QU'INHIBITEURS DE CDK ET/OU VEGF, LEUR PRODUCTION ET LEUR UTILISATION COMME MEDICAMENTS

(30) Priorität: 16.10.2003 DE 10349423
(43) Veröffentlichungstag der Anmeldung: 28.06.2006
(73) Patentinhaber: Bayer Schering Pharma Aktiengesellschaft, 13353 Berlin (DE)
(72) Erfinder: LÜCKING, Ulrich, 10245 Berlin (DE); KRÜGER, Martin, 13465 Berlin (DE); JAUTELAT, Rolf, 10439 Berlin (DE); SIEMEISTER, Gerhard, 13503 Berlin (DE)
(86) Internationale Anmeldenummer: PCT/EP2004/011661
(87) Internationale Veröffentlichungsnummer: WO 2005/037800

(56) Entgegenhaltungen:
- WO-A-02/096888
- WO-A-03/076437

## Beschreibung

Die vorliegende Erfindung betrifft sulfoximinsubstituierte Pyrimidinderivate, deren Verfahren zur Herstellung sowie deren Verwendung zur Herstellung von Medikamenten zur Behandlung verschiedener Erkrankungen.

Die Zyklin-abhängigen Kinasen (cyclin-dependent kinase, CDK) sind eine Enzymfamilie, die eine wichtige Rolle bei der Regulation des Zellzyklus spielt und somit ein besonders interessantes Ziel für die Entwicklung kleiner inhibitorischer Moleküle darstellt. Selektive Inhibitoren der CDKs können zur Behandlung von Krebs oder anderen Erkrankungen, die Störungen der Zellproliferation zur Ursache haben, verwendet werden.

Rezeptortyrosinkinasen und deren Liganden, die spezifisch die Funktion von Endothelzellen regulieren, sind in entscheidender Weise an der physiologischen, wie auch der pathogenen Angiogenese beteiligt. Von besonderer Bedeutung ist hier das Vascular Endothelial Growth Factors (VEGF) / VEGF-Rezeptor System. In pathologischen Situationen, die mit einer verstärkten Neovaskularisation einhergehen, wie z.B. Tumorerkrankungen, wurde eine erhöhte Expression von angiogenen Wachstumsfaktoren und ihrer Rezeptoren gefunden. Inhibitoren des VEGF/VEGF-Rezeptorsystems können die Ausbildung eines Blutgefäßsystems im Tumor inhibieren, damit den Tumor von der Sauerstoff- und Nährstoffversorgung abscheiden und somit das Tumorwachstum inhibieren.

Pyrimidine und Analoga sind bereits als Wirkstoffe beschrieben wie beispielsweise die 2-Anilino-Pyrimidine als Fungizide (DE 4029650) oder substituierte Pyrimidinderivate zur Behandlung von neurologischen oder neurodegenerativen Erkrankungen (WO 99/19305). Als CDK-Inhibitoren werden unterschiedlichste Pyrimidinderivate beschrieben, beispielsweise Bis(anilino)-pyrimidinderivate (WO 00/12486), 2-Amino-4-substituierte Pyrimidine (WO 01/ 14375), Purine (WO 99/02162), 5-Cyano-Pyrimidine (WO 02/04429), Anilinopyrimidine (WO 00/12486) und 2-Hydroxy-3-N,N-dimethylaminopropoxy-Pyrimidine (WO 00/39101).

Insbesondere wurden in WO 02/096888 und WO 03/7076437 Pyrimidinderivate offenbart, die inhibitorische Wirkungen bezüglich CDKs aufweisen. Verbindungen, die eine Phenylsulfonamid-Gruppe enthalten sind als Inhibitoren der humanen Carboanhydrasen (insbes. Carboanhydrase-2) bekannt und werden als Diuretica u.a. zur Behandlung von Glaucom eingesetzt. Das Stickstoffatom und die Sauerstoffatome des Sulfonamids binden über Wasserstoffbrücken mit dem Zink²⁺-Ion und der Aminosäure Thr 199 im aktiven Zentrum Carboanhydrase-2 und blockieren dadurch deren enzymatische Funtion (A. Casini, F. Abbate, A. Scozzafava, C.T. Supuran, Bioorganic. Med. Chem L. 2003, 1, 2759.3). Eine Erhöhung der Spezifität der bekannten CDK-Inhibitoren durch Reduktion oder Elimination der inhibitorischen Eigenschaften hinsichtlich der Carboanhydrasen könnte zu einer Verbesserung der pharmakologischen Eigenschaften und einer Veränderung des Nebenwirkungsspektrums führen.

Sulfoximine sind wie beispielsweise sulfonimidoyl- modifizierte Triazole als Fungizide (H. Kawanishi, H. Morimoto, T. Nakano, T. Watanabe, K. Oda, K. Tsujihara, Heterocycles 1998, 49, 181) oder Arylalkylsulfoximine als Herbizide und Pestizide (Shell International Research, Ger. P. 2 129 678) als Wirkstoffe beschrieben.

Die Aufgabe der vorliegenden Erfindung ist es Verbindungen bereitzustellen, die verbesserte pharmazeutische Eigenschaften, insbesondere eine Reduzierung der Carboanhydrase-2-Inhibition, als die bereits bekannten CDK-Inhibitoren aufweisen.

Es wurde nun gefunden, dass Verbindungen der allgemeinen Formel (I) 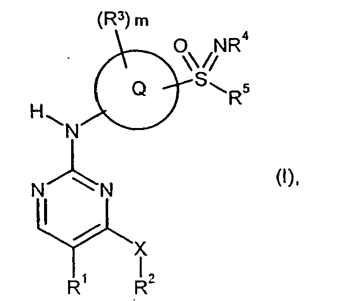 in der
- Q: für die Gruppe 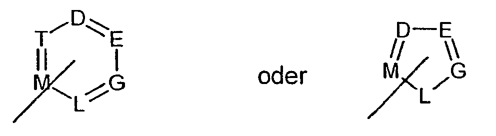
- D, E, G, L, M und T: jeweils unabhängig voneinander für Kohlenstoff, Sauerstoff, Stickstoff oder Schwefel stehen,
- R¹: für Wasserstoff, Halogen, C₁-C₆-Alkyl, CF₃, CN, Nitro oder für die Gruppe -COR⁸ oder -O-C₁-C₆-Alkyl steht,
- R²: für Wasserstoff oder gegebenenfalls ein- oder mehrfach, gleich oder verschieden mit Hydroxy, Halogen, C₁-C₆-Alkoxy, Amino, Cyano, C₁-C₆-Alkyl, -NH-(CH₂)ₙ-C₃-C₁₀-Cycloalkyl, -C₃-C₁₀-Cycloalkyl, C₁- C₆-Hydroxyalkyl, C₂-C₆-Alkenyl, C₂-C₆-Alkinyl, C₁-C₆-Alkoxy-C₁-C₆- Alkyl, C₁-C₆-Alkoxy-C₁-C₆-Alkoxy-C₁-C₆-Alkyl, -NHC₁-C₆-Alkyl, - N(C₁-C₆-Alkyl)₂, C₁-C₆-Alkanoyl, -CONR⁹R¹⁰, -COR⁸ , C₁-C₆- AlkylOAc, Carboxy, Aryl, Heteroaryl, -(CH₂)ₙ-Aryl, -(CH₂)ₙ- Heteroaryl, Phenyl-(CH₂)ₙ-R⁸, -(CH₂)ₙPO₃(R⁸)₂ oder mit der Gruppe -R⁶ oder -NR⁹R¹⁰ substituiertes C₁-C₁₀-Alkyl, C₂-C₁₀-Alkenyl, C₂-C₁₀- Alkinyl, C₃-C₁₀-Cycloalkyl, Aryl oder Heteroaryl steht und das Phenyl, C₃-C₁₀-Cycloalkyl, Aryl, Heteroaryl, -(CH₂)ₙ-Aryl und -(CH₂)ₙ- Heteroaryl selbst gegebenenfalls ein- oder mehrfach, gleich oder verschieden mit Halogen, Hydroxy, C₁-C₆-Alkyl, C₁-C₆-Alkoxy, oder mit der Gruppe -CF₃ oder -OCF₃ substituiert sein kann, und der Ring des C₃-C₁₀-Cycloalkyls und das C₁-C₁₀-Alkyl gegebenenfalls durch ein- oder mehrere Stickstoff, Sauerstoff und/ oder Schwefel-Atome unterbrochen sein kann und / oder durch ein oder mehrere -C(O)- Gruppen im Ring unterbrochen sein kann und / oder gegebenenfalls ein oder mehrere mögliche Doppelbindungen im Ring enthalten sein können,
- X: für Sauerstoff, Schwefel oder für die Gruppe -NH- oder -N(C₁-C₃- Alkyl)- steht,
- oder X und R²: gemeinsam einen C₃-C₁₀ -Cycloalkyl-Ring bilden, der gegebenenfalls ein oder mehrere Heteroatome enthalten kann, und gegebenenfalls ein- oder mehrfach, gleich oder verschieden mit Hydroxy, C₁-C₆-Alkyl, C₁-C₆-Alkoxy, Halogen oder der Gruppe - NR⁹R¹⁰ substituiert sein kann,
- R³: für Hydroxy, Halogen, CF₃, OCF₃ oder für die Gruppe -NR⁹R¹⁰ oder für gegebenenfalls ein- oder mehrfach, gleich oder verschieden mit Halogen, Hydroxy, C₁-C₆-Alkoxy oder der Gruppe -NR⁹R¹⁰ substituiertes C₁-C₆-Alkyl, C₃-C₆-Cycloalkyl oder C₁-C₆-Alkoxy steht,
- m: für 0 - 4 steht,
- R⁴: für Wasserstoff oder für die Gruppe -COR⁸, NO₂, Trimethylsilanyl (TMS), tert.-Butyl-dimethylsilanyl (TBDMS), tert.-Butyl- diphenylsilanyl (TBDPS), Triethylsilanyl (TES) oder -SO₂R⁷ steht oder für gegebenenfalls ein- oder mehrfach, gleich oder verschieden mit Hydroxy, Halogen, C₁-C₆-Alkoxy, C₁-C₆-Alkylthio, Cyano, C₃-C₁₀- Cycloalkyl, C₁-C₆-Hydroxyalkyl, C₂-C₆-Alkenyl, C₂-C₆-Alkinyl, C₁-C₆- Alkoxy-C₁-C₆-Alkyl, C₁-C₆-Alkoxy-C₁-C₆-Alkoxy-C₁-C₆-Alkyl oder mit der Gruppe -CONR⁹R¹⁰, -COR⁸, -CF₃, -OCF₃ oder -NR⁹R¹⁰ substituiertes C₁-C₁₀-Alkyl oder C₃-C₁₀-Cycloalkyl, steht,
- R⁵: für gegebenenfalls ein- oder mehrfach, gleich oder verschieden mit Hydroxy, C₁-C₆-Alkoxy, C₃-C₁₀-Cycloalkyl, Halogen oder der Gruppe -NR⁹R¹⁰ substituiertes C₁-C₁₀-Alkyl, C₂-C₆-Alkenyl, C₂-C₆-Alkinyl oder C₃-C₁₀-Cycloalkyl steht,
- oder R⁴ und R⁵: gemeinsam einen C₅-C₁₀-Cycloalkylring der Gruppe 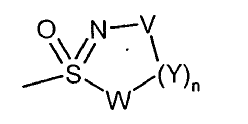 bilden kann, wobei
- V, W und Y: jeweils unabhängig voneinander für gegebenenfalls ein- oder mehrfach, gleich oder verschieden mit Hydroxy, C₁-C₁₀-Alkyl, C₁- C₁₀-Alkoxy oder -NR⁹R¹⁰ substituiertes -CH₂- steht, wobei C₁-C₁₀- Alkyl oder C₁-C₁₀-Alkoxy ebenfalls ein oder mehrfach, gleich oder verschieden mit Hydroxy, -NR⁹R¹⁰ oder C₁-C₁₀-Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
- R⁶: für einen Heteroaryl oder einen C₃-C₁₀-Cycloalkyl-Ring, der gegebenenfalls ein oder mehrere Heteroatome enthalten kann und gegebenenfalls ein- oder mehrfach, gleich oder verschieden mit Hydroxy, C₁-C₆-Alkyl, C₁-C₆-Alkoxy oder Halogen substituiert sein kann, steht,
- R⁷: für gegebenenfalls ein- oder mehrfach, gleich oder verschieden mit Halogen, Hydroxy, C₁-C₆-Alkyl, C₁-C₆-Alkoxy oder mit der Gruppe Trimethylsilanyl (TMS) oder -NR⁹R¹⁰ substituiertes C₁-C₁₀-Alkyl oder Aryl steht,
- R⁸: für Wasserstoff, C₁-C₆-Alkyl , Hydroxy, C₁-C₆-Alkoxy, C₁-C₆- Alkylthio, Benzoxy oder -NR⁹R¹⁰ steht,
- R⁹ und R¹⁰: jeweils unabhängig voneinander für Wasserstoff, C₁-C₆-Alkyl, C₁-C₆- Alkoxy, Hydroxy, Hydroxy-C₁-C₆-Alkyl, Dihydroxy-C₁-C₆-Alkyl, Phenyl, Heteroaryl oder für die Gruppe -(CH₂)ₙNR⁹R¹⁰, -CNHNH₂ oder -NR⁹R¹⁰ stehen,
- oder R⁹ und R¹⁰: gemeinsam einen C₃-C₁₀-Cycloalkyl-Ring bilden, der gegebenenfalls durch ein- oder mehrere Stickstoff, Sauerstoff und/ oder Schwefel- Atome unterbrochen sein kann und/ oder durch ein oder mehrere - C(O)- Gruppen im Ring unterbrochen sein kann und/ oder gegebenenfalls ein oder mehrere mögliche Doppelbindungen im Ring enthalten sein können, steht und
- n: für 1 - 6 steht,
bedeuten, sowie deren Isomeren, Diastereomeren, Enantiomeren und/oder Salze, nicht mehr in der Lage sind, Carboanhydrasen zu inhibieren,
wobei sie gleichzeitig zyklin-abhängige Kinasen und VEGF-Rezeptortyrosinkinasen bereits im nanomolaren Bereich inhibieren und somit die Proliferation der Tumorzellen und/oder die Tumorangiogenese inhibieren können.

Unter Alkyl ist jeweils ein geradkettiger oder verzweigter Alkylrest, wie beispielsweise Methyl, Ethyl, Propyl, Isopropyl, Butyl, Isobutyl, sek. Butyl, tert. Butyl, Pentyl, Isopentyl, Hexyl, Heptyl, Octyl, Nonyl und Decyl, zu verstehen.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, Pentyloxy, Isopentyloxy, Hexyloxy, Heptyloxy, Octyloxy, Nonyloxy, Decyloxy, Undecyloxy oder Dodecyloxy zu verstehen.

Unter Cycloalkyl ist jeweils Cyclopropyl, Cyclobutyl, Cyclopentyl, Cyclohexyl und Cycloheptyl zu verstehen.
Heterocycloalkyl steht für einen 3 - 12 Kohlenstoffatome umfassenden Alkylring, der anstelle des Kohlenstoffes ein oder mehrere, gleich oder verschiedene Heteroatome, wie z. B. Sauerstoff, Schwefel oder Stickstoff enthält.
Als Heterocycloalkyle seien z. B. genannt: Oxiranyl, Oxethanyl, Aziridinyl, Azetidinyl, Tetrahydrofuranyl, Pyrrolidinyl, Dioxolanyl, Imidazolidinyl, Pyrazolidinyl, Dioxanyl, Piperidinyl, Morpholinyl, Dithianyl, Thiomorpholinyl, Piperazinyl, Trithianyl, Chinuclidinyl etc.

Unter den Ringsystemen, bei denen gegebenenfalls ein- oder mehrere mögliche Doppelbindungen im Ring enthalten sein können, sind zum Beispiel Cycloalkenyle wie Cyclopropenyl, Cyclobutenyl, Cyclopentenyl, Cyclohexenyl, Cycloheptenyl zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, Ethinyl, Prop-1-in-1-yl, But-1-in-1-yl, But-2-in-1-yl, But-3-en-1-yl, Allyl.

Der Arylrest hat jeweils 6 - 12 Kohlenstoffatome wie beispielsweise Naphthyl, Biphenyl und insbesondere Phenyl.

Unter Heteroaryl ist ein Heteroarylrest zu verstehen, der jeweils auch benzokondensiert sein kann. Beispielsweise seien als 5-Ringheteroaromaten genannt: Thiophen, Furan, Oxazol, Thiazol, Imidazol, Pyrazol, Triazol, Thia-*4H-*Pyrazol und Benzoderivate davon und als 6-Ring-Heteroaromaten Pyridin, Pyrimidin, Triazin, Chinolin, Isochinolin und deren benzokondensierte Derivate.

Unter Isomeren sind chemische Verbindungen der gleichen Summenformel aber unterschiedlicher chemischer Struktur zu verstehen. Man unterscheidet im allgemeinen Konstitutionsisomere und Stereoisomere.

Konstitutionsisomere besitzen die gleiche Summenformel, unterscheiden sich jedoch durch die Verknüpfungsweise ihrer Atome oder Atomgruppen. Hierzu zählen Funktionelle Isomere, Stellungsisomere, Tautomere oder Valenzisomere.

Stereoisomere haben grundsätzlich die gleiche Struktur (Konstitution) - und damit auch die gleiche Summenformel - unterscheiden sich aber durch die räumliche Anordnung der Atome.

Man unterscheidet im allgemeinen Konfigurationsisomere und Konformationsisomere. Konfigurationsisomere sind Stereoisomere, die sich nur durch Bindungsbruch ineinander überführen lassen. Hierzu zählen Enantiomere, Diastereomere und E / Z (cis / trans) Isomere.
Enantiomere sind Stereoisomere, die sich wie Bild und Spiegelbild zueinander verhalten und keine Symmetrieebene aufweisen. Alle Stereoisomere, die keine Enantiomere sind, bezeichnet man als Diastereomere. Ein Spezialfall sind E / Z (cis / trans) Isomere an Doppelbindungen.
Konformationsisomere sind Stereoisomere, die sich durch die Drehung von Einfachbindungen ineinander überführen lassen.

Zur Abgrenzung der Isomerie-Arten voneinander siehe auch die IUPAC Regeln Sektion E (Pure Appl. Chem. 45, 11-30, 1976).

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Besonders wirksam sind solche Verbindungen der allgemeinen Formel (I) in der
- Q: für Aryl steht,
- R¹: für Wasserstoff, Halogen, C₁-C₆-Alkyl, CF₃, CN, Nitro oder für die Gruppe -COR⁸ oder -O-C₁-C₆-Alkyl steht,
- R²: für Wasserstoff oder gegebenenfalls ein- oder mehrfach, gleich oder verschieden mit Hydroxy, Halogen, C₁-C₆-Alkoxy, Amino, Cyano, C₁-C₆-Alkyl, -NH-(CH₂)ₙ-C₃-C₁₀-Cycloalkyl, -C₃-C₁₀-Cycloalkyl, C₁- C₆-Hydroxyalkyl, C₂-C₆-Alkenyl, C₂-C₆-Alkinyl, C₁-C₆-Alkoxy-C₁-C₆- Alkyl, C₁-C₆-Alkoxy-C₁-C₆-Alkoxy-C₁-C₆-Alkyl, -NHC₁-C₆-Alkyl, - N(C₁-C₆-Alkyl)₂, C₁-C₆-Alkanoyl, -CONR⁹R¹⁰, -COR⁸ , C₁-C₆- AlkylOAc, Carboxy, Aryl, Heteroaryl, -(CH₂)ₙ-Aryl, -(CH₂)ₙ- Heteroaryl, Phenyl-(CH₂)ₙ-R⁸, -(CH₂)ₙPO₃(R⁸)₂ oder mit der Gruppe -R⁶ oder -NR⁹R¹⁰ substituiertes C₁-C₁₀-Alkyl, C₂-C₁₀-Alkenyl, C₂-C₁₀- Alkinyl, C₃-C₁₀-Cycloalkyl, Aryl oder Heteroaryl steht und das Phenyl, C₃-C₁₀-Cycloalkyl, Aryl, Heteroaryl, -(CH₂)ₙ-Aryl und -(CH₂)ₙ- Heteroaryl selbst gegebenenfalls ein- oder mehrfach, gleich oder verschieden mit Halogen, Hydroxy, C₁-C₆-Alkyl, C₁-C₆-Alkoxy, oder mit der Gruppe -CF₃ oder -OCF₃ substituiert sein kann, und der Ring des C₃-C₁₀-Cycloalkyls und das C₁-C₁₀-Alkyl gegebenenfalls durch ein- oder mehrere Stickstoff, Sauerstoff und/ oder Schwefel-Atome unterbrochen sein kann und / oder durch ein oder mehrere -C(O)- Gruppen im Ring unterbrochen sein kann und / oder gegebenenfalls ein oder mehrere mögliche Doppelbindungen im Ring enthalten sein können,
- X: für Sauerstoff, Schwefel oder für die Gruppe -NH-, -N(C₁-C₃-Alkyl)- steht,
- oder X und R²: gemeinsam einen C₃-C₁₀ -Cycloalkyl-Ring bilden, der gegebenenfalls ein oder mehrere Heteroatome enthalten kann, und gegebenenfalls ein- oder mehrfach, gleich oder verschieden mit Hydroxy, C₁-C₆-Alkyl, C₁-C₆-Alkoxy, Halogen oder der Gruppe - NR⁹R¹⁰ substituiert sein kann,
- R³: für Hydroxy, Halogen, CF₃, OCF₃ oder für die Gruppe -NR⁹R¹⁰ oder für gegebenenfalls ein- oder mehrfach, gleich oder verschieden mit Halogen, Hydroxy, C₁-C₆-Alkoxy oder der Gruppe -NR⁹R¹⁰ substituiertes C₁-C₆-Alkyl, C₃-C₆-Cycloalkyl oder C₁-C₆-Alkoxy steht,
- m: für 0 - 4 steht,
- R⁴: für Wasserstoff oder für die Gruppe -COR⁸, NO₂, Trimethylsilanyl (TMS), tert.-Butyl-dimethylsilanyl (TBDMS), tert.-Butyl- diphenylsilanyl (TBDPS), Triethylsilanyl (TES) oder -SO₂R⁷ steht oder für gegebenenfalls ein- oder mehrfach, gleich oder verschieden mit Hydroxy, Halogen, C₁-C₆-Alkoxy, C₁-C₆-Alkylthio, Cyano, C₃-C₁₀- Cycloalkyl, C₁-C₆-Hydroxyalkyl, C₂-C₆-Alkenyl, C₂-C₆-Alkinyl, C₁-C₆- Alkoxy-C₁-C₆-Alkyl, C₁-C₆-Alkoxy-C₁-C₆-Alkoxy-C₁-C₆-Alkyl oder mit der Gruppe -CONR⁹R¹⁰, -COR⁸, -CF₃, -OCF₃ oder -NR⁹R¹⁰ substituiertes C₁-C₁₀-Alkyl, C₃-C₁₀-Cycloalkyl, steht,
- R⁵: für gegebenenfalls ein- oder mehrfach, gleich oder verschieden mit Hydroxy, C₁-C₆-Alkoxy, C₃-C₁₀-Cycloalkyl, Halogen oder der Gruppe -NR⁹R¹⁰ substituiertes C₁-C₁₀-Alkyl, C₂-C₆-Alkenyl, C₂-C₆-Alkinyl oder C₃-C₁₀-Cycloalkyl steht,
- oder R⁴ und R⁵: gemeinsam einen C₅-C₁₀-Cycloalkylring der Gruppe 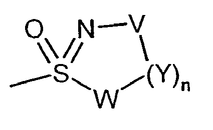 bilden kann, wobei
- V, W und Y: jeweils unabhängig voneinander für gegebenenfalls ein- oder mehrfach, gleich oder verschieden mit Hydroxy, C₁-C₁₀-Alkyl, C₁- C₁₀-Alkoxy oder -NR⁹R¹⁰ substituiertes -CH₂- steht, wobei C₁-C₁₀- Alkyl oder C₁-C₁₀-Alkoxy ebenfalls ein oder mehrfach, gleich oder verschieden mit Hydroxy, -NR⁹R¹⁰ oder C₁-C₁₀-Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
- R⁶: für einen Heteroaryl oder einen C₃-C₁₀-Cycloalkyl-Ring, der gegebenenfalls ein oder mehrere Heteroatome enthalten kann und gegebenenfalls ein- oder mehrfach, gleich oder verschieden mit Hydroxy, C₁-C₆-Alkyl, C₁-C₆-Alkoxy oder Halogen substituiert sein kann, steht,
- R⁷: für gegebenenfalls ein- oder mehrfach, gleich oder verschieden mit Halogen, Hydroxy, C₁-C₆-Alkyl, C₁-C₆-Alkoxy oder mit der Gruppe Trimethylsilanyl (TMS) oder -NR⁹R¹⁰ substituiertes C₁-C₁₀-Alkyl oder Aryl steht,
- R⁸: für Wasserstoff, C₁-C₆-Alkyl , Hydroxy, C₁-C₆-Alkoxy, C₁-C₆- Alkylthio, Benzoxy oder -NR⁹R¹⁰ steht,
- R⁹ und R¹⁰: jeweils unabhängig voneinander für Wasserstoff, C₁-C₆-Alkyl, C₁-C₆- Alkoxy, Hydroxy, Hydroxy-C₁-C₆-Alkyl, Dihydroxy-C₁-C₆-Alkyl, Phenyl, Heteroaryl oder für die Gruppe -(CH₂)ₙNR⁹R¹⁰, -CNHNH₂ oder -NR⁹R¹⁰ stehen,
- oder R⁹ und R¹⁰: gemeinsam einen C₃-C₁₀-Cycloalkyl-Ring bilden, der gegebenenfalls durch ein- oder mehrere Stickstoff, Sauerstoff und/ oder Schwefel- Atome unterbrochen sein kann und/ oder durch ein oder mehrere - C(O)- Gruppen im Ring unterbrochen sein kann und/ oder gegebenenfalls ein oder mehrere mögliche Doppelbindungen im Ring enthalten sein können, steht und
- n: für 1 - 6 steht,
bedeuten, sowie deren Isomeren, Diastereomeren, Enantiomeren und/oder Salze.

Besonders wirksam sind weiterhin solche Verbindungen der allgemeinen Formel (I) in der
- Q: für Phenyl steht,
- R¹: für Wasserstoff, Halogen, C₁-C₆-Alkyl, CF₃, CN, Nitro oder für die Gruppe -COR⁸ oder -O-C₁-C₆-Alkyl steht,
- R²: für Wasserstoff oder gegebenenfalls ein- oder mehrfach, gleich oder verschieden mit Hydroxy, Halogen, C₁-C₆-Alkoxy, Amino, Cyano, C₁-C₆-Alkyl, -NH-(CH₂)ₙ-C₃-C₁₀-Cycloalkyl, -C₃-C₁₀-Cycloalkyl, C₁- C₆-Hydroxyalkyl, C₂-C₆-Alkenyl, C₂-C₆-Alkinyl, C₁-C₆-Alkoxy-C₁-C₆- Alkyl, C₁-C₆-Alkoxy-C₁-C₆-Alkoxy-C₁-C₆-Alkyl, -NHC₁-C₆-Alkyl, - N(C₁-C₆-Alkyl)₂, C₁-C₆-Alkanoyl, -CONR⁹R¹⁰, -COR⁸ , C₁-C₆- AlkylOAc, Carboxy, Aryl, Heteroaryl, -(CH₂)ₙ-Aryl, -(CH₂)ₙ- Heteroaryl, Phenyl-(CH₂)ₙ-R⁸, -(CH₂)ₙPO₃(R⁸)₂ oder mit der Gruppe -R⁶ oder -NR⁹R¹⁰ substituiertes C₁-C₁₀-Alkyl, C₂-C₁₀-Alkenyl, C₂-C₁₀- Alkinyl, C₃-C₁₀-Cycloalkyl, Aryl oder Heteroaryl steht und das Phenyl, C₃-C₁₀-Cycloalkyl, Aryl, Heteroaryl, -(CH₂)ₙ-Aryl und -(CH₂)ₙ- Heteroaryl selbst gegebenenfalls ein- oder mehrfach, gleich oder verschieden mit Halogen, Hydroxy, C₁-C₆-Alkyl, C₁-C₆-Alkoxy, oder mit der Gruppe -CF₃ oder -OCF₃ substituiert sein kann, und der Ring des C₃-C₁₀-Cycloalkyls und das C₁-C₁₀-Alkyl gegebenenfalls durch ein- oder mehrere Stickstoff, Sauerstoff und/ oder Schwefel-Atome unterbrochen sein kann und / oder durch ein oder mehrere -C(O)- Gruppen im Ring unterbrochen sein kann und / oder gegebenenfalls ein oder mehrere mögliche Doppelbindungen im Ring enthalten sein können,
- X: für Sauerstoff, Schwefel oder für die Gruppe -NH-, -N(C₁-C₃-Alkyl)- steht,
- oder X und R²: gemeinsam einen C₃-C₁₀ -Cycloalkyl-Ring bilden, der gegebenenfalls ein oder mehrere Heteroatome enthalten kann, und gegebenenfalls ein- oder mehrfach mit Hydroxy, C₁-C₆-Alkyl, C₁-C₆- Alkoxy, Halogen oder der Gruppe -NR⁹R¹⁰ substituiert sein kann,
- R³: für Hydroxy, Halogen, CF₃, OCF₃ oder für die Gruppe -NR⁹R¹⁰ oder für gegebenenfalls ein- oder mehrfach, gleich oder verschieden mit Halogen, Hydroxy, C₁-C₆-Alkoxy oder der Gruppe -NR⁹R¹⁰ substituiertes C₁-C₆-Alkyl, C₃-C₆-Cycloalkyl oder C₁-C₆-Alkoxy steht,
- m: für 0 - 2 steht,
- R⁴: für Wasserstoff oder für die Gruppe -COR⁸, NO₂, Trimethylsilanyl (TMS), tert.-Butyl-dimethylsilanyl (TBDMS), tert.-Butyl- diphenylsilanyl (TBDPS), Triethylsilanyl (TES) oder -SO₂R⁷ steht oder für gegebenenfalls ein- oder mehrfach, gleich oder verschieden mit Hydroxy, Halogen, C₁-C₆-Alkoxy, C₁-C₆-Alkylthio, Cyano, C₃-C₁₀- Cycloalkyl, C₁-C₆-Hydroxyalkyl, C₂-C₆-Alkenyl, C₂-C₆-Alkinyl, C₁-C₆- Alkoxy-C₁-C₆-Alkyl, C₁-C₆-Alkoxy-C₁-C₆-Alkoxy-C₁-C₆-Alkyl oder mit der.Gruppe -CONR⁹R¹⁰, -COR⁸, -CF₃, -OCF₃ oder -NR⁹R¹⁰ substituiertes C₁-C₁₀-Alkyl, C₃-C₁₀-Cycloalkyl, steht,
- R⁵: für gegebenenfalls ein- oder mehrfach, gleich oder verschieden mit Hydroxy, C₁-C₆-Alkoxy, C₃-C₁₀-Cycloalkyl, Halogen oder der Gruppe -NR⁹R¹⁰ substituiertes C₁-C₁₀-Alkyl, C₂-C₆-Alkenyl, C₂-C₆-Alkinyl oder C₃-C₁₀-Cycloalkyl steht,
- oder R⁴ und R⁵: gemeinsam einen C₅-C₁₀-Cycloalkylring der Gruppe 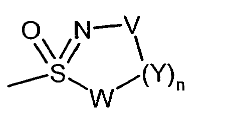 bilden kann, wobei
- V, W und Y: jeweils unabhängig voneinander für gegebenenfalls ein- oder mehrfach, gleich oder verschieden mit Hydroxy, C₁-C₁₀-Alkyl, C₁- C₁₀-Alkoxy oder -NR⁹R¹⁰ substituiertes -CH₂- steht, wobei C₁-C₁₀- Alkyl oder C₁-C₁₀-Alkoxy ebenfalls ein oder mehrfach, gleich oder verschieden mit Hydroxy, -NR⁹R¹⁰ oder C₁-C₁₀-Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
- R⁶: für einen Heteroaryl oder einen C₃-C₁₀-Cycloalkyl-Ring, der gegebenenfalls ein oder mehrere Heteroatome enthalten kann und gegebenenfalls ein- oder mehrfach, gleich oder verschieden mit Hydroxy, C₁-C₆-Alkyl, C₁-C₆-Alkoxy oder Halogen substituiert sein kann, steht,
- R⁷: für gegebenenfalls ein- oder mehrfach, gleich oder verschieden mit Halogen, Hydroxy, C₁-C₆-Alkyl, C₁-C₆-Alkoxy oder mit der Gruppe Trimethylsilanyl (TMS) oder -NR⁹R¹⁰ substituiertes C₁-C₁₀-Alkyl oder Aryl steht,
- R⁸: für Wasserstoff, C₁-C₆-Alkyl , Hydroxy, C₁-C₆-Alkoxy, C₁-C₆- Alkylthio, Benzoxy oder -NR⁹R¹⁰ steht,
- R⁹ und R¹⁰: jeweils unabhängig voneinander für Wasserstoff, C₁-C₆-Alkyl, C₁-C₆- Alkoxy, Hydroxy, Hydroxy-C₁-C₆-Alkyl, Dihydroxy-C₁-C₆-Alkyl, Phenyl, Heteroaryl oder für die Gruppe -(CH₂)ₙNR⁹R¹⁰, -CNHNH₂ oder -NR⁹R¹⁰ stehen,
- oder R⁹ und R¹⁰: gemeinsam einen C₃-C₁₀-Cycloalkyl-Ring bilden, der gegebenenfalls durch ein- oder mehrere Stickstoff, Sauerstoff und/ oder Schwefel- Atome unterbrochen sein kann und/ oder durch ein oder mehrere - C(O)- Gruppen im Ring unterbrochen sein kann und/ oder gegebenenfalls ein oder mehrere mögliche Doppelbindungen im Ring enthalten sein können, steht und
- n: für 1 - 6 steht,
bedeuten, sowie deren Isomeren, Diastereomeren, Enantiomeren und/oder Salze.

Insbesondere sind solche Verbindungen der allgemeinen Formel (I) wirksam, in der
- Q: für Phenyl steht,
- R¹: für Wasserstoff, Halogen, CN, NO₂ oder CF₃ steht,
- R²: für gegebenenfalls ein- oder mehrfach, gleich oder verschieden mit Hydroxy, Halogen, C₁-C₆-Alkyl, C₁-C₆-Alkoxy, C₂-C₆-Alkinyl oder mit der Gruppe -COR⁸ substituiertes C₁-C₁₀-Alkyl, C₂-C₁₀-Alkinyl, Aryl oder Heteroaryl steht,
- X: für Sauerstoff, Schwefel oder für die Gruppe -NH- steht,
- R³: für Halogen, Hydroxy oder gegebenenfalls ein- oder mehrfach mit Halogen oder Hydroxy substituiertes C₁-C₆-Alkyl oder C₁-C₆-Alkoxy steht,
- m: für 0 - 2 steht,
- R⁴: für Wasserstoff oder für die Gruppe NO₂, -CO-R⁸, -SO₂R⁷ oder für gegebenenfalls ein- oder mehrfach, gleich oder verschieden mit Halogen oder Hydroxy substituiertes C₁-C₁₀-Alkyl steht,
- R⁵: für gegebenenfalls ein- oder mehrfach, gleich oder verschieden mit Hydroxy oder C₃-C₁₀-Cycloalkyl substituiertes C₁-C₁₀-Alkyl oder C₃- C₁₀-Cycloalkyl steht,
- oder R⁴ und R⁵: gemeinsam einen C₅-C₁₀-Cycloalkylring der Gruppe 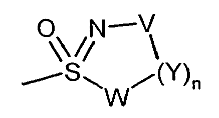 bilden kann, wobei
- V, W und Y: jeweils unabhängig voneinander für gegebenenfalls ein- oder mehrfach, gleich oder verschieden mit Hydroxy, C₁-C₁₀-Alkyl, C₁- C₁₀-Alkoxy oder -NR⁹R¹⁰ substituiertes -CH₂- steht, wobei C₁-C₁₀- Alkyl oder C₁-C₁₀-Alkoxy ebenfalls ein oder mehrfach, gleich oder verschieden mit Hydroxy, -NR⁹R¹⁰ oder C₁-C₁₀-Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
- R⁷: für gegebenenfalls ein- oder mehrfach, gleich oder verschieden mit der Gruppe Trimethylsilanyl (TMS) substituiertes C₁-C₁₀-Alkyl oder steht,
- R⁸: für Wasserstoff, C₁-C₆-Alkyl , C₁-C₆-Alkoxy oder C₃-C₆-Cycloalkyl, das gegebenenfalls ein- oder mehrfach mit C₁-C₆-Alkyl substituiert sein kann, steht,
- n: für 1 steht,
bedeuten, sowie deren Isomeren, Diastereomeren, Enantiomeren und/oder Salze.

Besonders wirksam sind weiterhin solche Verbindungen der allgemeinen Formel (I), in der
- Q: für Phenyl steht,
- R¹: für Wasserstoff oder Halogen steht,
- R²: für gegebenenfalls ein- oder mehrfach, gleich oder verschieden mit Hydroxy, Halogen, C₁-C₆-Alkyl, C₁-C₆-Alkoxy, C₂-C₆-Alkinyl oder mit der Gruppe -COR⁸ substituiertes C₁-C₁₀-Alkyl, C₂-C₁₀-Alkinyl oder Aryl steht,
- X: für Sauerstoff, Schwefel oder für die Gruppe -NH- steht,
- R³: für Halogen oder gegebenenfalls ein- oder mehrfach mit Halogen substituiertes C₁-C₆-Alkyl oder C₁-C₆-Alkoxy steht,
- m: für 0 - 2 steht,
- R⁴: für Wasserstoff oder für die Gruppe NO₂, -CO-R⁸, -SO₂R⁷ oder für C₁-C₁₀-Alkyl steht,
- R⁵: für gegebenenfalls ein- oder mehrfach, gleich oder verschieden mit Hydroxy oder C₃-C₁₀-Cycloalkyl substituiertes C₁-C₁₀-Alkyl oder C₃- C₁₀-Cycloalkyl steht,
- R⁷: für gegebenenfalls ein- oder mehrfach, gleich oder verschieden mit der Gruppe Trimethylsilanyl (TMS) substituiertes C₁-C₁₀-Alkyl oder steht,
- R⁸: für Wasserstoff, C₁-C₆-Alkyl , C₁-C₆-Alkoxy oder C₃-C₆-Cycloalkyl, das gegebenenfalls ein- oder mehrfach mit C₁-C₆-Alkyl substituiert sein kann, steht,
bedeuten, sowie deren Isomeren, Diastereomeren, Enantiomeren und/oder Salze.

Besonders ausgewählte Verbindungen der allgemeinen Formel (I) sind weiterhin solche, in denen
- Q: für Phenyl steht,
- R¹: für Wasserstoff oder Halogen steht,
- R²: für gegebenenfalls ein- oder mehrfach, gleich oder verschieden mit Hydroxy, Halogen, Methyl, Methoxy, Ethinyl oder mit der Gruppe - COH oder -COCH₃ substituiertes C₁-C₁₀-Alkyl, C₂-C₁₀-Alkinyl oder Aryl steht,
- X: für Sauerstoff, Schwefel oder für die Gruppe -NH- steht,
- R³: für Halogen, Methyl, Methoxy oder -CF₃ steht,
- m: für 0 - 2 steht,
- R⁴: für Wasserstoff, Methyl oder für die Gruppe NO₂,-COOC₂H₅ oder - SO₂-C₂H₄-Si(CH₃)₃ steht,
- R⁵: für Methyl, Ethyl, Cyclopropyl, Cyclopentyl, -(CH₂)-Cyclopropyl oder Hydroxyethyl steht,
bedeuten, sowie deren Isomeren, Diastereomeren, Enantiomeren und/oder Salze.

Die erfindungsgemäßen Verbindungen inhibieren im wesentlichen Zyklin-abhängige Kinasen, worauf auch deren Wirkung zum Beispiel gegen Krebs, wie solide Tumoren und Leukämie, Autoimmunerkrankungen wie Psoriasis, Alopezie, und Multiple Sklerose, Chemotherapeutika-induzierte Alopezie und Mukositis, kardiovaskuläre Erkrankungen, wie Stenosen, Arteriosklerosen und Restenosen, infektiöse Erkrankungen, wie z. B. durch unizelluläre Parasiten, wie Trypanosoma, Toxoplasma oder Plasmodium, oder durch Pilze hervorgerufen, nephrologische Erkrankungen, wie z. B. Glomerulonephritis, chronische neurodegenerative Erkrankungen, wie Huntington's Erkrankung, amyotrophe Lateralsklerose, Parkinsonsche Erkrankung, AIDS Dementia und Alzheimer'sche Erkrankung, akute neurodegenerative Erkrankungen, wie Ischämien des Gehirns und Neurotraumata, virale Infektionen, wie z. B. Cytomegalus-Infektionen, Herpes, Hepatitis B und C, und HIV Erkrankungen basiert.

Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinanderfolgende Phasen eingeteilt: die G1 Phase repräsentiert die Zeit vor der DNA-Replikation, in der die Zelle wächst und für externe Stimuli empfänglich ist. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen.

Die Zyklin-abhängigen Kinasen (CDKs), eine Familie von Serin/Threonin-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispielsweise CDK4(6)/CycD, CDK2/CycE, CDK2/CycA, CDK1/CycA und CDK1/CycB. Einige Mitglieder der CDK-Enzymfamilie haben eine regulatorische Funktion indem sie die Aktivität der vorgenannten Zellzyklus-CDKs beeinflussen, während anderen Mitgliedern der CDK-Enzymfamlie noch keine bestimmte Funktion zugeordnet werden konnte. Eine von diesen, CDK5, zeichnet sich dadurch aus, dass sie eine atypische, von den Zyklinen abweichende, regulatorische Untereinheit besitzt (p35), und ihre Aktivität im Gehirn am höchsten ist.

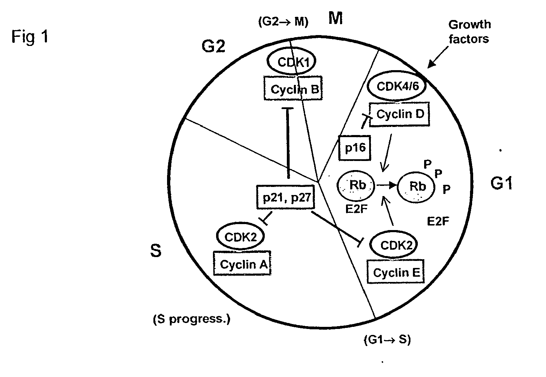

Der Eintritt in den Zellzyklus und das Durchlaufen des "Restriction Point", der die Unabhängigkeit einer Zelle von weiteren Wachstumssignalen für den Abschluss der begonnenen Zellteilung markiert, werden durch die Aktivität der CDK4(6)/CycD und CDK2/CycE Komplexe kontrolliert. Das wesentliche Substrat dieser CDK-Komplexe ist das Retinoblastoma-Protein (Rb), das Produkt des Retinoblastoma Tumorsuppressor Gens. Rb ist ein transkriptionelles Ko-Repressor Protein. Neben anderen noch weitgehend unverstandenen Mechanismen, bindet und inaktiviert Rb Transkriptionsfaktoren vom E2F-Typ, und bildet transkriptionelle Repressorkomplexe mit Histon-Deacetylasen (HDAC) (Zhang H.S. et al. (2000). Exit from G1 and S phase of the cell cycle is regulated by repressor complexes containing HDAC-Rb-hSWI/SNF and RbhSWI/SNF. Cell 101, 79-89). Durch die Phosphorylierung des Rb durch CDKs werden gebundene E2F Transkriptionsfaktoren freigesetzt und führen zu transkriptioneller Aktivierung von Genen, deren Produkte für die DNA Synthese und die Progression durch die S-Phase benötigt werden. Zusätzlich bewirkt die Rb-Phosphorylierung die Auflösung der Rb-HDAC Komplexe, wodurch weitere Gene aktiviert werden. Die Phosphorylierung von Rb durch CDK's ist mit dem Überschreiten des "Restriction Point" gleichzusetzen. Für die Progression durch die S-Phase und deren Abschluss ist die Aktivität der CDK2/CycE und CDK2/CycA Komplexe notwendig, z. B. wird die Aktivität der Transkriptionsfaktoren vom E2F-Typ mittels Phosphorylierung durch CDK2/CycA abgeschaltet sobald die Zellen in die S-Phase eingetreten sind. Nach vollständiger Replikation der DNA steuert die CDK1 im Komplex mit CycA oder CycB den Eintritt und das Durchlaufen der Phasen G2 und M (Abb. 1).

Entsprechend der außerordentlichen Bedeutung des Zellteilungszyklus ist das Durchlaufen des Zyklus streng reguliert und kontrolliert. Die Enzyme, die für die Progression durch den Zyklus notwendig sind, müssen zu dem richtigen Zeitpunkt aktiviert werden, und auch wieder abgeschaltet werden sobald die entsprechende Phase durchlaufen ist. Entsprechende Kontrollpunkte ("Checkpoints") arretieren die Progression durch den Zellzyklus falls DNA-Schäden detektiert werden, oder die DNA-Replikation, oder der Aufbau des Spindelapparates noch nicht beendet ist.
Die Aktivität der CDKs wird durch verschiedene Mechanismen, wie Synthese und Degradation der Zykline, Komplexierung der CDKs mit den entsprechenden Zyklinen, Phosphorylierung und Dephosphorylierung regulatorischer Threonin- und Tyrosin-Reste, und die Bindung natürlicher inhibitorischer Proteine, direkt kontrolliert. Während die Proteinmenge der CDKs in einer proliferierenden Zelle relativ konstant ist, oszilliert die Menge der einzelnen Zykline mit dem Durchlaufen des Zyklus. So wird zum Beispiel die Expression von CycD während der frühen G1 Phase durch Wachstumsfaktoren stimuliert, und die Expression von CycE wird nach Überschreiten des "Restriction Point" durch die Aktivierung der Transkriptionsfaktoren vom E2F-Typ induziert. Die Zykline selbst werden durch Ubiquitin-vermittelte Proteolyse abgebaut. Aktivierende und inaktivierende Phosphorylierungen regulieren die Aktivität der CDK's, zum Beispiel phosphorylieren CDK-aktivierende Kinasen (CAKs) Thr160/161 der CDK1, wohingegen die Familie der Wee1/Myt1 Kinasen CDK1 durch Phosphorylierung von Thr14 und Tyr15 inaktivieren. Diese inaktivierenden Phosphorylierungen können durch cdc25 Phosphatasen wieder aufgehoben werden. Sehr bedeutsam ist die Regulation der Aktivität der CDK/Cyc-Komplexe durch zwei Familien natürlicher CDK Inhibitorproteine (CKIs), den Proteinprodukten der p21 Genfamilie (p21, p27, p57) und der p16 Genfamilie (p15, p16, p18, p19). Mitglieder der p21 Familie binden an Zyklin-Komplexe der CDKs 1,2,4,6, inhibieren aber nur Komplexe die CDK1 oder CDK2 enthalten. Mitglieder der p16 Familie sind spezifische Inhibitoren der CDK4- und CDK6-Komplexe.

Oberhalb dieser Komplexen direkten Regulation der Aktivität der CDKs liegt die Ebene der Kontrollpunkt-Regulation. Kontrollpunkte erlauben der Zelle das geordnete Ablaufen der einzelnen Phasen während des Zellzyklusses zu verfolgen. Die wichtigsten Kontrollpunkte liegen am Übergang von G1 nach S und von G2 nach M. Der G1-Kontrollpunkt stellt sicher, dass die Zelle keine DNA-Synthese beginnt falls sie nicht entsprechend ernährt ist, mit anderen Zellen oder dem Substrat korrekt interagiert, und ihre DNA intakt ist. Der G2/M Kontrollpunkt stellt die vollständige Replikation der DNA und den Aufbau der mitotischen Spindel sicher, bevor die Zelle in die Mitose eintritt. Der G1 Kontrollpunkt wird von dem Genprodukt des p53 Tumorsuppressorgens aktiviert. p53 wird nach Detektion von Veränderungen im Metabolismus oder der genomischen Integrität der Zelle aktiviert und kann entweder einen Stopp der Zellzyklusprogression oder Apoptose auslösen. Dabei spielt die transkriptionelle Aktivierung der Expression des CDK Inhibitorproteins p21 durch p53 eine entscheidende Rolle. Ein zweiter Zweig des G1 Kontrollpunktes umfasst die Aktivierung der ATM und Chk1 Kinasen nach DNA-Schädigung durch UV-Licht oder ionisierende Strahlung und schließlich die Phosphorylierung und den nachfolgenden proteolytischen Abbau der cdc25A Phosphatase (Mailand N. et al. (2000). Rapid destruction of human cdc25A in response to DNA damage. Science 288, 1425-1429). Daraus resultiert eine Arretierung des Zellzykluses, da die inhibitorische Phosphorylierung der CDKs nicht entfernt wird. Nach Aktivierung des G2/M Kontrollpunktes durch Schädigung der DNA sind beide Mechanismen in ähnlicher Weise daran beteiligt, die Progression durch den Zellzyklus zu stoppen.

Der Verlust der Regulation des Zellzyklusses und der Verlust der Funktion der Kontrollpunkte sind Charakteristika von Tumorzellen. Der CDK-Rb-Signalweg ist in über 90% humaner Tumorzellen von Mutationen betroffen. Diese Mutationen, die schließlich zur inaktivierenden Phosphorylierung des RB führen, schließen die Überexpression von D- und E-Zyklinen durch Genamplifikation oder chromosomale Translokationen, inaktivierende Mutationen oder Deletionen von CDK-Inhibitoren des p16-Typs, sowie erhöhten (p27) oder verminderten (CycD) Proteinabbau ein. Die zweite Gruppe von Genen, die durch Mutationen in Tumorzellen getroffen sind, kodiert für Komponenten der Kontrollpunkte. So ist p53, das essentiell für die G1 und G2/M Kontrollpunkte ist, das am häufigsten mutierte Gen in humanen Tumoren (ca. 50%). In Tumorzellen, die p53 ohne Mutation exprimieren, wird es häufig aufgrund einer stark erhöhten Proteindegradation inaktiviert. In ähnlicher Weise sind die Gene anderer für die Funktion der Kontrollpunkte notwendiger Proteine von Mutationen betroffen, zum Beispiel ATM (inaktivierende Mutationen) oder cdc25 Phosphatasen (Überexpression).

Überzeugende experimentelle Daten deuten darauf hin, dass CDK2/Cyc-Komplexe eine entscheidende Position während der Zellzyklusprogression einnehmen: (1) Sowohl dominant-negative Formen der CDK2, wie die transkriptionelle Repression der CDK2 Expression durch anti-sense Oligonukleotide bewirken einen Stopp der Zellzyklusprogression. (2) Die Inaktivierung des CycA Gens in Mäusen ist letal. (3) Die Störung der Funktion des CDK2/CycA Komplexes in Zellen mittels zell-permeabler Peptide führte zur Tumorzell-selektiven Apoptose (Chen Y.N.P. et al. (1999). Selective killing of transformed cells by cyclin/cyclin-dependent kinase 2 antagonists. Proc. Natl. Acad. Sci. USA 96, 4325-4329).

Veränderungen der Zellzykluskontrolle spielen nicht nur bei Krebserkrankungen ein Rolle. Der Zellzyklus wird durch eine Reihe von Viren, sowohl durch transformierende, wie durch nicht-transformierende, aktiviert um die Vermehrung der Viren in der Wirtszelle zu ermöglichen. Der fälschliche Eintritt in den Zellzyklus von normalerweise post-mitotischen Zellen wird mit verschiedenen neurodegenerativen Erkrankungen in Zusammenhang gebracht. Die Mechanismen der Zellzyklusregulation, ihrer Veränderungen in Krankheiten und eine Vielzahl von Ansätzen zur Entwicklung von Inhibitoren der Zellzyklusprogression und speziell der CDKs wurden bereits in mehreren Publikationen ausführlich zusammenfassend beschrieben (Sielecki T.M. et al. (2000). Cyclin-dependent kinase inhibitors: useful targets in cell cycle regulation. J. Med. Chem. 43, 1-18; Fry D.W. & Garrett M.D. (2000). Inhibitors of cyclin-dependent kinases as therapeutic agents for the treatment of cancer. Curr. Opin. Oncol. Endo. Metab. Invest. Drugs 2, 40-59; Rosiania G.R. & Chang Y.T. (2000). Targeting hyperproliferative disorders with cyclin dependent kinase inhibitors. Exp. Opin. Ther. Patents 10, 215-230; Meijer L. et al. (1999). Properties and potential applications of chemical inhibitors of cyclin-dependent kinases. Pharmacol. Ther. 82, 279-284; Senderowicz A.M. & Sausville E.A. (2000). Preclinical and clinical development of cyclin-dependent kinase modulators. J. Natl. Cancer Inst. 92, 376-387).

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg,. vorzugsweise 50-200 mg, wobei die Dosis als einmal zu verabreichende Einzeldosis oder unterteilt in 2 oder mehreren Tagesdosen gegeben werden kann.

Erfindungsgemäße Verbindungen der allgeimeinen Formel I inhibieren zum anderen auch Rezeptortyrosinkinasen und deren Liganden, die spezifisch die Funktion von Endothelzellen regulieren, inhibieren. Rezeptortyrosinkinasen und deren Liganden, die spezifisch die Funktion von Endothelzellen regulieren, sind in entscheidender Weise an der physiologischen, wie auch der pathogenen Angiogenese beteiligt. Von besonderer Bedeutung ist hier das VEGFNEGF-Rezeptor System. In pathologischen Situationen die mit einer verstärkten Neovaskularisation einhergehen wurde eine erhöhte Expression von angiogenen Wachstumsfaktoren und ihrer Rezeptoren gefunden. So exprimieren die meisten soliden Tumoren große Mengen an VEGF, und die Expression der VEGF-Rezeptoren ist vorzugsweise in den Endothelzellen, die in der Nähe der Tumoren liegen oder durch diese hindurchführen, deutlich erhöht (Plate et al., Cancer Res. 53, 5822-5827, 1993). Die Inaktivierung des VEGF/VEGF-Rezeptorsystems durch VEGF-neutralisierende Antikörper (Kim et al., Nature 362, 841-844, 1993), retrovirale Expression dominant-negativer VEGF-Rezeptorvarianten (Millauer et al., Nature 367, 576-579, 1994), rekominanter VEGF-neutralisierender Rezeptorvarianten (Goldman et al., Proc. Natl. Acad. Sci. USA 95, 8795-8800, 1998), oder niedermolekularer Inhibitoren der VEGF-Rezeptortyrosinkinase (Fong et al., Cancer Res. 59, 99-106, 1999; Wedge et al., Cancer Res. 60, 970-975, 2000; Wood et al., Cancer Res. 60, 2178-2189, 2000) resultierten in einem verringerten Tumorwachstum und einer verringerten Tumorvaskularisierung. Somit ist die Hemmung der Angiogenese ein möglicher Behandlungsmodus für Tumorerkrankungen.

Erfindungsgemäße Verbindungen können dementsprechend entweder Zyklin-abhängigen Kinasen, wie CDK1, CDK2, CDK3, CDK4, CDK5, CDK6, CDK7, CDK8 und CDK9, sowie der Glycogen-Synthase-Kinase (GSK-3β) und VEGF-Rezeptortyrosinkinasen oder Zyklin-abhängigen Kinasen oder VEGF-Rezeptortyrosinkinasen inhibieren. Diese Wirkungen tragen dazu bei, dass die erfindungsgemäßen Verbindungen verwendet werden können bei der Behandlung von Krebs, Angiofribroma, Arthritis, Augenerkrankungen, Autoimmunerkrankungen, Chemotherapeutika-induzierter Alopezie und Mukositis, Crohn-Krankheit, Endometriose, fibrotische Erkrankungen, Hämangioma, kardiovaskulären Erkrankungen, infektiösen Erkrankungen, nephrologischen Erkrankungen, chronischen und akuten neurodegenerativen Erkrankungen, sowie von Verletzungen des Nervengewebes, viralen Infektionen, zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, zur Unterstützung der narbenfreien Wundheilung, bei Altersflecken und bei Kontaktdermatitis, wobei
unter Krebs solide Tumoren, Tumor- oder Metastasenwachstum, Kaposis Sarkom, Morbus Hodgkin und Leukämie,
unter Arthritis, rheumatoide Arthritis, unter Augenerkrankungen, diabetische Retinopathie, Neovaskulares Glaukom,
unter Autoimmunerkrankungen Psoriasis, Alopezie und Multiple Sklerose,
unter fibrotische Erkrankungen, Leberzirrhose, mesangialzellproliferative Erkrankungen, Arteriosklerose,
unter infektiösen Erkrankungen durch unizelluläre Parasiten hervorgerufene Erkrankungen,
unter kardiovaskulären Erkrankungen Stenosen, wie z. B. Stent-induzierte Restenose, Arteriosklerosen und Restenosen,
unter nephrologischen Erkrankungen Glomerulonephritis, diabetische Nephropatie, maligne Nephrosklerose, thrombische mikroangiopatische Syndrome, Transplantationsabstoßungen und Glomerulopathie,
unter chronisch neurodegenerativen Erkrankungen Huntington's Erkrankung, amyotrophe Lateralsklerose, Parkinsonsche Erkrankung, AIDS Dementia und Alzheimer'sche Erkrankung,
unter akut neurodegenerativen Erkrankungen Ischämien des Gehirns und Neurotraumata,
und unter viralen Infektionen Cytomegalus-Infektionen, Herpes, Hepatitis B oder C, und HIV Erkrankungen zu verstehen sind.

Ebenfalls Gegenstand der vorliegenden Erfindung sind Arzneimittel zur Behandlung der oben aufgeführten Erkrankungen, die mindestens eine Verbindung gemäß der allgemeinen Formel (I) enthalten, sowie Arzneimittel mit geeigneten Formulierungs- und Trägerstoffen.

Die erfindungsgemäßen Verbindungen der allgemeinen Formel I sind unter anderem hervorragende Inhibitoren der Zyklin-abhängigen Kinasen, wie CDK1, CDK2, CDK3, CDK4, CDK5, CDK6, CDK7, CDK8 und CDK9, sowie der Glycogen-Synthase-Kinase (GSK-3β).

Die für die Herstellung der erfindungsgemäßen Verbindungen der allgemeinen Formel I vorzugsweise verwendeten Zwischenprodukte der allgemeinen Formeln (IIa) oder (IIb) 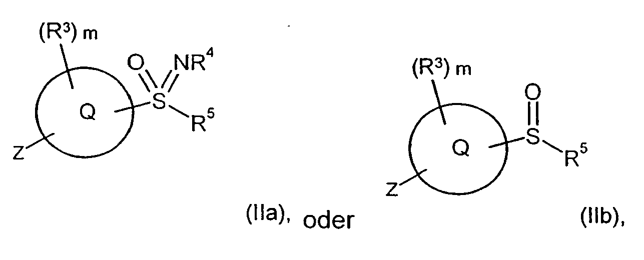 in der Z für -NH₂ oder NO₂ steht und m, R³, R⁴ und R⁵ die in der allgemeinen Formel (I) angegebenen Bedeutungen haben, sowie deren Isomeren, Diastereomeren, Enantiomeren und Salze als Zwischenprodukte sind ebenfalls Gegenstand der vorliegenden Erfindung.

Die für die Herstellung der erfindungsgemäßen Verbindungen der allgemeinen Formel I ebenfalls vorzugsweise verwendeten Zwischenprodukte der allgemeinen Formel (IIIa), (IIIb) oder (IIIc) 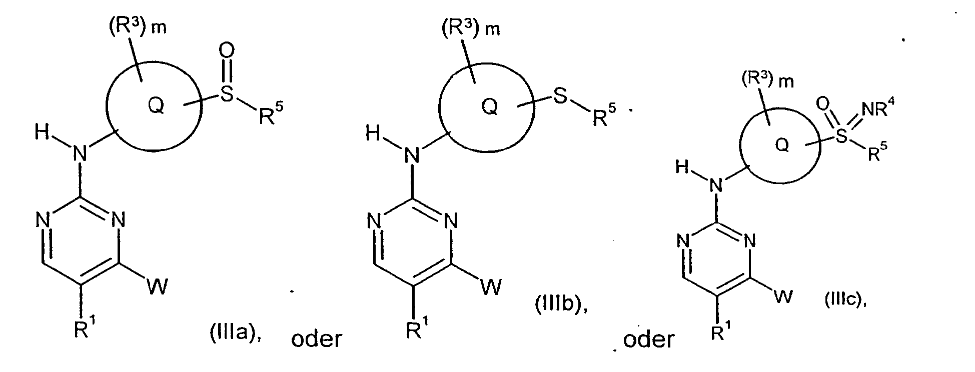 in der W für Halogen, Hydroxy oder X-R² steht und R¹, R², R³, R⁵, m und X die in der allgemeinen Formel (I) angegebenen Bedeutungen haben, sowie deren Isomeren, Diastereomeren, Enantiomeren und Salze als Zwischenprodukte zur Herstellung der Verbindung der allgemeinen Formel (I).

Ebenfalls Gegenstand der vorliegenden Erfindung sind für die Herstellung der erfindungsgemäßen Verbindungen der allgemeinen Formel (I) vorzugsweise verwendeten Zwischenprodukte der allgemeinen Formel (IV) 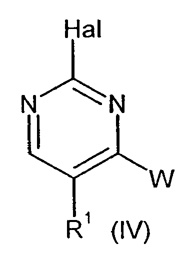 in der
Hal für Halogen, W für Halogen, Hydroxy oder X-R² steht und R¹, R² und X die in der allgemeinen Formel (I) angegebenen Bedeutungen haben, sowie deren Isomeren, Diastereomeren, Enantiomeren und Salze.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.
Die Isomerengemische können nach üblichen Methoden wie beispielsweise Kristallisation, Chromatographie oder Salzbildung in die Enantiomeren bzw. E/Z-Isomeren aufgetrennt werden.
Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

### Herstellung der erfindungsgemäßen Verbindungen

Eine der wichtigsten Methoden der Darstellung von Sulfoximinen ist die Umsetzung eines Sulfoxides mit Stickstoffwasserstoffsäure, die in situ z.B. aus der Reaktion von Natriumazid und konz. Schwefelsäure erzeugt wird (M. Reggelin, C. Zur, Synthesis 2000, 1, 1). Die Reaktion kann in einem organischen Lösungsmittel, wie Chloroform, durchgeführt werden. Weitere Methoden zur Synthese von Sulfoximinen sind z.B. die Umsetzung von Sulfoxiden mit
a) TsN₃ ((a) R. Tanaka, K. Yamabe, J. Chem. Soc. Chem. Commun. 1983, 329; (b) H. Kwart, A.A. Kahn, J. Am. Chem. Soc. 1967, 89, 1959)).
b) N-tosylimino phenyl iodinan und kat. Mengen Cu(I)triflat (J.F.K. Müller, P. Vogt, Tetrahedron Lett. 1998, 39, 4805)
c) Boc-Azid und kat. Mengen Eisen(II)chlorid (T. Bach, C. Korber, Tetrahedron Lett. 1998, 39, 5015) oder
d) o-Mesitylensulfonylhydroxylamin (MSH) (C.R. Johnson, R.A. Kirchhoff, H.G. Corkins, J. Org. Chem. 1974, 39, 2458).
e) [N-(2-(Trimethylsilyl)ethanesulfonyl)imino]phenyliodinane (PhI=NSes) (S. Cren, T.C. Kinahan, C.L. Skinner and H. Tye, Tetrahedron Lett. 2002, 43, 2749).

Sulfoximine besitzen in bezug auf Struktur und Konfiguration in der Regel eine hohe Stabilität (C. Bolm, J.P. Hildebrand, J. Org. Chem. 2000, 65, 169). Diese Eigenschaften der funktionellen Gruppe erlauben oftmals auch drastische Reaktionsbedingungen und ermöglichen die einfache Derivatisierung der Sulfoximine am Imin-Stickstoff und dem α-Kohlenstoff. Enantiomerenreine Sulfoximine werden auch als Auxiliare in der diastereoselektiven Synthese verwendet ((a) S.G. Pyne, Sulfur Reports 1992, 12, 57; (b) C.R. Johnson, Aldrichchimica Acta 1985, 18, 3). Die Darstellung enantiomerenreiner Sulfoximine ist z.B. über die Racematspaltung mit enantiomerenreiner Campher-10-sulfonsäure beschrieben ((a) C.R. Johnson, C.W. Schroeck, J. Am. Chem. Soc. 1973, 95, 7418; (b) C.S. Shiner, A.H. Berks, J. Org. Chem. 1988, 53, 5543). Eine weitere Methode zur Darstellung optisch aktiver Sulfoximine besteht in der stereoselektiven Iminierung von optisch aktiven Sulfoxiden.unter Verwendung von MSH ((a) C. Bolm, P. Müller, K. Harms, Acta Chem. Scand. 1996, 50, 305; (b) Y. Tamura, J. Minamikawa, K. Sumoto, S. Fujii, M. Ikeda, J. Org. Chem. 1973, 38, 1239).

Die nachfolgenden Beispiele erläutern die Herstellung der erfindungsgemäßen Verbindungen.

### Verfahrensvariante 1

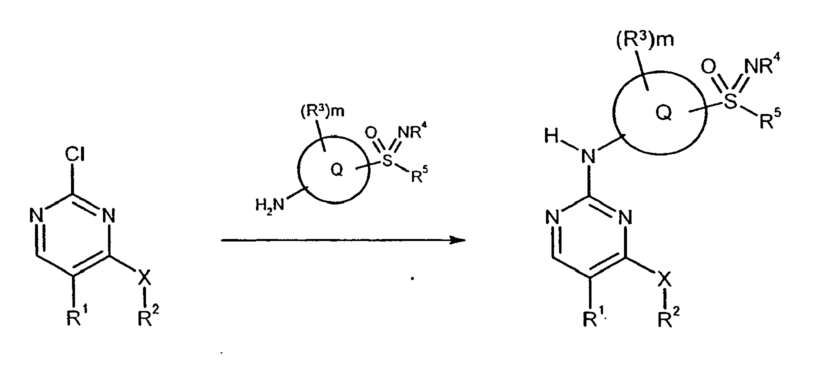

Die Substituenten Q, R¹, R², R³, R⁴, R⁵ und m haben die in der allgemeinen Formel (I) angegebene Bedeutung.

### Bsp. 1.0) Herstellung von (RS)-S-[4-({5-Brom-4-[(R)-(2-hydroxy-1-methyl-ethyl)amino]pyrimidin-2-yl}amino)phenyl]-S-methylsulfoximid

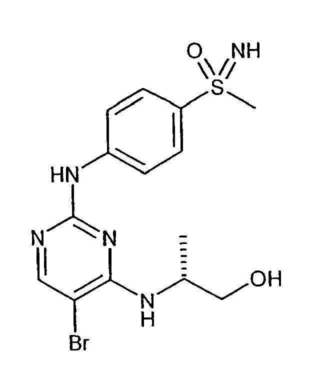

### Methode A

40 mg (0,23 mmol) (RS)-S-(4-Aminophenyl)-S-methylsulfoximid und 62 mg (0,23 mmol) (R)-2-[(5-Brom-2-chlorpyrimidin-4-yl)amino]propan-1-ol werden unter Argon mit 0,5 ml 1-Butyl-3-Methyl-imidazoliumtetrafluoroborat (Übersichtsartikel zu ionischen Flüssigkeiten: a) T. Welton, Chem. Rev. 1999, 99, 2071 b) H. Zhao, Aldrichimica Acta 2002, 35, 75 c) M.J. Earle, K.R. Seddon, ACS Symposium Series 2002, 819, 10) versetzt und 10 min bei Raumtemperatur gerührt. Das Reaktionsgemisch wird auf 60 °C erwärmt und weitere 3 h bei dieser Temperatur gerührt. Man versetzt mit 0,08 ml einer 4 molaren Lösung von Chlorwasserstoff in Dioxan und rührt 60 h bei 60 °C. Nach dem Abkühlen wird das Reaktionsgemisch mit 10 ml Essigester versetzt und 10 min gerührt. Das organische Lösungsmittel wird dekantiert und der Rückstand in 10 ml Methanol gelöst. Man versetzt mit 200 ml Essigester und wäscht anschließend mit 50 ml einer gesättigten NaCl-Lösung. Die organische Phase wird getrocknet (Na₂SO₄), filtriert und eingeengt. Der verbleibende Rückstand wird chromatographisch (DCM / Ethanol 8 : 2) gereinigt. Man erhält 23 mg (0,06 mmol, entsprechend 26 % d. Theorie) des Produktes.

### Methode B

Eine Lösung von 267 mg (1,0 mmol) (R)-2-[(5-Brom-2-chlorpyrimidin-4-yl)amino]propan-1-ol in 2 ml Acetonitril wird bei Raumtemperatur zu 171 mg (1,0 mmol) (RS)-S-(4-Aminophenyl)-S-methylsulfoximid in 1 ml Acetonitril gegeben. Der Ansatz wird mit 0,25 ml einer 4 molaren Lösung von Chlorwasserstoff in Dioxan versetzt und über Nacht unter Rückfluss gerührt. Das Lösungsmittel wird abgezogen und der verbleibende Rückstand chromatographisch (DCM / EtOH 8 : 2) gereinigt. Das erhaltene Rohprodukt wird abschliessend per HPLC gereinigt:

| | |
|---|---|
| Säule: | Luna C18(2) 5µ |
| Länge x ID: | 150 x 21,2 mm |
| Eluenten: | A = H₂O, B = ACN, A / 0.5 g NH₄Ac / I |
| Fluß: | 10,0 ml / min |
| Gradient: | 5 → 100% B(5')-5 → 100% B(30')+100%B(5') |
| Detektor: | PDA 214 nm |
| Temperatur: | 21 °C |
| RT in min: | 20,3 |

Man erhält 53 mg (0,13 mmol, entsprechend 13% d. Theorie) des Produktes.

¹H-NMR (DMSO): 9.71 (s, 1 H), 8.11 (s, 1 H), 7.91 (d, 2H), 7.78 (d, 2H), 6.41 (d, 1 H), 4,89 (t, 1 H), 4. 25 (m, 1 H), 3.96 (br, 1 H), 3.53 (m, 2H), 3.03 (s, 3H), 1.21 (d, 3H).
MS: 400 (ES).

### Bsp. 1.1) Herstellung von (RS)-S-[3-({5-Brom-4-[(R)-(2-hydroxy-1-methylethyl)amino]pyrimidin-2-yl}amino)phenyl]-S-methyl-N-nitrosulfoximid

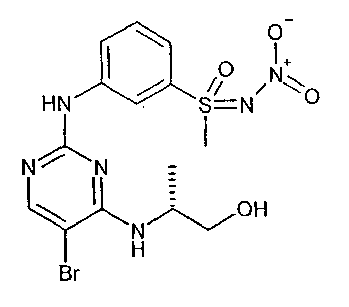

Eine Lösung von 37 mg (0,17 mmol) (RS)-S-(3-Aminophenyl)-S-methyl-N-nitrosulfoximid in 3 ml Acetonitril wird mit 91 mg (0,34 mmol) (R)-2-[(5-Brom-2-chlorpyrimidin-4-yl)amino]propan-1-ol und 0,06 ml einer 4 molaren Lösung von Chlorwasserstoff in Dioxan versetzt und über Nacht unter Rückfluss gerührt. Es werden weitere 0,05 ml der 4 molaren Lösung von Chlorwasserstoff in Dioxan zugegeben und weitere 6 h refluxiert. Nach DC Kontrolle wird erneut mit 92 mg (0,34 mmol) (R)-2-[(5-Brom-2-chlorpyrimidin-4-yl)amino]propan-1-ol versetzt und über Nacht refluxiert. Nach dem Erkalten wird der Ansatz mit gesättigter NaHCO₃ Lösung basisch gestellt und gegen Essigester extrahiert. Die vereinten organischen Phasen werden getrocknet (Na₂SO₄), filtriert und eingeengt. Der erhaltene Rückstand wird chromatographisch (DCM / EtOH 95 : 5) gereinigt. Man erhält 24 mg (0,05 mmol, entsprechend 32 % d. Theorie) des Produktes (Diastereomere **A / B** 1 : 1).

¹H-NMR (DMSO): 9.85 (s, 2H, **A+B**), 8.73 (m, 1H, **A**), 8.69 (m, 1H, **B**), 8.11 (s, 1 H, **A**), 8.10 (s, 1 H, **B**), 7.92 (m, 2H, **A+B**), 7.58 (m, 4H, **A+B**), 6.40 (m, 2H, **A+B**), 4.86 (t, 2H, **A+B**), 4.32 (m, 2H, **A+B**), 3.68 (s, 3H, **A**), 3.66 (s, 3H, **B**), 3.55 (m, 4H, **A+B**), 1.23 (d, 3H, **A**), 1.21 (d, 3H, **B**).
MS: 445 (ES).

In analoger Verfahrensweise zu den oben beschriebenen Verfahrensvarianten werden auch die nachfolgenden Verbindungen hergestellt:

### Bsp. 1.2) Herstellung von (RS)-S-[4-({5-Brom-4-[(1R,2R)-(2-hydroxy-1-methylpropyl)amino]pyrimidin-2-yl}amino)phenyl]-S-methylsulfoximid

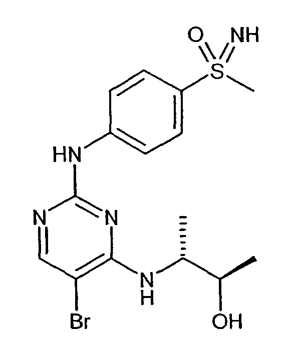

¹H-NMR (DMSO): 9.73 (s, 1 H), 8.12 (s, 1 H), 7.91 (d, 2H), 7.86 (d, 2H), 6.14 (d, 1 H), 5.02 (br, 1 H), 4.09 (m, 1 H), 3.97 (s, 1 H), 3.78 (m, 1 H), 3.02 (s, 3H), 1.25 (d, 3H), 1.09 (d, 3H).
MS: 414 (ES).

### Bsp. 1.3) Herstellung von (RS)-S-[4-({5-Brom-4-[(R)-(2-hydroxy-1,2-dimethylpropyl)amino]pyrimidin-2-yl}amino)phenyl]-S-methylsulfoximid

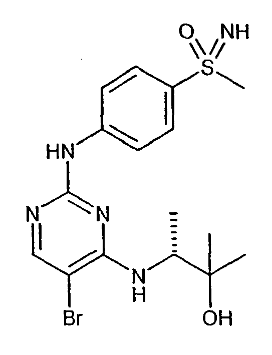

¹H-NMR (DMSO): 9.72 (s, 1H), 8.11 (s, 1 H), 7.90 (d, 2H), 7.78 (d, 2H), 6.10 (d, 1H), 4.87 (s, 1 H), 4.07 (m, 1H), 3.98 (s, 1 H), 3.01 (s, 3H), 1.19 (m, 9H).
MS: 428 (ES).

### Bsp. 1.4) Herstellung von (RS)-S-[4-({5-Brom-4-[(1R,2R)-2-hydroxy-1-methylpropoxy]pyrimidin-2-yl}amino)phenyl]-S-methylsulfoximid

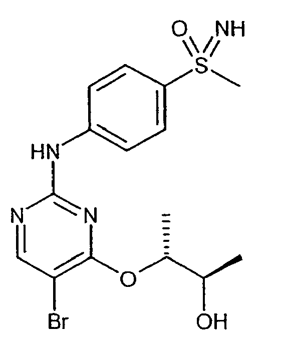

¹H-NMR (DMSO): 10.12 (s, 1 H), 8.45 (s, 1 H), 7.92 (d, 2H), 7.84 (d, 2H), 5.21 (m, 1H), 4.91 (d, 1 H), 4.04 (s, 1 H), 3.87 (m, 1 H), 3.03 (s, 3H), 1.28 (d, 3H), 1.13 (d, 3H).
MS: 415 (ES).

### Bsp. 1.5) Herstellung von (RS)-S-[4-({5-Brom-4-[(R)-(2-hydroxy-1,2-dimethylpropyl)amino]pyrimidin-2-yl}amino)phenyl]-S-cyclopropyl-N-[2-(trimethylsilyl)ethylsulfonyl]sulfoximid

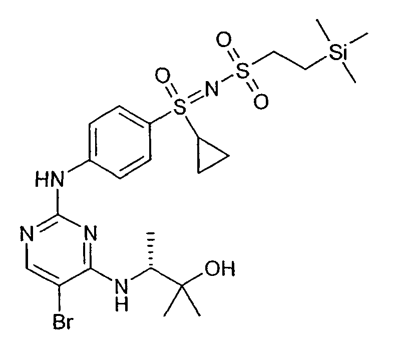

95 mg (0,32 mmol) (*R*)-3-[(5-Brom-2-chlorpyrimidin-4-yl)amino]-2-methyl-butan-2-ol werden in 2 ml Acetonitril gelöst und mit 116 mg (0,32 mmol) (*RS*)-*S*-(4-Aminophenyl)-*S*-cyclopropyl-*N*-[2-(trimethylsilyl)ethylsulfonyl]sulfoximid versetzt. Nach Zugabe von 0,08 ml einer ca. 4N Lösung von HCl in Dioxan und 0,08 ml Wasser wird die Mischung in einem geschlossenen Gefäß 16 Stunden auf 75 °C erwärmt. Die Suspension wird filtriert und das Filtrat durch Flash-Chromatografie (Dichlormethan - Dichlormethan/Ethanol 95:5, 15 ml/min) aufgetrennt. Die Fraktionen 43-51 min enthalten 50 mg (25 % d. Th.) des gewünschten Produktes.

¹H-NMR (DMSO): 9.91 (s, 1 H), 8.16 (s, 1H), 8.01 (d, 2H), 7.83 (d, 2H), 6.14 (d, 1 H), 4.87 (s, 1 H), 4.10 (m, 1 H), 3.18 (m, 1 H), 2.92 (m, 2H), 1.37-1.00 (m, 4H), 1.21 (s, 3H), 1.20 (d, 3H), 1.14 (s, 3H), 0.93 (m, 2H), 0.01 (s, 9H).
MS: 618/620 (100 %, ES).

### Bsp. 1.6) Herstellung von (RS)-S-[4-({5-Brom-4-[(R)-(2-hydroxy-1,2-dimethylpropyl)amino]pyrimidin-2-yl}amino)phenyl]-S-cyclopropylsulfoximid

### Methode C

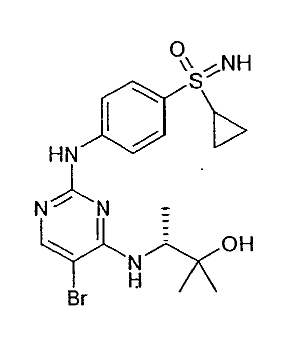

50 mg (RS)-S-[4-({5-Brom-4-[(R)-(2-hydroxy-1,2-dimethylpropyl)amino]pyrimidin-2-yl}amino)phenyl]-S-cyclopropyl-N-[2-(trimethylsilyl)ethylsulfonyl]sulfoximid werden in 1 ml Tetrahydrofuran gelöst und mit 0,3 ml einer 1 M Lösung von Tetrabutylammoniumfluorid in Tetrahydrofuran versetzt. Die Mischung wird 3 Tage bei 50 °C gerührt und durch Flash-Chromatografie (Dichlormethan - Dichlormethan/Ethanol 9:1) gereinigt. Man erhält 10 mg (28 % d. Th.) des Produktes.

¹H-NMR (DMSO): 9.72 (s, 1H), 8.13 (s, 1 H), 7.90 (d, 2H), 7.74 (d, 2H), 6.10 (d, 1 H), 4.86 (s, 1 H), 4.10 (m, 1 H), 3.95 (s, 1 H), 3.16 (m, 1 H), 1.40-1.00 (m, 4H), 1.20 (s, 3H), 1.19 (d, 3H), 1.15 (s, 3H).
MS: 454/456 (20 %, ES).

In analoger Verfahrensweise zu den vorbeschriebenen Verfahrensvarianten werden auch die nachfolgenden Verbindungen hergestellt.

### Bsp. 1.7) Herstellung von (RS)-S-[4-({5-Brom-4-[(R)-(1-hydroxy-1-methylethyl)amino]pyrimidin-2-yl}amino)phenyl]-S-(cyclopropylmethyl)-N-[2-(trimethylsilyl)ethylsulfonyl]sulfoximid

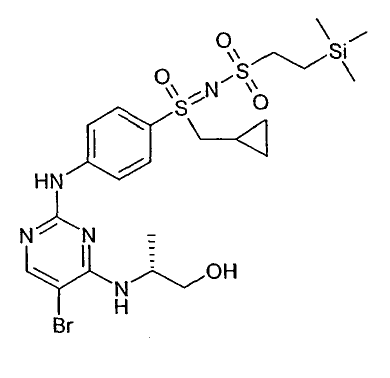

¹H-NMR (DMSO): 10.33 (s, 1 H), 8.23 (s, 1H), 8.01 (d, 2H), 7.88 (d, 2H), 7.02 (d, 1 H), 5.58 (s br, 1 H), 4.28 (m, 1 H), 3.67 (d, 2H), 3.55 (m, 2H), 2.98 (m, 2H), 1.21 (d, 3H), 0.97 (m, 2H), 0.86 (m, 1 H), 0.44 (m, 2H), 0.12 (m, 2H), 0.01 (s, 9H) MS: 604/606 (100 %, ES).
Schmp.: 195 °C (Zers.).

### Bsp. 1.8) Herstellung von (RS)-S-[4-({5-Brom-4-[(R)-(2-hydroxy-1,2-dimethylpropyl)amino]pyrimidin-2-yl}amino)phenyl]-S-(cyclopropylmethyl)-N-[2-(trimethylsilyl)ethylsulfonyl]sulfoximid

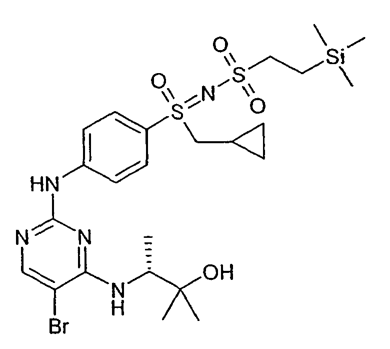

¹H-NMR (DMSO): 9.93 (s, 1 H), 8.16 (s, 1 H), 8.02 (d, 2H), 7.83 (d, 2H), 6.14 (d, 1 H), 4.87 (s, 1 H), 4.10 (m, 1 H), 3.64 (d, 2H), 2.96 (m, 2H), 1.21 (s, 3H), 1.20 (d, 3H), 1.15 (s, 3H), 0.98 (m, 2H), 0.87 (m, 1H), 0.46 (m, 2H), 0.13 (m, 2H), 0.02 (s, 9H).
MS: 632/634 (40 %, ES).

### Bsp. 1.9) Herstellung von (RS)-S-[4-({5-Brom-4-[(R)-(2-hydroxy-1,2-dimethylpropyl)amino]pyrimidin-2-yl}amino)phenyl]-S-(cyclopropylmethyl)sulfoximid

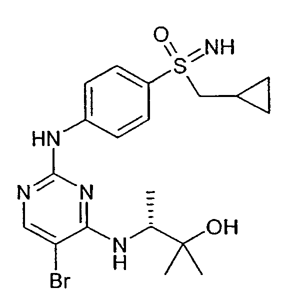

¹H-NMR (DMSO): 9.73 (s, 1H), 8.13 (s, 1 H), 7.92 (d, 2H), 7.75 (d, 2H), 6.10 (d, 1H), 4.85 (s, 1 H), 4.10 (m, 1 H), 3.92 (s, 1 H), 3.02 (m, 2H), 1.20 (s, 3H), 1.19 (d, 3H), 1.14 (s, 3H), 0.87 (m, 1H), 0.37 (m, 2H), 0.00 (m, 2H).

### Bsp. 1.10) Herstellung von (RS)-S-[4-({5-Brom-4-[(R)-(2-hydroxy-1,2-dimethylpropyl)amino]pyrimidin-2-yl}amino)phenyl]-S-cyclopentyl-N-[2-(trimethylsilyl)ethylsulfonyl]sulfoximid

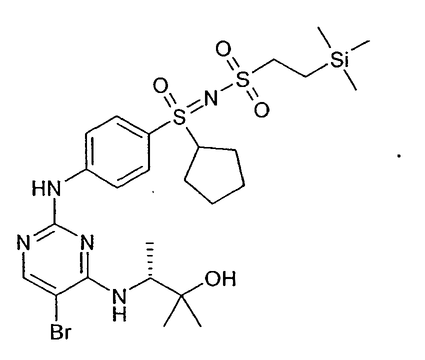

Schmp.: 200-201 °C.

### Bsp. 1.11) Herstellung von (RS)-S-[4-({5-Brom-4-[(R)-(2-hydroxy-1,2-dimethylpropyl)amino]pyrimidin-2-yl}amino)phenyl]-S-cyclopentylsulfoximid

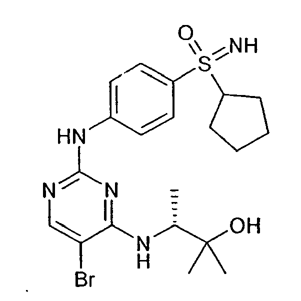

Schmp.: 194-196 °C.

### 1.12) Herstellung von (RS)-S-[4-({5-Brom-4-[(R)-(2-hydroxy-1,2-dimethylpropyl)amino]pyrimidin-2-yl}amino)phenyl]-S-(2-hydroxyethyl)-N-[2-(trimethylsilyl)ethylsulfonyl]sulfoximid

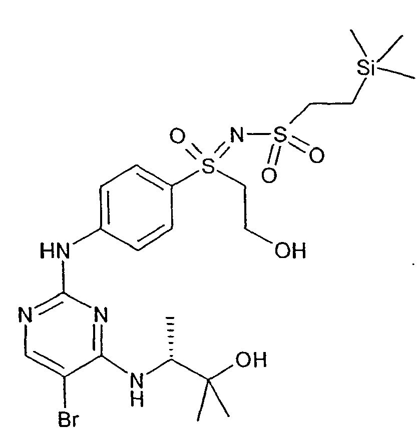

Eine Lösung von 200 mg (0,55 mmol) (RS)-S-(4-Aminophenyl)-S-(2-hydroxyethyl)-N-[2-(trimethylsilyl)ethylsulfonyl]sulfoximid in 2 ml Acetonitril und 0,5 ml Wasser wird mit 0,17 ml einer 4 N Lösung von HCl in Dioxan versetzt. Man gibt 198 mg (0,67 mmol) (*R*)-3-[(5-Brom-2-chlorpyrimidin-4-yl)amino]-2-methyl-butan-2-ol in 1,5 ml Acetonitril hinzu, und der Ansatz wird 20 Stunden bei 80 °C gerührt. Das Lösungsmittel wird entfernt und der verbleibende Rückstand chromatographisch (DCM / EtOH 9 : 1) gereinigt. Man erhält 148 mg (0,24 mmol, entsprechend 44 % d. Theorie) des Produktes.

¹H-NMR (DMSO): 10.21 (s, 1H), 8.21 (s, 1H), 7.97 (m, 2H), 7.85 (m, 2H), 6.42 (d, 1 H), 4.10 (m, 1 H), 3.80 (m, 2H), 3.70 (m, 2H), 2.95 (m, 2H), 1.20 (m, 9H), 0.96 (m, 2H), 0.03 (s, 9H).

### Bsp. 1.13) Herstellung von (RS)-S-[4-({5-Brom-4-[(R)-(2-hydroxy-1,2-dimethylpropyl)amino]pyrimidin-2-yl}amino)phenyl]-S-(2-hydroxyethyl)-sulfoximid

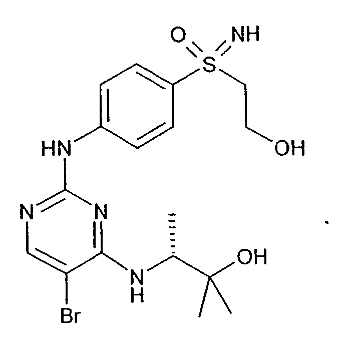

¹H-NMR (DMSO): 9.75 (s, 1 H), 8.13 (s, 1 H), 7.91 (m, 2H), 7.75 (m, 2H), 6.12 (d, 1 H), 4.85 (m, 2H), 4.11 (m, 2H), 3.65 (m, 2H), 3.23 (m, 2H), 1.17 (m, 9H).

Das erhaltene Diastereomerengemisch wird mittels präperativer HPLC in die reinen Diastereomere gespalten:

| | |
|---|---|
| Säule: | Chiralpak AD 20µ |
| Länge x ID: | 250 x 60 mm |
| Eluenten: | Hexan / Ethanol 70 : 30 |
| Fluss: | 80 ml / min |
| Detektor: | UV 300nm |
| Temperatur: | Raumtemperatur |
| RT in min: | 23,41; Diastereomer 1 (Bsp.**1.14**) |
| | 54,16; Diastereomer 2 (Bsp.**1.15**) |

### Bsp. 1.16) Herstellung von (RS)-S-[4-({5-Brom-4-[(1R,2R)-(2-hydroxy-1-methylpropyl)amino]pyrimidin-2-yl}amino)phenyl]-S-(2-hydroxyethyl)-N-[2-(trimethylsilyl)ethylsulfonyl]sulfoximid

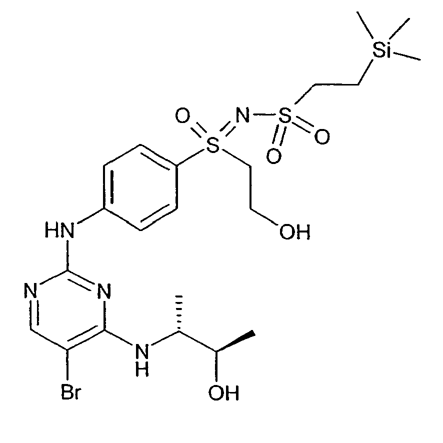

¹H-NMR (DMSO): 10.53 (s, 1H), 8.28 (s, 1H), 7.95 (m, 2H), 7.88 (m, 2H), 6.86 (d, 1 H), 4.13 (m, 1 H), 3.76 (m, 5H), 2.90 (m, 2H), 1.25 (d, 3H), 1.11 (d, 3H), 0.93 (m, 2H), 0.03 (s, 9H).

### 1.17) Herstellung von (RS)-S-[4-({5-Brom-4-[(1R,2R)-(2-hydroxy-1-methyl-propyl)amino]pyrimidin-2-yl}amino)phenyl]- S-(2-hydroxyethyl)sulfoximid

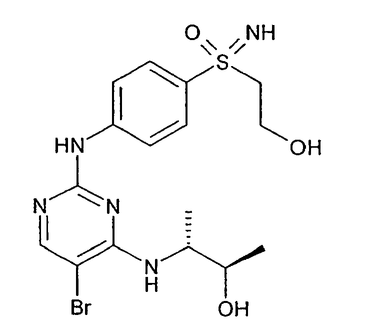

¹H-NMR (DMSO): 9.75 (s, 1 H), 8.11 (s, 1 H), 7.92 (m, 2H), 7.72 (m, 2H), 6.14 (d, 1 H), 5.02 (d, 1 H), 4.85 (tr, 1 H), 4.10 (m, 2H), 3.78 (m, 1 H), 3.62 (m, 2H), 3.22 (m, 2H), 1.23 (d, 3H), 1.08 (d, 3H).
MS: 444 (ES).

Das erhaltene Diastereomerengemisch wird mittels präperativer HPLC in die reinen Diastereomere gespalten:

| | |
|---|---|
| Säule: | Chiralpak AD-H 5µ |
| Länge x ID: | 250 x 20 mm |
| Eluenten: | A: Hexan, C: Ethanol |
| Fluss: | 10 ml / min |
| Gradient: | Isokratisch 50 % C |
| Detektor: | UV 300nm |
| Temperatur: | Raumtemperatur |
| RT in min: | 13,1; Diastereomer 1 (Bsp.**1.18**) |
| | 18,9; Diastereomer 2 (Bsp.**1.19**) |

### 1.20) Herstellung von (RS)-S-[4-({5-Brom-4-[(1R,2R)-2-hydroxy-1-methyl-propoxy]pyrimidin-2-yl}amino)phenyl]-S-(2-hydroxyethyl)-N-[2-(trimethylsilyl)ethylsulfonyl]sulfoximid

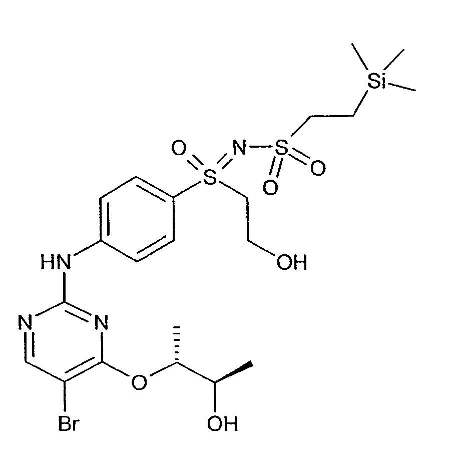

Eine Lösung von 205 mg (0,56 mmol) (RS)-S-(4-Aminophenyl)-S-(2-hydroxyethyl)-N-[2-(trimethylsilyl)ethylsulfonyl]sulfoximid in 2 ml Acetonitril wird mit 0,15 ml eine 4 N Lösung von HCl in Dioxan versetzt. Man gibt 175 mg (0,62 mmol) (2*R*, 3*R*)-3-[(5-Brom-2-chlorpyrimidin-4-yl)oxy]-butan-2-ol in 2 ml Acetonitril hinzu und rührt den Ansatz 24 Stunden bei 70 °C . Anschließend wird weitere 24 h bei 85 °C gerührt. Das Lösungsmittel wird entfernt und der verbleibende Rückstand chromatographisch (DCM / EtOH 9 : 1) gereinigt. Man erhält 110 mg (0,18 mmol, entsprechend 32 % d. Theorie) des Produktes.

¹H-NMR (DMSO): 10.31 (s, 1 H), 8.45 (s, 1 H), 7.99 (m, 2H), 7.83 (m, 2H), 5.25 (m, 1 H), 4.93 (m, 2H), 3.75 (m, 5H), 2.90 (m, 2H), 1.32 (d, 3H), 1.13 (d, 3H), 0.93 (m, 2H), 0.05 (s, 9H).
MS: 609 (ES).

### 1.21) Herstellung von (RS)-S-[4-({5-Brom-4-[(1R,2R)-2-hydroxy-1-methyl-propoxy]pyrimidin-2-yl}amino)phenyl]-S-(2-hydroxyethyl)sulfoximid

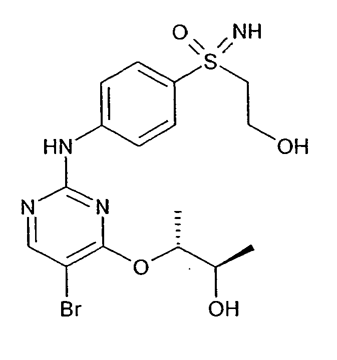

| | |
|---|---|
| Säule: | Kromasil C8 5µ |
| Länge x ID: | 125 x 20 mm |
| Eluenten: | A: H₂O + 0,1 % NH₃, B: ACN |
| Fluss: | 15 ml / min |
| Gradient: | 24->38%B(10')->95(1') |
| Detektor: | UV 300nm |
| Temperatur: | Raumtemperatur |
| RT in min: | 10,9 |

¹H-NMR (DMSO): 10,10 (s, 1 H), 8.42 (s, 1 H), 7.88 (m, 2H), 7.77 (m, 2H), 5.23 (m, 1 H), 4.88 (d, 1 H), 4.85 (tr, 1 H), 4.18 (s, 1 H), 3.84 (m, 1 H), 3.63 (m, 2H), 3.22 (m, 2H), 1.28 (d, 3H), 1.14 (d, 3H).
MS: 445 (ES).

### Bsp. 1.22) Herstellung von (RS)-S-[3-({5-Brom-4-[(R)-(2-hydroxy-1,2-dimethylpropyl)amino]pyrimidin-2-yl}amino)phenyl]-S-methylsulfoximid

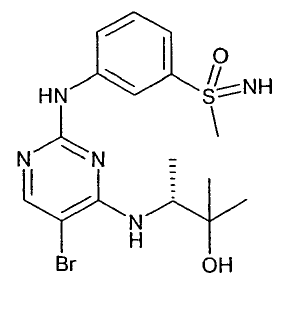

127 mg (0,43 mmol (*R*)-3-[(5-Brom-2-chlorpyrimidin-4-yl)amino]-2-methyl-butan-2-ol in 1 ml Acetonitril werden zu 74 mg (0,43 mmol) (RS)-S-(3-Aminophenyl)-S-methylsulfoximid in 0,5 ml Acetonitril gegeben. Es wird mit 0,1 ml einer 4 N Lösung von HCl in Dioxan versetzt und der Ansatz über Nacht refluxiert. Das Lösungsmittel wird abgezogen und der verbleibende Rückstand chromatographisch (DCM / EtOH 9 : 1) gereinigt. Man erhält 37 mg (0,09 mmol, entsprechend 20 % d. Theorie) des Produktes.
¹H-NMR (DMSO): 9.65 (s, 1 H), 8.75 (m, 1H), 8.08 (s, 1 H), 7.64 (m, 1 H), 7.42 (m, 2H), 6.04 (m, 1 H), 4.82 (br, 1 H), 4.20 (m, 1 H), 4.06 (m, 1 H), 3.03 (s, 3H), 1.18 (m, 9H).
MS: 428 (ES).

### Bsp. 1.23) Herstellung von (RS)-S-[4-({5-Brom-4-[(R)-(2-hydroxy-1,2-dimethylpropyl)amino]pyrimidin-2-yl}amino)-2-methoxyphenyl]-S-methylsulfoximid

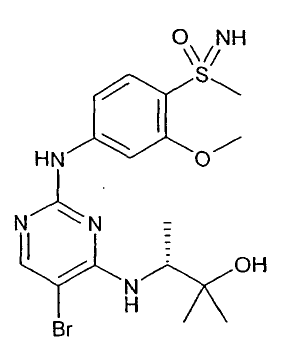

¹H-NMR (DMSO): 9.32 (s, 1 H), 8.49 (m, 1H), 8.02 (s, 1H), 7.64 (m, 1H), 7.15 (m, 1 H), 5.97 (d, 1 H), 4.81 (s, 1H), 4.19 (m, 1H), 4.06 (m, 1H), 3.87 (s, 3H), 3.15 (s, 3H), 1.15 (m, 9H).
MS: 458 (ES).

### Bsp. 1.24) Herstellung von (RS)-S-[4-({5-Brom-4-[(1R,2R)-2-hydroxy-1-methylpropoxy]pyrimidin-2-yl}amino)-2-methoxyphenyl]-S-methylsulfoximid

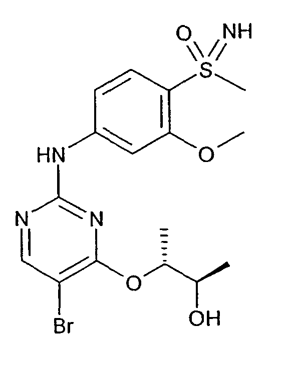

220 mg (1,1 mmol) (*RS*)-*S*-(4-Amino-2-methoxyphenyl)-*S*-methylsulfoximid und 280 mg (1,0 mmol) (2*R*, 3*R*)-3-[(5-Brom-2-chlorpyrimidin-4-yl)oxy]-butan-2-ol in 10 ml Acetonitril werden mit 0,28 ml einer 4 N Lösung von HCl in Dioxan versetzt und über Nacht unter Rückfluss gerührt. Man versetzt mit 1 ml einer Lösung von n-Butanol / Methanol (9:1) und rührt weitere 5 Tage unter Rückfluss. Der Ansatz wird eingeengt und der Rückstand chromatographisch (DCM / Ethanol 8 :2) gereinigt. Man erhält 36 mg (0,1 mmol, entsprechend 8 % d. Theorie) des Produktes.

¹H-NMR (DMSO): 9.81 (s, 1H), 8.32 (m, 2H), 7.71 (m, 1H), 7.18 (m, 1 H), 5.25 (m, 1 H), 4.95 (br, 1 H), 4.18 (m, 1H), 3.91 (s, 3H), 3.83 (m, 1 H), 3.15 (s, 3H), 1.25 (m, 3H), 1.10 (m, 3H).
MS: 445 (ES).

### Bsp. 1.25) Herstellung von (RS)-S-[4-({5-Brom-4-[(1R,2R)-(2-hydroxy-1-methylpropyl)amino]pyrimidin-2-yl}amino)-2-methoxyphenyl]-S-methylsulfoximid

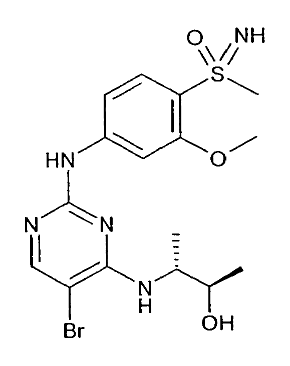

¹H-NMR (DMSO): 9.37 (s, 1 H), 8.43 (m, 1 H), 8.02 (s, 1 H), 7.70 (m, 1 H), 7.14 (m, 1 H), 5.98 (d, 1 H), 5.01 (d, 1H), 4,20 (m, 1 H), 4.07 (s, 1 H), 3.87 (s, 3H), 3.75 (m, 1H), 3.14 (s, 3H), 1.15 (d, 3H), 1.07 (d, 3H).
MS: 444 (ES).

Das erhaltene Diastereomerengemisch wird mittels präperativer HPLC in die reinen Diastereomere gespalten:

| | |
|---|---|
| Säule: | Chiralpak AD 20µ |
| Länge x ID: | 250 x 60 mm |
| Eluenten: | A = Hexan, B = Ethanol |
| Fluss: | 80 ml / min |
| Gradient: | Isokratisch 50% B |
| Detektor: | UV 280nm |
| Temperatur: | Raumtemperatur |
| RT in min: | 20,3; Diastereomer 1 (Bsp.**1.26**) |
| | 34,8; Diastereomer 2 (Bsp.**1.27**) |

### Bsp. 1.28) Herstellung von (RS)-S-[4-({5-Brom-4-[(1R,2R)-(2-hydroxy-1-methylpropyl)amino]pyrimidin-2-yl}amino)phenyl]-N,S-dimethyl-sulfoximid

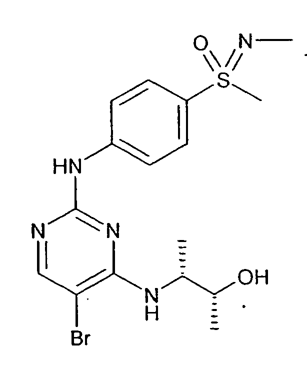

¹H-NMR (DMSO): 9.73 (s, 1 H), 8.11 (s, 1 H), 7.96 (m, 2H), 7.65 (m, 2H), 6.14 (d, 1 H), 5.01 (d, 1 H), 4.10 (m, 1 H), 3.79 (m, 1 H), 3.05 (s, 3H), 2.46 (s, 3H), 1.25 (d, 3H), 1.12 (d, 3H).
MS: 428 (ES)

Das erhaltene Diastereomerengemisch wird mittels präperativer HPLC in die reinen Diastereomere gespalten:

| | |
|---|---|
| Säule: | Chiralpak AD-H 5µ |
| Länge x ID: | 250 x 4,6 mm |
| Eluenten: | A = Hexan, B = Ethanol A / 0,1% DEA |
| Fluss: | 15 ml / min |
| Gradient: | Isokratisch 15% B |
| Detektor: | UV 300nm |
| Temperatur: | Raumtemperatur |
| RT in min: | 25,45; Diastereomer 1 (Bsp.**1.29**) |
| | 29,32; Diastereomer 2 (Bsp.**1.30**) |

### Bsp. 1.31) Herstellung von (RS)-S-[4-({5-Brom-4-[(R)-(2-hydroxy-1,2-dimethylpropyl)amino]pyrimidin-2-yl}amino)phenyl]-N-(ethoxycarbonyl)-S-methylsulfoximid

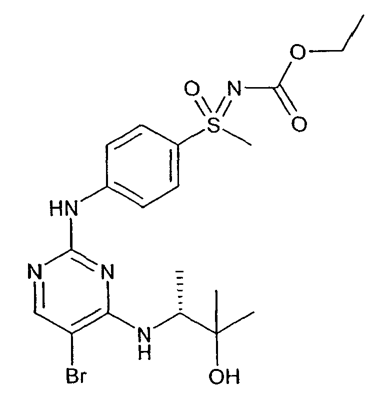

600 mg (2,48 mmol) (*RS*)-*S*-(4-Aminophenyl)-*N*-(ethoxycarbonyl)-S-methylsulfoximid und 610 mg (2,07 mmol) (*R*)-3-[(5-Brom-2-chlorpyrimidin-4-yl)amino]-2-methyl-butan-2-ol in 8 ml Acetonitril werden mit 0,52 ml Wasser und 0,52 ml einer 4 N Lösung von HCl in Dioxan versetzt. Der Ansatz wird 24 Stunden bei 60 °C gerührt und anschließend eingeengt. Der verbleibende Rückstand wird chromatographisch (DCM / EtOH 8 : 2) gereinigt. Man erhält 649 mg (1,30 mmol, entsprechend 53 % d. Theorie) des Produktes.

¹H-NMR (DMSO): 10.10 (s, 1 H), 8.20 (s, 1 H), 7.97 (m, 2H), 7.85 (m, 2H), 6.39 (d, 1 H), 4.10 (m, 1 H), 3.91 (m, 2H), 3.30 (s, 3H), 1.10 (m, 12H).

### Bsp. 1.32) Herstellung von (RS)-S-[4-({5-Brom-4-[(1R,2R)-(2-hydroxy-1-methylpropyl)amino]pyrimidin-2-yl}amino)phenyl]-N-(ethoxycarbonyl)-S-methylsulfoximid

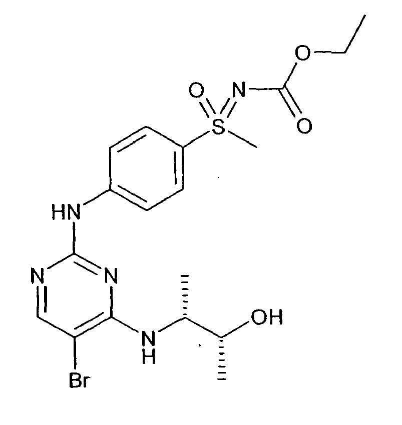

¹H-NMR (DMSO): 9.88 (s, 1H), 8.13 (s, 1H), 7.98 (m, 2H), 7.79 (m, 2H), 6.18 (d, 1 H), 5.01 (d, 1 H), 4.10 (m, 1 H), 3.90 (q, 2H), 3.78 (m, 1 H), 3.41 (s, 3H), 1.21 (d, 3H), 1.08 (m, 6H).

### Bsp. 1.33) Herstellung von (RS)-S-{4-[(5-Brom-4-{[(1R,2R)-2-hydroxy-1-(methoxymethyl)propyl]amino}pyrimidin-2-yl)amino]phenyl}-N-(ethoxycarbonyl)-S-ethylsulfoximid

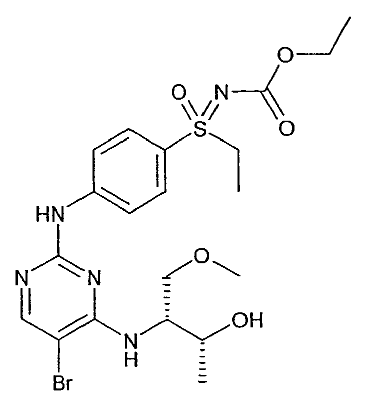

¹H-NMR (DMSO): 9.92 (s, 1 H), 8.17 (s, 1 H), 7.99 (m, 2H), 7.70 (m, 2H), 6.08 (d, 1 H), 5.12 (m, 1 H), 4.20 (m, 1 H), 4.00 (m, 1 H), 3.89 (m, 2H), 3.50 (m, 4H), 3.28 (s, 3H), 1.08 (m, 9H).

### Bsp. 1.34) Herstellung von (RS)-S-{4-[(5-Brom-4-{[(1R,2R)-2-hydroxy-1-(methoxymethyl)propyl]amino}pyrimidin-2-yl)amino]phenyl}-N-(ethoxycarbonyl)-S-methylsulfoximid

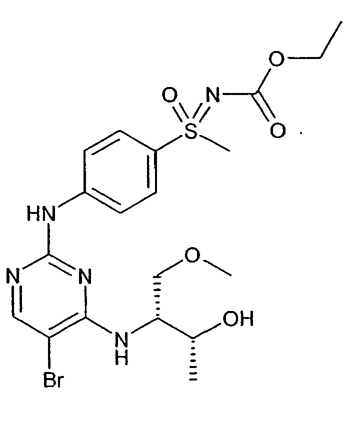

¹H-NMR (DMSO): 9.91 (s, 1 H), 8.17 (s, 1 H), 7.95 (m, 2H), 7.78 (m, 2H), 6.08 (d, 1 H), 5.13 (m, 1 H), 4.20 (m, 1 H), 3.95 (m, 3H), 3.48 (m, 2H) , 3.40 (s, 3H), 3.27 (s, 3H), 1.10 (m, 6H).

### Bsp. 1.35) Herstellung von (RS)-S-[4-({5-Brom-4-[(R)-(2-hydroxy-1,2-dimethylpropyl)amino]pyrimidin-2-yl}amino)phenyl]-N-(ethoxycarbonyl)-S-ethylsulfoximid

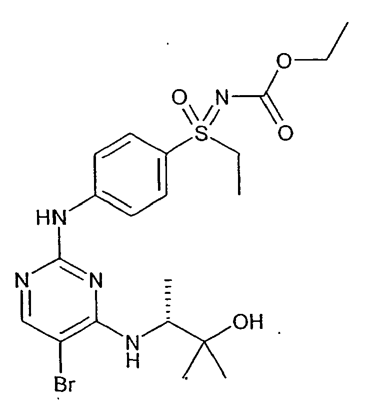

¹H-NMR (DMSO): 9.89 (s, 1 H), 8.14 (s, 1 H), 7.99 (m, 2H), 7.72 (m, 2H), 6.13 (d, 1 H), 4.84 (s, 1 H), 4.09 (m, 1 H), 3.90 (m, 2H), 3.54 (q, 2H), 1.15 (m, 15H).

### Bsp. 1.36) Herstellung von (RS)-S-[4-({5-Brom-4-[(1R,2R)-(2-hydroxy-1-methylpropyl)amino]pyrimidin-2-yl}amino)phenyl]-N-(ethoxycarbonyl)-S-ethylsulfoximid

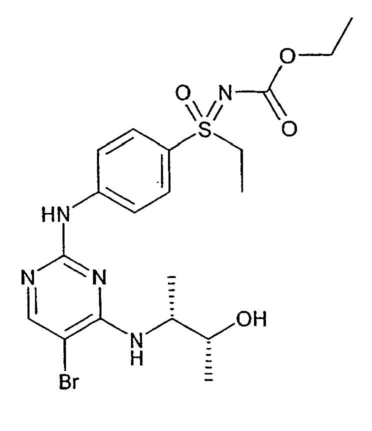

¹H-NMR (DMSO): 9.92 (s, 1 H), 8.13 (s, 1 H), 7.97 (m, 2H), 7.72 (m, 2H), 6.27 (d, 1 H), 4.10 (m, 1 H), 9.92 (m, 2H), 3.80 (m, 1 H), 3.55 (q, 2H), 1.23 (d, 3H), 1.10 (m, 9H).

### Bsp. 1.37) Herstellung von (RS)-S-[4-({5-Brom-4-[(R)-(2-hydroxy-1,2-dimethylpropyl)amino]pyrimidin-2-yl}amino)-2-methylphenyl]-N-(ethoxycarbonyl)-S-methylsulfoximid

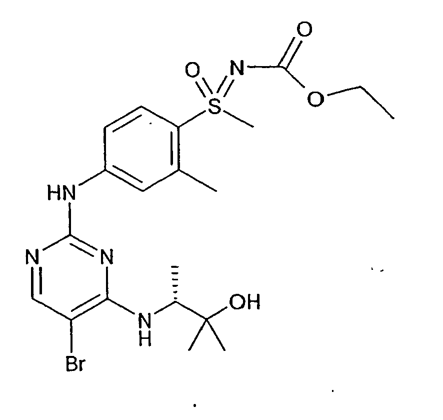

¹H-NMR (DMSO): 9.98 (s, 1 H), 8.18 (s, 1 H), 7.75 (m, 3H), 6.22 (d, 1H), 4.05 (m, 1 H), 3.88 (q, 2H), 3.39 (s, 3H), 2.57 (s, 3H), 1.15 (m, 12H).
MS: 514 (ES).

### Bsp. 1.38) Herstellung von (RS)-S-[4-({5-Brom-4-[(1R,2R)-(2-hydroxy-1-methylpropyl)amino]pyrimidin-2-yl}amino)-2-methylphenyl]-N-(ethoxycarbonyl)-S-methylsulfoximid

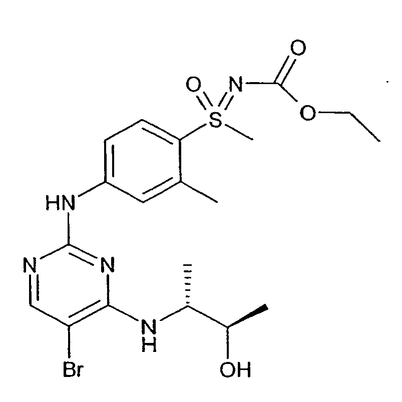

¹H-NMR (DMSO): 9.88 (s, 1 H), 8.13 (s, 1 H), 7.79 (m, 3H), 6.33 (d, 1 H), 4.04 (m, 1 H), 3.90 (q, 2H), 3.82 (m, 1 H), 3.30 (s, 3H), 2.62 (s, 3H), 1.22 (d, 3H), 1.08 (m, 6H).

### Bsp. 1.39) Herstellung von (RS)-S-[4-({5-Brom-4--[(1R,2R)-2-hydroxy-1-methylpropoxy]pyrimidin-2-yl}amino)phenyl]-N-(ethoxycarbonyl)-S-ethylsulfoximid

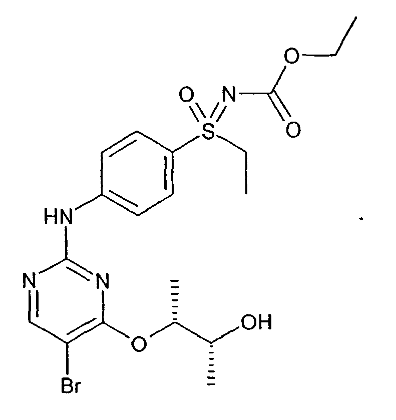

128 mg (0,51 mmol) (*RS*)-*S*-(4-Aminophenyl)-*N*-(ethoxycarbonyl)-S-ethylsulfoximid und 150 mg (0,53 mmol) (2R, 3R)-3-[(5-Brom-2-chlorpyrimidin-4-yl)oxy]-butan-2-ol in 2 ml Acetonitril werden mit 0,12 ml einer 4 N Lösung von HCl in Dioxan versetzt. Der Ansatz wird 2 Tage bei 60 °C gerührt. Das Lösungsmittel wird entfernt und der Rückstand chromatographisch (DCM / EtOH 95 : 5) gereinigt. Man erhält 43 mg (0,09 mmol,.entsprechend 17 % d. Theorie) des Produktes.

¹H-NMR (DMSO): 10.28 (s, 1H), 8.45 (s, 1H), 7.99 (m, 2H), 7.78 (m, 2H), 5.22 (m, 1 H), 4.91 (d, 1 H), 3.88 (m, 3H), 3.53 (q, 2H), 1.30 (d, 3H), 1.10 (m, 9H).

### Bsp. 1.40) Herstellung von (RS)-S-[4-({5-Brom-4-[(1R,2R)-2-hydroxy-1-methylpropoxy]pyrimidin-2-yl}amino)phenyl]-N-(ethoxycarbonyl)-S-methylsulfoximid

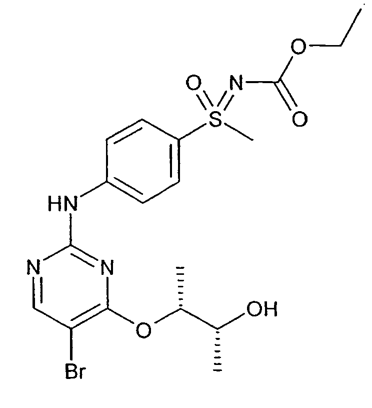

¹H-NMR (DMSO): 10.24 (s, 1 H), 8.45 (s, 1H), 7.97 (m, 2H), 7.85 (m, 2H), 5.22 (m, 1 H), 4.91 (d, 1 H), 3.90 (m, 3H), 3.43 (s, 3H), 1.30 (d, 3H), 1.11 (m, 6H).

### Methode D

### Bsp. 1.41/1.42) Herstellung und Auftrennung in die Diastereomeren von (RS)-S-[4-({5-Brom-4-[(R)-(2-hydroxy-1,2-dimethylpropyl)amino]pyrimidin-2-yl}amino)phenyl]-S-methylsulfoximid (Bsp. 1.3)

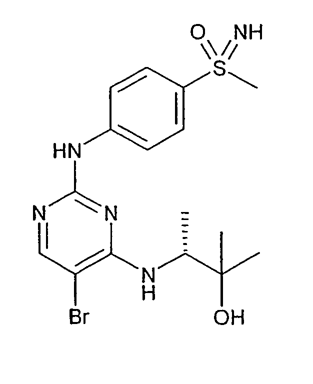

1,65 g (3,30 mmol) (*RS*)-*S*-[4-({5-Brom-4-[(*R*)-(2-hydroxy-1,2-dimethylpropyl)amino]pyrimidin-2-yl}amino)phenyl]-*N*-(ethoxycarbonyl)-S-methylsulfoximid in 6,5 ml Ethanol werden mit 19,1 ml (6,69 mmol) einer 0,35 molaren Lösung von NaOEt in Ethanol versetzt und 5 Stunden unter Rückfluss gerührt. Der Ansatz wird über Nacht bei Raumtemperatur gerührt und anschließend in eine gesättigte NaCl Lösung gegeben. Es wird mit Essigester extrahiert und die vereinten organischen Phasen werden getrocknet (Na₂SO₄), filtriert und eingeengt. Der verbleibende Rückstand wird chromatographisch (DCM / EtOH 9 : 1) gereinigt. Man erhält 0,95 g (2,22 mmol, entsprechend 67 % d. Theorie) des Produktes.

Die analytischen Daten stimmen mit denen von Bsp. 1.3. aus Verfahrensvariante 1, Methode A überein.

Das Diastereomerengemisch wird mittels präperativer HPLC in die Diastereomere gespalten:

| | |
|---|---|
| Säule: | Chiralpak OJ 20µ |
| Länge x ID: | 290 x 50,8 mm |
| Eluenten: | A = Hexan + 0,1% DEA, B = Ethanol |
| Fluss: | 80 ml / min |
| Gradient: | Isokratisch 15% B |
| Detektor: | UV 300nm |
| Temperatur: | Raumtemperatur |
| **RT in min:** | **29,4; Diastereomer 1 (Bsp.1.41)** |
| | **37,1; Diastereomer 2 (Bsp.1.42)** |

In analoger Weise werden hergestellt:

### Bsp. 1.43/1.44) Herstellung und Auftrennung in die Diastereomeren von (RS)-S-[4-({5-Brom-4-[(1R,2R)-(2-hydroxy-1-methylpropyl)amino]pyrimidin-2-yl}amino)phenyl]-S-methylsulfoximid (Bsp. 1.2)

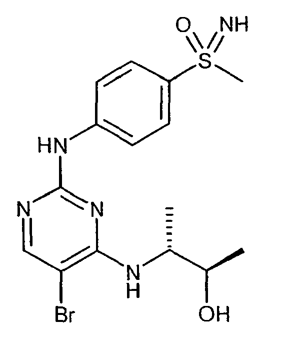

Die analytischen Daten stimmen mit denen von Bsp. 1.2 aus Verfahrensvariante 1, Methode A überein.

Das Diastereomerengemisch wird mittels präperativer HPLC in die Diastereomere gespalten:

| | |
|---|---|
| Säule: | Chiralpak OJ 20µ |
| Länge x ID: | 290 x 50,8 mm |
| Eluenten: | A = Hexan + 0,1 % DEA, B = Ethanol |
| Fluss: | 80 ml / min |
| Gradient: | Isokratisch 15% B |
| Detektor: | UV 280nm |
| Temperatur: | Raumtemperatur |
| **RT in min:** | **44,6; Diastereomer 1 (Bsp.1.43)** |
| | **57,3; Diastereomer 2 (Bsp.1.44)** |

### Bsp. 1.45) Herstellung von (RS)-S-{4-[(5-Brom-4-{[(1R,2R)-2-hydroxy-1-(methoxymethyl)-propyl]amino}pyrimidin-2-yl)amino]phenyl}-S-methylsulfoximid

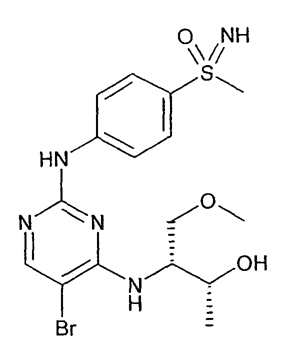

¹H-NMR (DMSO): 9.77 (s, 1 H), 8.14 (s, 1 H), 7.91 (m, 2H), 7.76 (m, 2H), 6.05 (d, 1 H), 5.12 (br, 1 H), 4.20 (m, 1 H), 3.98 (m, 2H), 3.49 (m, 2H), 3.29 (s, 3H), 3.02 (s, 3H), 1.19 (d, 3H).

Das Diastereomerengemisch wird mittels präperativer HPLC in die Diastereomere gespalten:

| | |
|---|---|
| Säule: | Chiralcel OJ 20µ |
| Länge x ID: | 290 x 50,8 mm |
| Eluenten: | Hexan / Ethanol 80 : 20 |
| Fluss: | 80,0 ml / min |
| Detektor: | UV 300 nm |
| Temperatur: | Raumtemperatur |
| **RT in min:** | **47,55 : Diastereomer 1 (Bsp. 1.46)** |
| | **61,02 : Diastereomer 2 (Bsp. 1.47)** |

### Bsp. 1.48) Herstellung von (RS)-S-{4-[(5-Brom-4-{[(1R,2R)-2-hydroxy-1-(methoxymethyl)-propyl]amino}pyrimidin-2-yl)amino]phenyl}-S-ethylsulfoximid

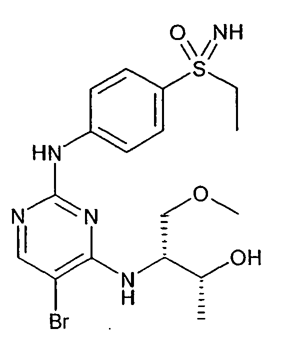

¹H-NMR (DMSO): 9.78 (s, 1 H), 8.14 (s, 1 H), 7.94 (m, 2H), 7.70 (m, 2H), 6.05 (d, 1 H), 5.11 (d, 1 H), 4.19 (m, 1 H), 3.97 (m, 2H), 3.50 (m, 2H), 3.30 (s, 3H), 3.05 (q, 2H), 1.07 (m, 6H).

Das Diastereomerengemisch wird mittels präperativer HPLC in die Diastereomere gespalten:

| | |
|---|---|
| Säule: | Chiralcel OJ 20µ |
| Länge x ID: | 290 x 50,8 mm |
| Eluenten: | Hexan : Ethanol 80 : 20 |
| Fluss: | 80 ml / min |
| Detektor: | UV 300 nm |
| Temperatur: | Raumtemperatur |
| **RT in min:** | **45,5 : Diastereomer 1 (Bsp. 1.49)** |
| | **53,1 : Diastereomer 2 (Bsp. 1.50)** |

### Bsp. 1.51) Herstellung von (RS)-S-[4-({5-Brom-4-[(1R,2R)-(2-hydroxy-1-methylpropyl)amino]pyrimidin-2-yl}amino)phenyl]-S-ethylsulfoximid

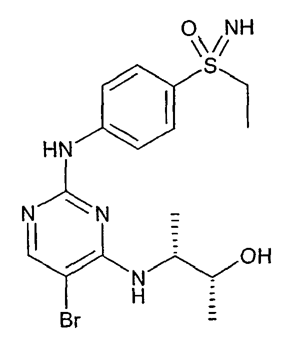

¹H-NMR (DMSO): 9.71 (s, 1 H), 8.11 (s, 1 H), 7.90 (m, 2H), 7.71 (m, 2H), 6.13 (d, 1H), 5.01 (d, 1 H), 4.08 (m, 1 H), 3.93 (s, 1 H), 3.78 (m, 1 H), 3.03 (q, 2H), 1.22 (d, 3H), 1.10 (m, 6H).

Das Diastereomerengemisch wird mittels präperativer HPLC in die Diastereomere gespalten:

| | |
|---|---|
| Säule: | Chiracel OJ 20µ |
| Länge x ID: | 250 x 50,8 mm |
| Eluenten: | A: Hexan + 0,1% DEA; B: Ethanol |
| Fluss: | 80 ml / min |
| Gradient: | Isokratisch 15% B |
| Detektor: | UV 300 nm |
| Temperatur: | Raumtemperatur |
| **RT in min:** | **34,0 : Diastereomer 1 (Bsp. 1.52)** |
| | **43,7 : Diastereomer 2 (Bsp. 1.53)** |

### Bsp. 1.54) Herstellung von (RS)-S-[4-({5-Brom-4-[(R)-(2-hydroxy-1,2-dimethylpropyl)amino]pyrimidin-2-yl}amino)phenyl]-S-ethylsulfoximid

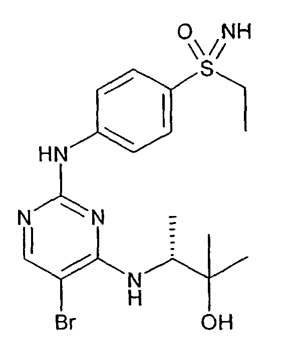

¹H-NMR (DMSO): 9.74 (s, 1H), 8.13 (s, 1H), 7.92 (m, 2H), 7.71 (m, 2H), 6.09 (d, 1 H), 4.84 (s, 1 H), 4.08 (m, 1 H), 3.92 (s, 1 H), 3.06 (q, 2H), 1.15 (m, 12H).

Das Diastereomerengemisch wird mittels präperativer HPLC in die Diastereomere gespalten:

| | |
|---|---|
| Säule: | Chiralpak AD 20µ |
| Länge x ID: | 250 x 60 mm |
| Eluenten: | Hexan / 2-Propanol 80 : 20 |
| Fluss: | 80 / 100 ml/min |
| Detektor: | UV 280 nm |
| Temperatur: | Raumtemperatur |
| **RT in min:** | **222,2 : Diastereomer 1 (Bsp. 1.55)** |
| | **249,8 : Diastereomer 2 (Bsp. 1.56)** |

### Bsp. 1.57) Herstellung von (RS)-S-[4-({5-Brom-4-[(R)-(2-hydroxy-1,2-dimethylpropyl)amino]pyrimidin-2-yl}amino)-2-methylphenyl]-S-methylsulfoximid

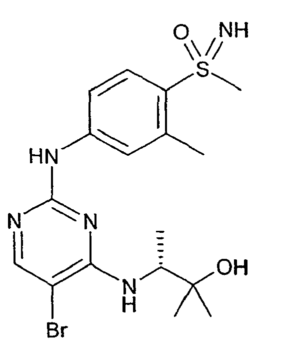

¹H-NMR (DMSO): 9.63 (s, 1 H), 8.11 (s, 1 H), 7.81 (m, 2H), 7.63 (m, 1 H), 6.08 (d, 1 H), 4.88 (s, 1 H), 4.06 (m, 2H), 3.03 (s, 3H), 2.67 (s, 3H), 1.2 (m, 9H).
MS: 442 (ES).

Das Diastereomerengemisch wird mittels präperativer HPLC in die Diastereomere gespalten:

| | |
|---|---|
| Säule: | Chiralpak AS 20µ |
| Länge x ID: | 250 x 50,8 mm |
| Eluenten: | A = Hexan, B = Ethanol |
| Fluss: | 80 ml / min |
| Gradient: | Isokratisch 15% B |
| Detektor: | UV 300nm |
| Temperatur: | Raumtemperatur |
| **RT in min:** | **18,96; Diastereomer 1 (Bsp.1.58)** |
| | **21,56; Diastereomer 2 (Bsp.1.59)** |

### Bsp. 1.60) Herstellung von (RS)-S-[4-({5-Brom-4-[(1R,2R)-(2-hydroxy-1-methylpropyl)amino]pyrimidin-2-yl}amino)-2-methylphenyl]-S-methylsulfoximid

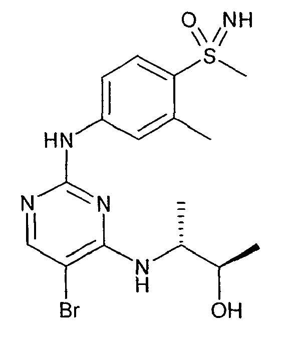

¹H-NMR (DMSO): 9.62 (s, 1 H), 8.11 (s, 1 H), 7.82 (m, 2H), 7.66 (m, 1 H), 6.14 (d, 1 H), 5.02 (d, 1 H), 4.04 (m, 2H), 3.80 (m, 1 H), 3.03 (s, 3H), 2.65 (s, 3H), 1.22 (d, 3H), 1.10 (d, 3H).

Das Diastereomerengemisch wird mittels präperativer HPLC in die Diastereomere gespalten:

| | |
|---|---|
| Säule: | Chiralpak AD 20µ |
| Länge x ID: | 250 x 50,8 mm |
| Eluenten: | A: Hexan + 0,1 % DEA, B: Ethanol |
| Fluss: | 80 ml / min |
| Gradient: | Isokratisch 25%B |
| Detektor: | UV 280nm |
| Temperatur: | Raumtemperatur |
| **RT in min:** | **104, Diastereomer 1 (Bsp. 1.61)** |
| | **124, Diastereomer 2 (Bsp. 1.62)** |

### Bsp. 1.63) Herstellung von (RS)-S-{4-[(5-Brom-4-ethoxypyrimidin-2-yl)amino]phenyl}-S-ethylsulfoximid

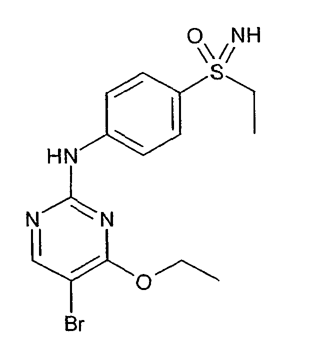

28 mg (0,056 mmol) (*RS*)-*S*-[4-({5-Brom-4-[(1*R*,2*R*)-2-hydroxy-1-methylpropoxy]pyrimidin-2-yl}amino)phenyl]-*N*-(ethoxycarbonyl)-S-methylsulfoximid in 0,11 ml Ethanol werden mit 0,32 ml (0,113 mmol) einer 0,35 molaren Lösung von NaOEt in Ethanol versetzt und 6 Stunden unter Rückfluss gerührt. Der Ansatz wird über Nacht bei Raumtemperatur gerührt und anschließend in eine gesättigte NaCl Lösung gegeben. Es wird mit Essigester extrahiert und die vereinten organischen Phasen werden getrocknet (Na₂SO₄), filtriert und eingeengt. Der verbleibende Rückstand wird chromatographisch (DCM / EtOH 85 : 15) gereinigt. Man erhält 9 mg (0,023 mmol, entsprechend 42 % d. Theorie) des Produktes.

¹H-NMR (DMSO): 10.17 (s, 1 H), 8.45 (s, 1 H), 7.94 (m, 2H), 7.78 (m, 2H), 4.49 (q, 2H), 3.98 (s, 1 H), 3.07 (q, 2H), 1.40 (tr, 3H), 1.04 (tr, 3H).
MS: 385 (ES).

### Bsp. 1.64) Herstellung von (RS)-N-(Ethoxycarbonyl)-S-(4-{[5-iod-4-(prop-2-in-1-ylamino)pyrimidin-2-yl]amino}phenyl)-S-methylsulfoximid

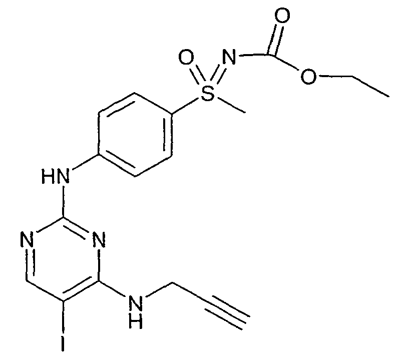

400 mg (1,65 mmol) (2-Chlor-5-iodpyrimidin-4-yl)-prop-2-in-1-yl-amin und 630 mg (2,15 mmol) (RS)-S-(4-Aminophenyl)-N-(ethoxycarbonyl)-S-methylsulfoximid in 7 ml Acetonitril werden mit 0,6 ml einer 4 N Lösung von HCl in Dioxan und 1 ml Wasser versetzt. Der Ansatz wird 24 Stunden bei 50 °C gerührt. Das Lösungsmittel wird abgezogen und der verbleibende Rückstand chromatographisch (DCM / EtOH 9 : 1) gereinigt. Man erhält 279 mg (0,56 mmol, entsprechend 54 % d. Theorie) des Produktes.

¹H-NMR (DMSO): 10.19 (s, 1H), 8.30 (s, 1H), 8.05 (m, 2H), 7.81 (m, 2H), 7.59 (br, 1H), 4.17 (d, 2H), 3.88 (q, 2H), 3.43 (s, 3H), 3.18 (br, 1H), 1.10 (tr, 3H).
MS: 500 (ES).

### Bsp. 1.65) Herstellung von (RS)-N-(Ethoxycarbonyl)-S-{4-[(4-{(R)-[1-(hydroxymethyl)-2-methylpropyl]amino}-5-iodpyrimidin-2-yl) amino]phenyl}-S-methylsulfoximid

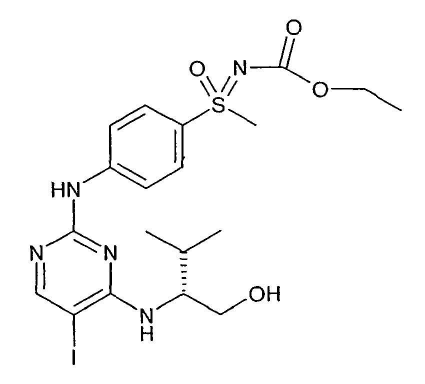

¹H-NMR (DMSO): 9.81 (s, 1 H), 8.22 (s, 1 H), 7.98 (m, 2H), 7.78 (m, 2H), 5.89 (d, 1 H), 4.85 (tr, 1 H), 4.04 (m, 1 H), 3.92 (q, 2H), 3.65 (m, 1 H), 3.56 (m, 1 H), 3.41 (s, 3H), 2.02 (m, 1 H), 1.10 (tr, 3H), 0.95 (dd, 6H).
MS: 548 (ES)

### Bsp. 1.66) Herstellung von (RS)-S-{4-[(4-{(R)-[1-(hydroxymethyl)-2-methylpropyl]amino}-5-iodpyrimidin-2-yl)amino]phenyl}-S-methylsulfoximid

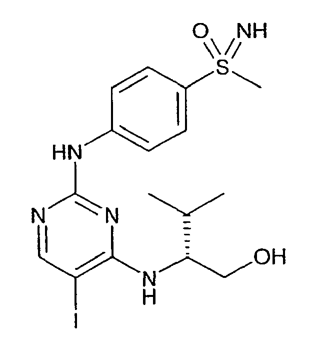

¹H-NMR (DMSO): 9.68 (s, 1 H), 8.21 (s, 1 H), 7.92 (m, 2H), 7.75 (m, 2H), 5.87 (d, 1 H), 4.86 (tr, 1 H), 4.01 (m, 2H), 3.66 (m, 1 H), 3.55 (m, 1 H), 3.01 (s, 3H), 2.02 (m, 1 H), 0.94 (m, 6H).

### Bsp. 1.67) Herstellung von Herstellung von (RS)-S-[4-({5-Brom-4-[(1R,2R)-(2-hydroxy-1-methylpropyl)amino]pyrimidin-2-yl}amino)-2-fluorphenyl]-N-(ethoxycarbonyl)-S-methylsulfoximid

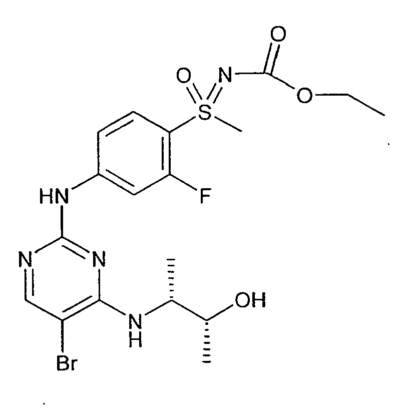

¹H-NMR (DMSO): 10.08 (s, 1 H), 8.18 (s, 1 H), 8.02 (m, 1 H), 7.68 (m, 2H), 6.27 (d, 1 H), 5.03 (br, 1 H), 4.08 (m, 1 H), 3.88 (m, 2H), 3.79 (m, 1 H), 3.48 (s, 3H), 1.21 (d, 3H), 1.09 (m, 6H).
MS: 504 (ES).

### Bsp. 1.68) Herstellung von (RS)-S-[4-({5-Brom-4-[(R)-(2-hydroxy-1,2-dimethylpropyl)amino]pyrimidin-2-yl}amino)-2-fluorphenyl]-N-(ethoxycarbonyl)-S-methylsulfoximid

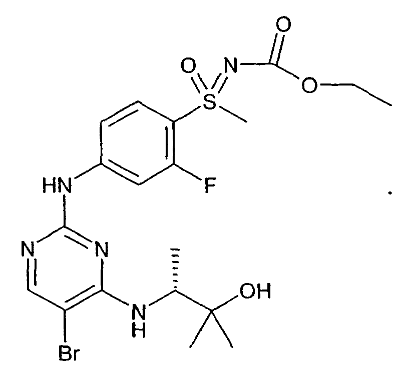

¹H-NMR (DMSO): 10.12 (s, 1H), 8.17 (s, 1H), 8.02 (m, 1 H), 7.73 (m, 1H), 7.63 (m, 1 H), 6.26 (d, 1 H), 4.08 (m, 1 H), 3.85 (m, 2H), 3.42 (s, 3H), 1.11 (m, 12H). MS: 518 (ES).

### Bsp. 1.69) Herstellung von (RS)-S-[4-({5-Brom-4-[(R)-(2-hydroxy-1,2-dimethylpropyl)amino]pyrimidin-2-yl}amino)-2-trifluormethylphenyl]-N-(ethoxycarbonyl)-S-methylsulfoximid

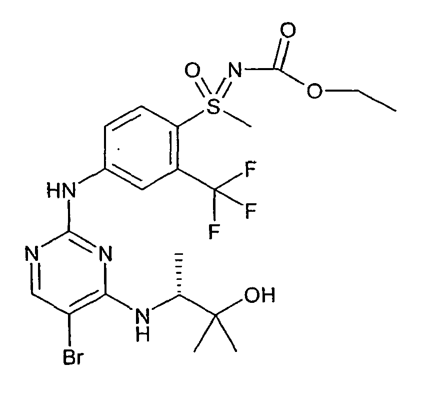

¹H-NMR (DMSO): 10.21 (s, 1 H), 8.65 (s, 1 H), 8.19 (s, 1 H), 8.05 (s, 2H), 6.18 (d, 1 H), 4.90 (br, 1 H), 4.05 (m, 1 H), 3.89 (q, 2H), 3.40 (s, 3H), 1.12 (m, 12H).
MS: 568 (ES).

### Verfahrensvariante 2

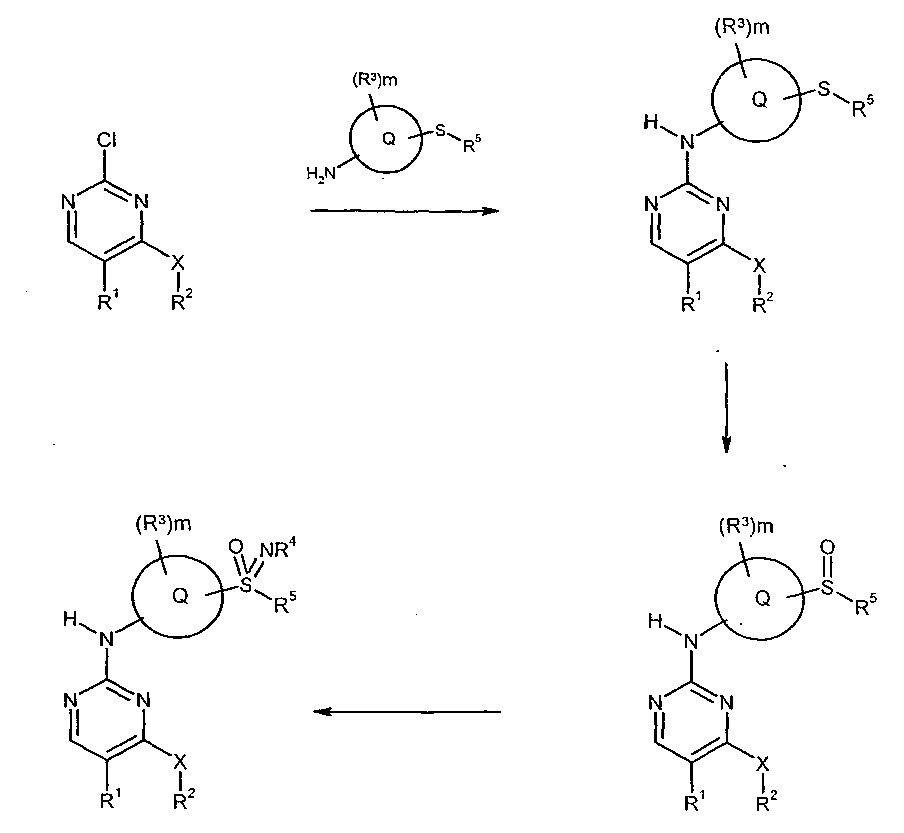

Die Substituenten R¹, R², R³, R⁴, R⁵, Q,und m haben die in der allgemeinen Formel (I) angegebene Bedeutung.

### Methode A

### Bsp. 2.0) Herstellung von (RS)-S-(4-{[5-Brom-4-(isopropylamino)pyrimidin-2-yl]amino}phenyl)-S-methylsulfoximid

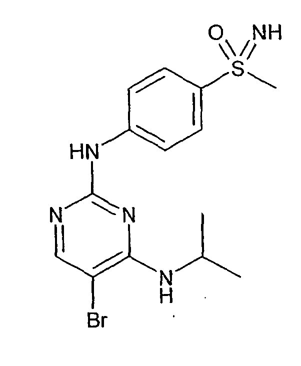

185 mg (0, 50 mmol) (RS)-5-Brom-*N*⁴-isopropyl-*N*²-[4-(methylsulfinyl)phenyl]-pyrimidin-2,4-diamin in 1 ml DCM werden mit 40 mg (0,55 mmol) Natriumazid versetzt. Der Ansatz wird bei 0 °C langsam mit 0,13 ml konzentrierter Schwefelsäure versetzt und anschliessend auf 45 °C erwärmt. Nach 16 h wird der Ansatz auf Raumtemperatur abgekühlt, mit 2 ml 1 N NaOH Lösung versetzt und gegen Essigester extrahiert. Die vereinten organischen Phasen werden getrocknet (Na₂SO₄), filtriert und eingeengt. Der verbleibende Rückstand wird chromatographisch (DCM / EtOH 9:1) gereinigt. Man erhält 38 mg (0,10 mmol, entsprechend 20 % d. Theorie) des Produktes.

¹H-NMR (DMSO): 9.70 (s, 1 H), 8.08 (s, 1 H), 7.90 (d, 2H), 7.77 (d, 2H), 6.62 (d, 1 H), 4.35 (m, 1 H), 3.99 (s, 1 H), 3.03 (s, 3H), 1.29 (d, 6H).
MS: 384 (ES).

### Methode B

### Bsp. 2.1) Herstellung von (RS)-S-[4-({5-Brom-4-[(R)-(2-hydroxy-1-methylethyl)amino]pyrimidin-2-yl}amino)phenyl]-S-methyl-N-[2-(trimethylsilyl)ethylsulfonyl]sulfoximid

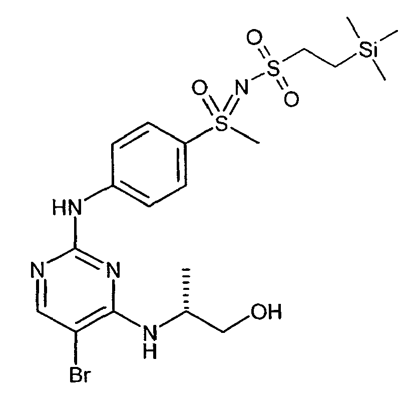

50 mg (0,13 mmol) (R)-2-[5-Brom-2-{(RS)-4-methylsulfinyl-phenylamino}-pyrimidin-4-ylamino]-propan-1-ol in 3 ml Acetonitril werden mit einer Spatelspitze CuPF₆[CH₃CN]₄ (ca. 0,05 equiv.) versetzt und 30 Minuten bei Raumtemperatur gerührt. Die Mischung wird im Eisbad gekühlt, mit 55 mg (0,13 mmol) [N-(2-(Trimethylsilyl)ethanesulfonyl)imino]phenyliodinane versetzt und 4 Stunden bei Raumtemperatur gerührt. Es wird nochmals im Eisbad gekühlt, mit einer Spatelspitze CuPF₆[CH₃CN]₄ und mit 22 mg (0,06 mmol) [N-(2-(Trimethylsilyl)ethanesulfonyl)imino]phenyliodinane versetzt und weitere 3 Stunden bei Raumtemperatur gerührt. Die Mischung wird zur Trockene eingeengt und der verbleibende Rückstand chromatographisch gereinigt. Man erhält 20 mg (RS)-S-[4-({5-Brom-4-[(R)-(2-hydroxy-1-methylethyl)amino]-pyrimidin-2-yl}amino)phenyl]-S-methyl-N-[2-(trimethylsilyl)ethylsulfonyl]-sulfoximid vom Schmelzpunkt 194-197 °C.
¹H-NMR (DMSO): 9.92 (s, 1H), 8.14 (s, 1 H), 8.02 (d, 2H), 7.87 (d, 2H), 6.48 (d, 1 H), 4,90 (t, 1 H), 4. 27 (m, 1 H), 3.53 (s, 3H), 3.52 (m, 2H), 2.95 (m, 2H), 1.22 (d, 3H), 0.95 (m, 2H), 0.01 (s, 9H).
MS: 564/566 (100 %, ES).
Analog zu den vorgenannten Methoden A und B der Verfahrensvariate 2 werden folgende Verbindungen hergestellt:

### Bsp. 1.0) Herstellung von (RS)-S-[4-({5-Brom-4-[(R)-(2-hydroxy-1-methylethyl)amino]pyrimidin-2-yl}amino)phenyl]-S-methylsulfoximid

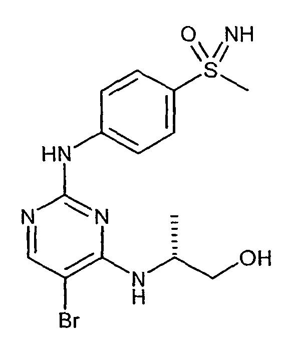

Nach Abspaltung der Ses-Schutzgruppe mit Tetrabutylammoniumfluorid analog Beispiel 1.6 (wie in Tetrahedron Lett. 2002, 43, 2751 beschrieben), erhält man 10 mg (0.02 mmol, entsprechend 70% d. Theorie) des Produktes.

### Bsp. 2.2) Herstellung von (RS)-S-(4-{[5-Brom-4-(phenylamino)pyrimidin-2-yl]amino}phenyl)-S-methyl-N-[2-(trimethylsilyl)ethylsulfonyl]sulfoximid

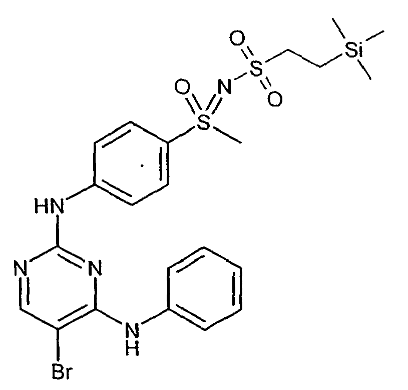

¹H-NMR (DMSO): 9.98 (s, 1H), 8.83 (s, 1H), 8.32 (s, 1H), 7.88 (d, 2H), 7.71 (d, 2H), 7.59 (d, 2H), 7.44 (t, 2H), 7.23 (t, 1 H), 3.53 (s, 3H), 2.86-3.03 (m, 2H), 0.82-1.01 (m, 2H), 0.00 (s, 9H).
MS: 582/584 (100 %, ES).

### Bsp. 2.3) Herstellung von (RS)-S-(4-{[5-Brom-4-(phenylamino)pyrimidin-2-yl]amino}phenyl)-S-methylsulfoximid

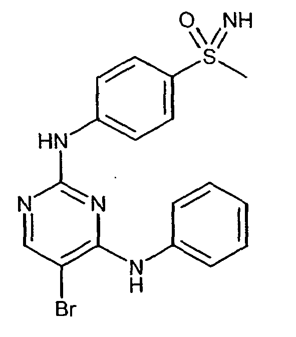

¹H-NMR (DMSO): 9.82 (s, 1 H), 8.79 (s, 1 H), 8.30 (s, 1 H), 7.78 (d, 2H), 7.65 (d, 2H), 7.60 (d, 2H), 7.42 (t, 2H), 7.23 (t, 1 H), 3.96 (s, 1 H), 3.00 (s, 3H).
MS: 418/420 (20 %, ES).

### Bsp. 2.4) Herstellung von (RS)-S-[4-({4-[(2-Fluor-5-methylphenyl)amino]pyrimidin-2-yl}amino)phenyl]-S-methyl-N-[2-(trimethylsilyl)ethylsulfonyl]sulfoximid

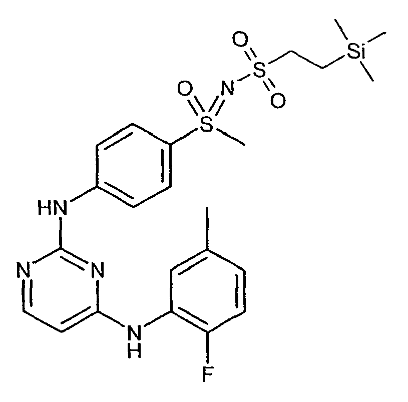

¹H-NMR (DMSO): 9.85 (s, 1H), 9.22 (s, 1H), 7.98 (d, 2H), 7.88 (d, 1H), 7.76 (d, 2H), 7.63 (d, 1 H), 7.21 (m, 1 H), 7.02 (m, 1 H), 6.40 (m, 1 H), 3.53 (s, 3H), 2.81-2.90 (m, 2H), 0.87-1.00 (m, 2H), 0.00 (s, 9H).
MS: 536 (100 %, ES).

### Bsp. 2.5) Herstellung von (RS)-S-[4-({4-[(2-Fluor-5-methylphenyl)amino]-pyrimidin-2-yl}amino)phenyl]-S-methylsulfoximid

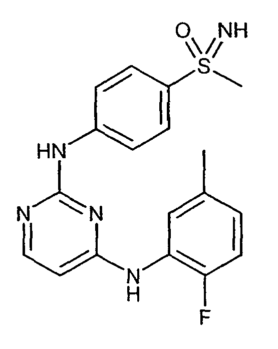

¹H-NMR (DMSO): 9.65 (s, 1 H), 9.18 (s, 1 H), 8.09 (d, 1 H), 7.87 (d, 2H), 7.69 (d, 2H), 7.65 (d, 1 H), 7.19 (m, 1 H), 7.02 (m, 1 H), 6.37 (m, 1H), 3.02 (s, 3H).
MS: 372 (10 %, ES).

### Verfahrensvariante 3

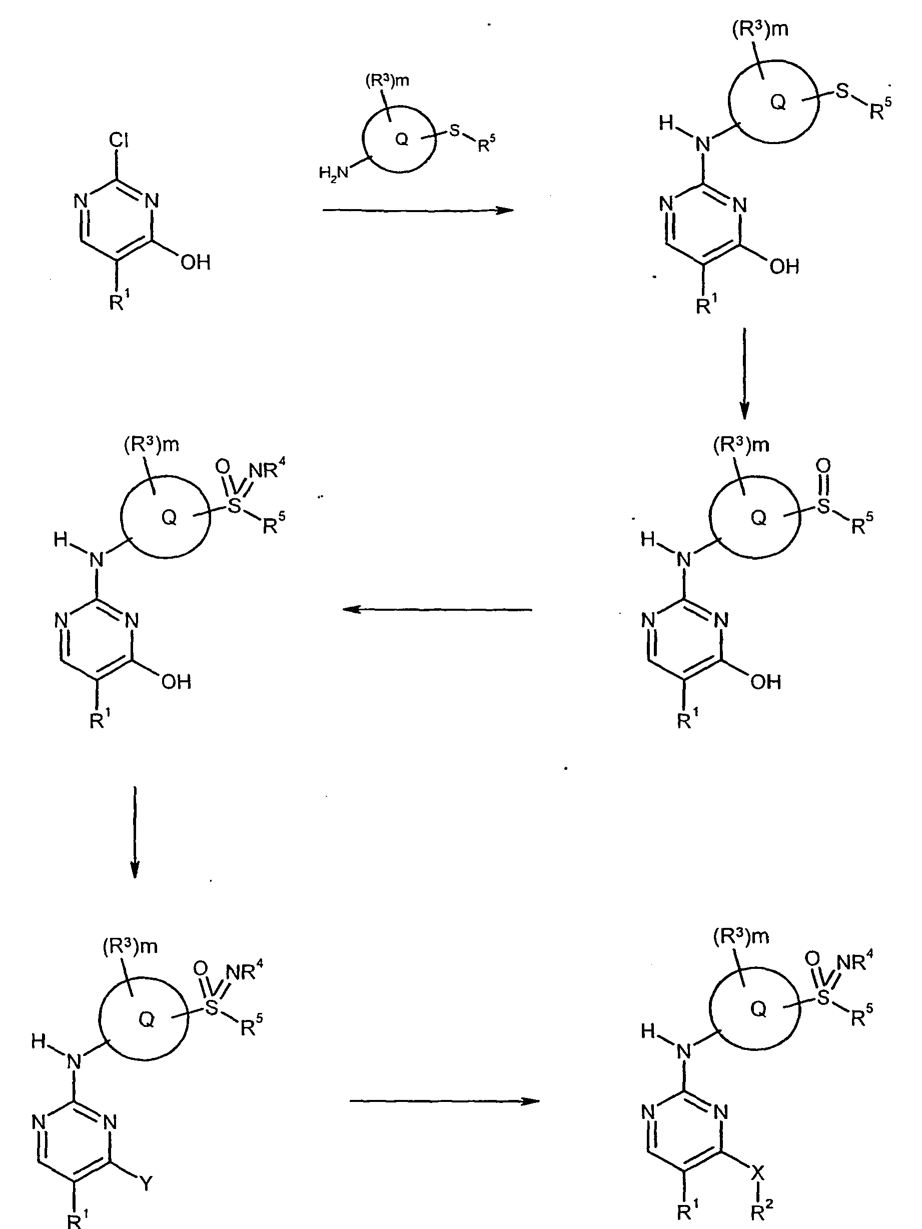

Die Substituenten R¹, R², R³, R⁴, R⁵, Q,und m haben die in der allgemeinen Formel (I) angegebene Bedeutung. Y hat die Bedeutung von Halogen.

### Bsp. 3.0) Herstellung von (RS)-S-[4-({5-Brom-4-[(RS)-(1-hydroxymethylpropyl)sulfanyl]pyrimidin-2-yl}amino)phenyl]-S-methylsulfoximid

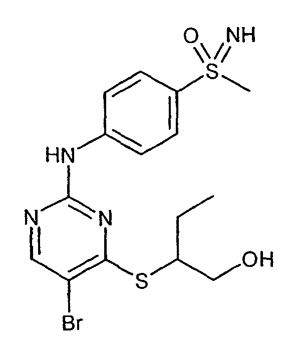

362 mg (RS)-S-{4-[(5-Brom-4-chlorpyrimidin-2-yl)amino]phenyl}-S-methylsulfoximid werden in 1,5 ml Dimethylformamid gelöst, mit 0,5 ml Triethylamin und 320 mg (RS)-2-Mercapto-butan-1-ol versetzt und 18 Stunden bei Raumtemperatur gerührt. Die Mischung wird im Vakuum zur Trockene eingeengt und durch Flash-Chromatografie (Dichlormethan/Ethanol) gereinigt. Man erhält 255 mg des Produktes vom Schmelzpunkt 175-180 °C.

¹H-NMR (DMSO): 10.18 (s, 1 H), 8.39 (s, 1 H), 7.91 (d, 2H), 7.83 (d, 2H), 5.13 (t, 1 H), 4.05 (s, 1 H), 4.00 (m, 1H), 3.74 (m, 1 H), 3.63 (m, 1 H), 3.03 (s, 3H), 1.93 (m, 1 H), 1.69 (m, 1 H), 1.00 (t, 3H).
MS: 431/433 (95/100 %, ES).

In analoger Weise werden die folgenden Beispiele hergestellt:

### Bsp. 3.1) Herstellung von (RS)-S-[4-({5-Brom-4-[(RS)-(1-methylpropyl)sulfanyl]pyrimidin-2-yl}amino)phenyl]-S-methylsulfoximid

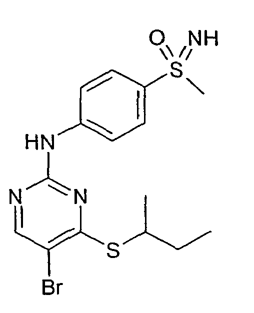

Schmelzpunkt: 175-183 °C
¹H-NMR (DMSO): 10.19 (s, 1H), 8.40 (s, 1H), 7.95 (d, 2H), 7.85 (d, 2H), 4.04 (s, 1 H), 3.97 (m, 1H), 3.03 (s, 3H), 1.76 (m, 2H), 1.42 (d, 2H), 1.01 (t, 3H).
MS: 415/417 (90/100 %, ES).

### Bsp. 3.2) Herstellung von (RS)-S-[4-({5-Brom-4-[(RS)-(1-methyl-2-oxo-propyl)sulfanyl]pyrimidin-2-yl}amino)phenyl]-S-methylsulfoximid

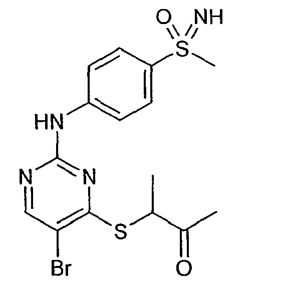

¹H-NMR (DMSO): 10.18 (s, 1 H), 8.44 (s, 1 H), 7.87 (s, 4H), 4.82 (q, 1 H), 3.06 (s, 3H), 2.26 (s, 3H), 1.52 (d, 3H).
MS: 429/431 (90/100 %, ES).

### Bsp. 3.3) Herstellung von (RS)-S-[4-({4-[(RS)-(1-Acetylpropyl)sulfanyl]-5-brompyrimidin-2-yl}amino)phenyl]-S-methylsulfoximid

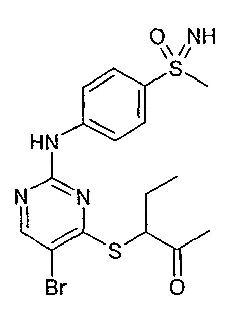

¹H-NMR (DMSO): 10.16 (s, 1 H), 8.44 (s, 1 H), 7.86 (s, 4H), 4.79 (t, 1 H), 3.04 (s, 3H), 2.25 (s, 3H), 2.04 (m, 1H), 1.89 (m, 1 H), 1.18 (t, 1.5H), 0.96 (t, 1.5H).
MS: 443/445 (90/100 %, ES).

### Bsp. 3.4) Herstellung von (RS)-S-[4-({5-Brom-4-[(RS)-(2-hydroxypropyl)sulfanyl]pyrimidin-2-yl}amino)phenyl]-S-methylsulfoximid

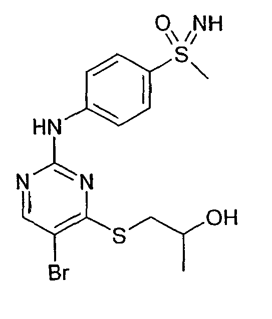

¹H-NMR (DMSO): 10.17 (s, 1 H), 8.39 (s, 1 H), 7.90 (d, 2H), 7.84 (d, 2H), 5.04 (d, 1 H), 4.04 (s, 1 H), 3.93 (m, 1 H), 3.26 (d, 1 H), 3.03 (s, 3H), 1.21 (d, 3H).
MS: 417/419 (90/100 %, ES).

### Bsp. 3.5) Herstellung von (RS)-S-[4-({5-Brom-4-[(RS, RS)-(2-hydroxy-1-methylpropyl)sulfanyl]pyrimidin-2-yl}amino)phenyl]-S-methylsulfoximid

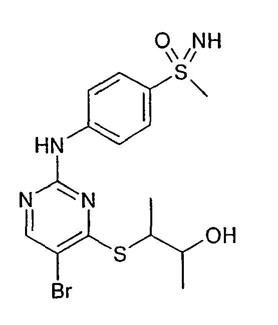

¹H-NMR (DMSO): 10.17 (s, 1 H), 8.38 (s, 1 H), 7.93-7.81 (m, 4H), 5.13+5.06 (d, 1 H), 4.06 (m, 1 H), 4.04 (s, 1 H),3.95 (m, 1 H), 3.03 (s, 3H), 1.42+1.36 (d, 3H), 1.18 (m, 3H).
MS: 431/433 (94/100 %, ES).

### Bsp. 3.6) Herstellung von (RS)-S-[4-({5-Birom-4-[(RS, RS)-(1-ethyl-2-hydroxypropyl)sulfanyl]pyrimidin-2-yl}amino)phenyl]-S-methylsulfoximid

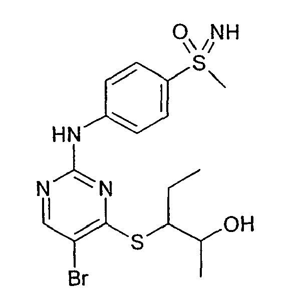

Wird durch Umsetzung von (*RS*)-*S*-[4-({4-[(R*S*)-(1-Acetylpropyl)sulfanyl]-5-brompyrimidin-2-yl}amino)phenyl]-*S*-methylsulfoximid mit 1 Äquivalent Natriumborhydrid in Tetrahydrofuran/Methanol (1:1) erhalten.
Schmelzpunkt: 192-194 °C
¹H-NMR (DMSO): 10.14 (s, 1 H), 8.38 (s, 1 H), 7.90 (d, 2H), 7.83 (d, 2H), 5.06+4.98 (d, 1 H), 4.08 (s, 1 H), 4.00 (m, 2H), 3.03 (s, 3H), 1.93 (m, 1 H), 1.66 (m, 1H), 1.16 (d, 3H), 0.99 (t, 3H).
MS: 445/447 (96/100 %, ES).

### Bsp. 3.7) Herstellung von (RS)-S-[4-({5-Brom-4-[(RS)-(2-hydroxy-1,2-dimethylpropyl)sulfanyl]pyrimidin-2-yl}amino)phenyl]-S-methylsulfoximid

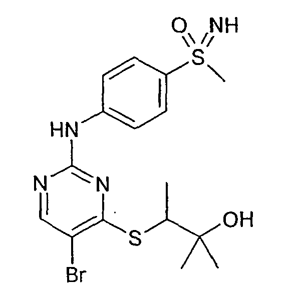

Wird durch Umsetzung von (RS)-S-[4-({5-Brom-4-[(RS)-(1-methyl-2-oxo-propyl)-sulfanyl]pyrimidin-2-yl}amino)phenyl]-S-methylsulfoximid mit 6 Äquivalenten Methylmagnesiumbromid in Tetrahydrofuran erhalten.

Schmelzpunkt: 201-202 °C
¹H-NMR (DMSO): 10.18 (s, 1 H), 8.38 (s, 1 H), 7.92 (d, 2H), 7.83 (d, 2H), 4.89 (s, 1 H), 4.09 (m, 1 H), 4.05 (s, 1 H), 3.03 (s, 3H), 1.43 (d, 3H), 1.27 (s, 6H).
MS: 445/447 (93/100 %, ES).

### Bsp. 3.8) Herstellung von (RS)-S-[4-({5-Brom-4-[(RS)-(1-ethyl-2-hydroxy-2-methylpropyl)sulfanyl]pyrimidin-2-yl}amino)phenyl]-S-methylsulfoximid

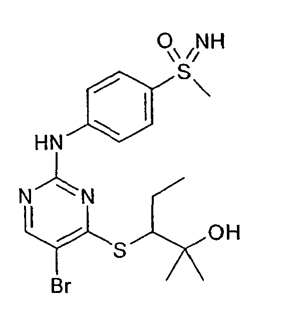

Wird durch Umsetzung von (*RS*)-*S*-[4-({4-[(*RS*)-(1-Acetylpropyl)sulfanyl]-5-brompyrimidin-2-yl}amino)phenyl]-*S*-methylsulfoximid mit 6 Äquivalenten Methylmagnesiumbromid in Tetrahydrofuran erhalten.
Schmelzpunkt: 218 °C (Zersetzung)
¹H-NMR (DMSO): 10.17 (s, 1 H), 8.38 (s, 1 H), 7.92 (d, 2H), 7.83 (d, 2H), 4.78 (s, 1H), 4.12 (dd, 1H), 4.05 (s, 1 H), 3.03 (s, 3H), 2.10 (m, 1 H), 1.48 (m, 1 H), 1.24 (s, 6H), 0.95 (dd, 3H).
MS: 459/461 (93/100 %, ES).

### Bsp. 3.9) Herstellung von (RS)-S-[4-({5-Brom-4-[(2-hydroxy-2-methylpropyl)sulfanyl]pyrimidin-2-yl}amino)phenyl]-S-methylsulfoximid

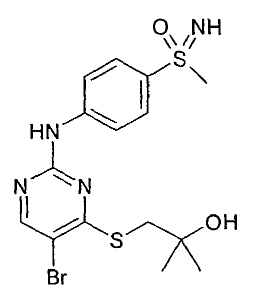

Wird durch Umsetzung von (RS)-S-[4-({5-Brom-4-[(4-methoxycarbonylmethyl)-sulfanyl]pyrimidin-2-yl}amino)phenyl]-S-methylsulfoximid mit 6 Äquivalenten Methylmagnesiumbromid in Tetrahydrofuran erhalten.

¹H-NMR (DMSO): 10.17 (s, 1 H), 8.39 (s, 1 H), 7.92 (d, 2H), 7.83 (d, 2H), 4.84 (s, 1 H), 4.05 (s, 1 H), 3.41 (s, 2H), 3.03 (s, 3H), 1.26 (s, 6H).
MS: 431/433 (94/100 %, ES).

### Bsp. 3.10) Herstellung von (RS)-S-[4-({5-Brom-4-[(RS)-(2-hydroxy-1-methyl-ethyl)sulfanyl]pyrimidin-2-yl}amino)phenyl]-S-methylsulfoximid

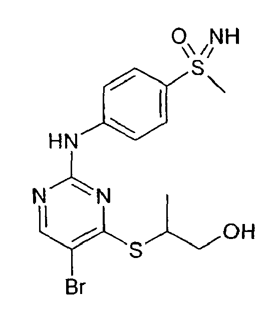

Schmelzpunkt: 218-220 °C
¹H-NMR (DMSO): 10.19 (s, 1 H), 8.40 (s, 1 H), 7.92 (d, 2H), 7.84 (d, 2H), 5.18 (t, 1 H), 4.07 (m, 2H), 3.69 (m, 1 H), 3.61 (m, 1 H), 3.04 (s, 3H), 1.42 (d, 3H).
MS: 417/419 (92/100 %, ES).

### Bsp. 3.11) Herstellung von (RS)-S-[4-({5-Brom-4-[(4-methoxycarbonylmethyl)sulfanyl]pyrimidin-2-yl}amino)phenyl]-S-methylsulfoximid

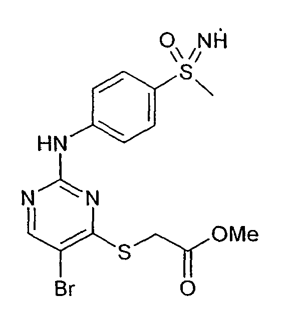

¹H-NMR (DMSO): 10.22 (s, 1 H), 8.44 (s, 1 H), 7.82 (s, 4H), 4.22 (s, 2H), 4.16 (s (br), 1 H), 3.58 (s, 3H), 3.05 (s, 3H).
MS: 431/433 (91/100 %, ES).

### Bsp. 3.12/3.13) Herstellung und Auftrennung in die Diastereomeren von (RS)-S-[4-({5-Brom-4-[(1R,2R)-2-hydroxy-1-methylpropoxy]pyrimidin-2-yl}amino)phenyl]-S-methylsulfoximid (Bsp. 1.4)

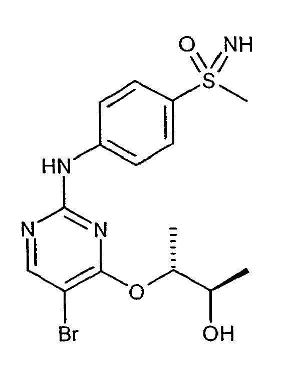

Eine Lösung von 674 mg (7,5 mmol) (R,R)-(-)-2,3-Butandiol in 6 ml DMSO wird unter Wasserkühlung portionsweise mit 330 mg Natriumhydrid (55-60%ig) versetzt und anschliessend 45 Minuten bei Raumtemperatur gerührt. Der Ansatz wird mit 196 mg (0,54 mmol) (RS)-S-{4-[(5-Brom-4-chlorpyrimidin-2-yl)amino]-phenyl}-S-methylsulfoximid in 0,5 ml DMSO versetzt und über Nacht gerührt. Man versetzt erneut mit 191 mg (0,53 mmol) (RS)-S-{4-[(5-Brom-4-chlorpyrimidin-2-yl)amino]phenyl}-S-methylsulfoximid in 0,5 ml DMSO und rührt weitere zwei Stunden. Abschliessend wird mit 190 mg (RS)-S-{4-[(5-Brom-4-chlorpyrimidin-2-yl)amino]phenyl}-S-methylsulfoximid (0,53 mmol) in 0,5 ml DMSO versetzt und eine Stunde gerührt. Der Ansatz wird auf Eiswasser gegeben und gegen Essigester extrahiert (4x). Die vereinten organischen Phasen werden mit NaCl Lösung gewaschen, über einen Whatman Filter filtriert und eingeengt. Der verbleibende Rückstand wird chromatographisch (DCM / EtOH 9 : 1) gereinigt. Man erhält 166 mg (0,41 mmol, entsprechend 25 % d. Theorie) des Produktes.
Die analytischen Daten stimmen mit denen aus der Darstellung nach Verfahrensvariante 1 überein.
Das Diastereomerengemisch wird mittels präperativer HPLC in die Diastereomere gespalten:

| | |
|---|---|
| Säule: | Chiracel OJ 20µ |
| Länge x ID: | 290 x 50,8 mm |
| Eluenten: | A: Hexan, B: Ethanol |
| Fluss: | 80 ml/min |
| Gradient: | Isokratisch 15%B |
| Detektor: | UV 300 nm |
| Temperatur: | Raumtemperatur |
| **RT in min:** | **32,8 : Diastereomer 1 (Bsp. 3.12)** |
| | **39,2 : Diastereomer 2 (Bsp. 3.13)** |

### Herstellung der Zwischenprodukte

### a) Herstellung von (RS)-S-(4-Aminophenyl)-S-methylsulfoximid

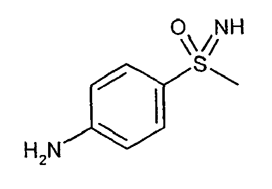

Eine Lösung von 2,45 g (12,2 mmol) (RS)-S-(4-Nitrophenyl)-S-methylsulfoximid in 150 ml Ethanol wird bei Raumtemperatur unter Verwendung von 0,80 g Pd / C (10% x 50% H₂O) unter einer Wasserstoffatmosphäre bei Normaldruck über 4 h hydriert. Die Wasserstoffaufnahme beträgt 920 ml. Der Ansatz wird filtriert und eingeengt. Der erhaltene Rückstand wird mit Diisopropylether digeriert. Man erhält 1,90 g (11,2 mmol, entsprechend 92 % d. Theorie).

¹H-NMR (DMSO): 7.53 (d, 2H), 6.64 (d, 2H), 5.91 (s, 2H), 3.68 (s, 1H), 2.93 (s, 3H).
ES: 171 (ES).

### b) Herstellung von (RS)-S-(4-Nitrophenyl)-S-methylsulfoximid

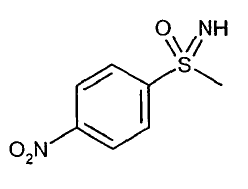

1,56 g (8,5 mmol) 1-(Methylsulfinyl)-4-nitrobenzol in 20 ml DCM werden mit 0,70 g (9,5 mmol) Natriumazid versetzt. Der Ansatz wird bei 0 °C langsam mit 2,3 ml konzentrierter Schwefelsäure versetzt und anschließend auf 45 °C erwärmt. Nach 16 h wird der Ansatz auf Raumtemperatur abgekühlt, mit Wasser versetzt und gegen DCM extrahiert. Die wässrige Phase wird mit 15 %iger NaOH Lösung auf pH 11 gestellt und gegen DCM extrahiert. Die vereinten organischen Phasen werden getrocknet (Na₂SO₄), filtriert und eingeengt. Man erhält 1,08 g (5,4 mmol, entsprechend 63 % d. Theorie) des Produktes.

¹H-NMR (DMSO): 8.43 (d, 2H), 8.17 (d, 2H), 4.62 (s, 1 H), 3.18 (s, 3H).
ES: 201 (ES).

### c) Herstellung von 1-(Methylsulfinyl)-4-nitrobenzol

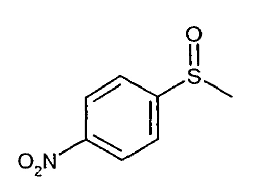

Eine Lösung von 16,0 g (95 mmol) 1-Methylsulfanyl-4-nitro-benzol in 400 ml DCM wird bei Raumtemperatur mit 24,6 g (100 mmol) 3-Chlorperoxybenzoesäure ( ca. 70 %) versetzt. Nach 1 h wird der Ansatz mit DCM verdünnt und mit gesättigter NaHCO₃-Lösung gewaschen. Die organische Phase wird getrocknet (Na₂SO₄), filtriert und eingeengt. Der verbleibende Rückstand wird chromatographisch (DCM / EtOH 8 : 2) gereinigt. Man erhält 7,6 g (41 mmol, entsprechend 43 % d. Theorie) des Produktes.

¹H-NMR (DMSO): 8.41 (d, 2H), 7.97 (d, 2H), 2.86 (s, 3H).
ES: 186 (ES).

### d) Herstellung von (RS)-S-(3-Aminophenyl)-S-methylsulfoximid

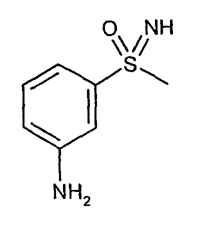

Eine Lösung von 200 mg (1,00 mmol) (*RS*)-*S*-Methyl-*S*-(3-nitrophenyl)sulfoximid in 20 ml THF wird bei Raumtemperatur mit 8 ml einer ca. 10%igen Lösung von Ti(III)Cl in 20-30%iger Salzsäure versetzt. Nach 3 h werden weitere 2 ml der ca. 10%igen Lösung von Ti(III)Cl in 20-30%iger Salzsäure zugegeben und über Nacht bei Raumtemperatur gerührt. Der Ansatz wird mit 1 N NaOH Lösung basisch gestellt und mit Essigester versetzt. Es wird filtriert und der Filterkuchen mit Essigester / MeOH (3 : 2) gewaschen. Das organische Lösungsmittel wird am Rotationsverdampfer abgezogen und der Rückstand gegen Essigester extrahiert. Die vereinten organischen Phasen werden getrocknet (Na₂SO₄), filtriert und eingeengt. Der erhaltene Rückstand wird chromatographisch (DCM / EtOH 95 :5) gereinigt. Man erhält 82 mg (0,48 mmol entsprechend 48% d. Theorie) des Produktes.

¹H-NMR (DMSO): 7.19 (m, 1 H), 7.11 (m, 1H), 7.00 (m, 1 H), 6.75 (m, 1 H), 5.56 (s, 2H), 3.96 (s, 1H), 2.98 (s, 3H).
ES: 171 (ES).

### e) Herstellung von (RS)-S-(3-Aminophenyl)-S-methyl-N-nitrosulfoximid

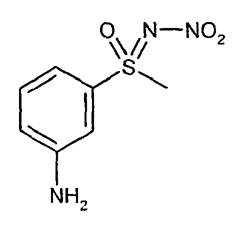

Eine Lösung von 100 mg (0,41 mmol) (*RS*)-*S*-Methyl-*N*-nitro-*S*-(3-nitrophenyl)sulfoximid in 8 ml THF wird bei Raumtemperatur mit 3,1 ml einer ca. 10%igen Lösung von Ti(III)Cl in 20-30%iger Salzsäure versetzt. Nach 1 h werden weitere 1,0 ml der ca. 10%igen Lösung von Ti(III)Cl in 20-30%iger Salzsäure zugegeben und weitere 45 min bei Raumtemperatur gerührt. Der Ansatz wird mit 1 N NaOH Lösung basisch gestellt und mit Essigester extrahiert. Die vereinten organischen Phasen werden getrocknet (Na₂SO₄), filtriert und eingeengt. Der erhaltene Rückstand wird chromatographisch (DCM / EtOH 95 :5) gereinigt. Man erhält 40 mg (0,19 mmol, entsprechend 45% d. Theorie) des Produktes.

¹H-NMR (DMSO): 7.33 (m, 1 H), 7.13 (m, 1 H), 7.03 (m, 1 H), 6.90 (m, 1 H), 5.88 (s, 2H), 3.59 (s, 3H).
ES: 216 (ES).

### f) Herstellung von (RS)-S-Methyl-N-nitro-S-(3-nitrophenyl)sulfoximid (A) (A) und (RS)-S-Methyl-S-(3-nitrophenyl)sulfoximid (B)

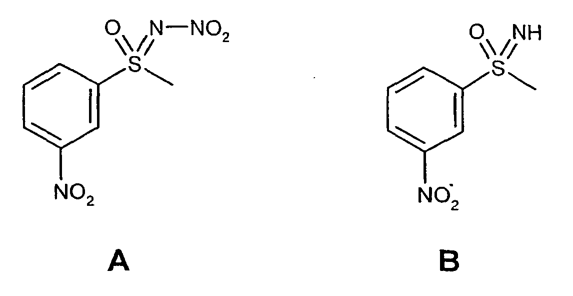

1,0 g (6,45 mmol) (RS)-S-Phenyl-S-methylsulfoximid werden vorsichtig mit 3 ml konz. Schwefelsäure versetzt. Der Ansatz wird unter Rühren bei 0 °C vorsichtig tropfenweise mit 1 ml rauchender Salpetersäure versetzt und langsam über Nacht auf Raumtemperatur erwärmt. Die Reaktionslösung wird vorsichtig auf eisgekühlte 1 N NaOH Lösung gegeben. Der basische Ansatz wird gegen Essigester extrahiert. Die vereinten organischen Phasen werden getrocknet (Na₂SO₄), filtriert und eingeengt. Der erhaltene Rückstand wird mit 15 ml MeOH versetzt. Der gebildete Niederschlag wird abgesaugt und mit Diisopropylether gewaschen. Nach dem Trocknen erhält man 485 mg (1,98 mmol, entsprechend 31% d. Theorie) des Produktes **A**. Das Filtrat wird einrotiert und der gebildete Niederschlag chromatographisch (DCM / EtOH 97 : 3) gereinigt. Man erhält 200 mg (1,00 mmol, entsprechend 16 % d. Theorie) des Produktes **B**.
**(A):**
   ¹H-NMR (DMSO): 8.79 (m, 1 H), 8.64 (m, 1 H), 8.49 (m, 1 H), 8.05 (m, 1H), 3.88 (s, 3H).
**(B):**
   ¹H-NMR (DMSO): 8.65 (m, 1 H), 8.48 (m, 1 H), 8.35 (m, 1 H), 7.90 (m, 1 H), 4.62 (s, 1 H), 3.17 (s, 3H).
   MS: 201 (ES).

### g) Herstellung von 5-Brom-N⁴-isopropyl-N²-[4-(methylsulfinyl)phenyl)-pyrimidin-2,4-diamin

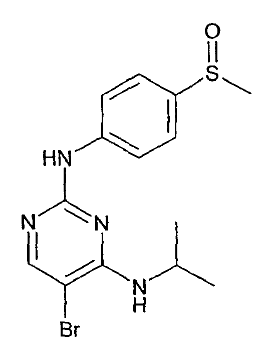

1,77 g (4,6 mmol) 5-Brom-*N*⁴-isopropyl-*N*²-[4-(methylsulfanyl)phenyl)-pyrimidin-2,4-diamin hydrochlorid werden in 40 ml DCM aufgenommen und mit 1,73 g (5,5 mmol) 3-Chlorperoxybenzoesäure (55%) versetzt. Der Ansatz wird 90 min bei Raumtemperatur gerührt und anschließend mit DCM verdünnt. Man wäscht mit gesättigter NaHCO₃-Lösung und gesättigter NaCl-Lösung. Die organische Phase wird getrocknet (Na₂SO₄), filtriert und eingeengt. Der verbleibende Rückstand wird chromatographisch (DCM / EtOH 9 :1) gereinigt. Man erhält 553 mg (1,5 mmol, entsprechend 33 % d. Theorie) des Produktes.

¹H-NMR (DMSO): 9.55 (s, 1H), 8.08 (s, 1 H), 7.90 (d, 2H), 7.53 (d, 2H), 6.53 (d, 1 H), 4.35 (m, 1 H), 2.70 (s, 3H), 1.25 (d, 6H).
MS: 369 (ES).

### h) Herstellung von 5-Brom-N⁴-isopropyl-N²-[4-(methylsulfanyl)phenyl)-pyrimidin-2,4-diamin

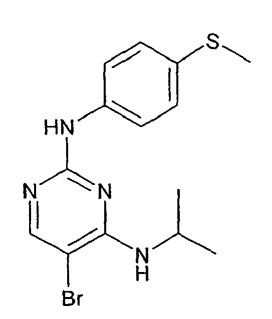

Eine Lösung von 4,08 g (16,3 mmol) (5-Brom-2-chlorpyrimidin-4-yl)-isopropylamin in 20 ml Acetonitril wird bei Raumtemperatur mit einer Lösung von 2 ml (16,3 mmol) 4-Methylsulfanyl-phenylamin in 10 ml Acetonitril versetzt. Der Ansatz wird mit 4,1 ml einer 4 molaren Lösung von Chlorwasserstoff in Dioxan und 4,1 ml Wasser versetzt und anschließend 16 h unter Rückfluss gerührt. Nach dem Erkalten wird der gebildete Niederschlag abgesaugt, mit Wasser gewaschen und getrocknet. Man erhält 4,94 g (12,7 mmol, entsprechend 78 % d. Theorie) des Produktes in Form des Hydrochlorides.

¹H-NMR (DMSO): 10.39 (s, 1H), 8.18 (s, 1H), 7.88 (br, 1H), 7.49 (d, 2H), 7.29 (d, 2H), 4.30 (m, 1 H), 2.5 (s, 3H), 1.21 (d, 6H).
MS: 353 (ES).

### Weitere Zwischenprodukte

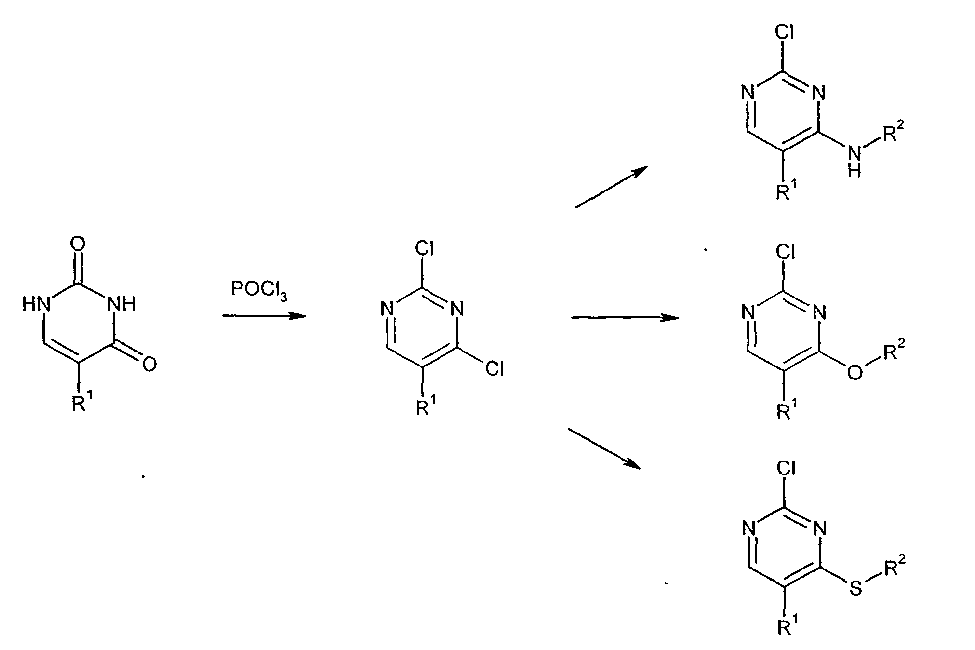

Die Substituenten R¹ und R² haben die in der allgemeinen Formel (I) angegebene Bedeutung.

### i) Herstellung von (R)-2-[(5-Brom-2-chlorpyrimidin-4-yl)amino]propan-1-ol

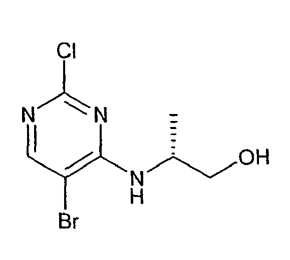

Eine Lösung von 22,8 g (100 mmol) 5-Brom-2,4-dichlorpyrimidin in 100 ml Acetonitril wird bei 0 °C zunächst mit 17,0 ml (125 mmol) Triethylamin und anschließend mit 9,4 g (125 mmol) D-Alaninol versetzt. Der Ansatz wird über Nacht bei Raumtemperatur gerührt. Der gebildete Niederschlag wird abgesaugt, mit Wasser gewaschen und vollständig getrocknet. Man erhält 21,5 g (81 mmol, entsprechend 81% d. Theorie) des Produktes.

¹H-NMR (DMSO): 8.21 (s, 1 H), 7.05 (d, 1 H), 4.86 (t, 1 H), 4.16 (m, 1 H), 3.41 (m, 2H), 1.17 (d, 3H).

### j) Herstellung von (2R, 3R)-3-[(5-Brom-2-chlorpyrimidin-4-yl)oxy]-butan-2-ol

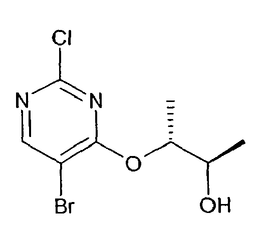

Eine Lösung von 1,35 g (15,0 mmol) (*R*, *R*)-(-)-2,3-Butandiol in 50 ml THF wird bei 0 °C portionsweise mit 480 mg (11,0 mmol) Natriumhydrid (55 %ige Dispersion) versetzt und anschließend 10 min bei Raumtemperatur gerührt. Die entstandene Lösung wird bei 0 °C zu 2,27 g (10.0 mmol) 5-Brom-2,4-dichlorpyrimidin in 25 ml THF gegeben. Der Ansatz wird langsam auf Raumtemperatur erwärmt und 12 h gerührt. Das Lösungsmittel wird abgezogen und der erhaltene Rückstand chromatographisch (Hexan / Essigester 1 : 1) gereinigt. Man erhält 2,29 g (8,1 mmol, entsprechend 81 % d. Theorie) des Produktes.

¹H-NMR (DMSO): 8.44 (s, 1H) , 5.18 (q, 1 H), 3.96 (q, 1 H), 2.02 (d, 1 H), 1.4 (d, 3H), 1.28 (d, 3H).
MS: 281 (ES).

### k) Herstellung von (R)-3-[(5-Brom-2-chlorpyrimidin-4-yl)amino]-2-methylbutan-2-ol

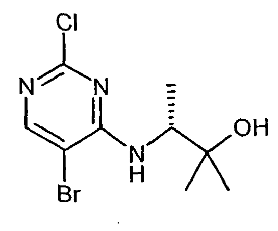

Eine eisgekühlte Lösung von 2,95 g (10,0 mmol) Methyl-*N*-(5-brom-2-chlorpyrimidin-4-yl)-D-alaninat in 150 ml THF wird tropfenweise mit 30 ml (90 mmol) einer 3 molaren Lösung von Methylmagnesiumbromid in Diethylether versetzt. Nach 2,5 Stunden bei Raumtemperatur wird der Ansatz mit 30 ml gesättigter Ammoniumchlorid Lösung versetzt. Man verdünnt mit Wasser und extrahiert gegen Essigester (3x). Die vereinten organischen Phasen werden getrocknet (Na₂SO₄), filtriert und eingeengt. Der verbleibende Rückstand wird chromatographisch (Hexan / Essigester: 4:1 - 1:1) gereinigt. Man erhält 2,81 g (9,5 mmol, entsprechend 95% der Theorie) des Produktes.

¹H-NMR (CDCl₃): 8.1 (s,1H), 5.9 (d,1 H), 4.2 (m,1H), 1.8 (br,1 H), 1.2 (m, 9H).

### ka) Herstellung von Methyl-N-(5-brom-2-chlorpyrimidin-4-yl)-D-alaninat

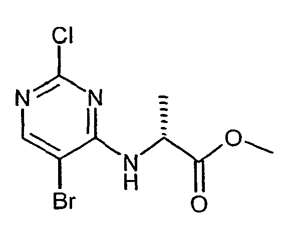

22,8 g (100mmol) 5-Brom-2,4-dichlorpyrimidin und 14,0 g (100 mmol) D-Alaninsäuremethylester Hydrochlorid werden in 300 ml THF und 75 ml DMF gelöst. Der eisgekühlte Ansatz wird mit 33,5 ml (240 mmol) Triethylamin versetzt und anschließend langsam auf Raumtemperatur erwärmt. Nach 48 Stunden wird das Lösungsmittel am Rotationsverdampfer entfernt und der verbleibende Rückstand chromatographisch (Hexan / Essigester: 4:1 - 2:1) gereinigt. Man erhält 25,5 g (86,1 mmol, entsprechend 86% der Theorie) des Produktes.

¹H-NMR (CDCl₃): 8.2 (s,1H), 6.1 (d,1H), 4.8 (m,1H), 3.8 (s,3H), 1.6 (d,3H).

### l) Herstellung von (2R,3R)-3-[(5-Brom-2-chlorpyrimidin-4-yl)amino]butan-2-ol

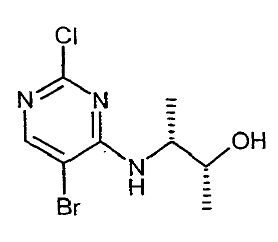

32,7 g (159 mmol) Kupfer(I)bromid Dimethylsulfid Komplex werden unter Stickstoffatmosphäre in 1000 ml Diethylether vorgelegt und auf -78 °C gekühlt. Über einen Zeitraum von ca. 25 Minuten werden 200 ml einer 1,6 molaren Lösung von Methyllithium in Diethylether zugetropft und anschliessend das Kühlbad entfernt. Der Ansatz wird 40 Minuten gerührt, die Temperatur steigt auf -35 °C. Es wird auf -55 °C abgekühlt und 18,9 g (71,5 mmol) (*R*)-2-[(5-Brom-2-chlorpyrimidin-4-yl)amino]propanal über einen Zeitraum von 20 Minuten zugegeben. Es wird 6 Stunden bei -55 °C gerührt, anschliessend wird das Kühlbad erneut mit Trockeneis gefüllt, mit Alufolie abgedeckt und der Ansatz über Nacht gerührt. Es werden 200 ml einer gesättigten Ammoniumchlorid Lösung zugetropft und der Ansatz auf Raumtemperatur erwärmt. Es wird mit 500 ml Diethylether verdünnt, die organische Phase abgetrennt und die wässrige Phase mit Diethylether extrahiert. Die vereinten organischen Phasen werden mit gesättigter Ammoniumchlorid Lösung und gesättigter NaCl Lösung gewaschen, getrocknet (Na₂SO₄), filtriert und eingeengt. Der verbleibende Rückstand wird chromatographisch (Hexan / Essigester: 4:1 - 1:1) gereinigt. Man erhält 8,4 g (30,0 mmol, entsprechend 42 % der Theorie) des Produktes.

¹H-NMR (CDCl₃): 8.1 (s,1H), 5.8 (d,1H), 4.2 (m,1 H), 3.9 (m,1H), 2.0 (d,1H), 1.3 (d,3H), 1.2 (d,3H).

HPLC-Analytik:

| | |
|---|---|
| Säule: | Chiralpak AD-H 5µ |
| Länge x ID: | 150 x 4,6 mm |
| Eluenten: | A= Hexan, C = Ethanol |
| Fluß: | 1,0 ml/min |
| Gradient: | Isokratisch 5%C |
| Detektor: | UV 254nm |
| Temperatur: | 25 °C |
| RT in min: | 6,04 |

### Ia) Herstellung von (R)-2-[(5-Brom-2-chlorpyrimidin-4-yl)amino]propanal

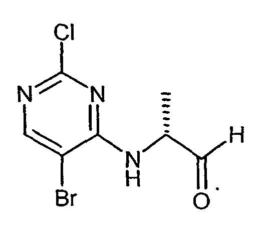

Eine Lösung von 40,0g (135,8 mmol) Methyl-N-(5-brom-2-chlorpyrimidin-4-yl)-D-alaninat in 800 ml Toluol wird bei -78°C mit 310 ml einer 1,2 molaren Lösung von Diisobutylaluminiumhydrid versetzt. Nach 30 Minuten wird vorsichtig mit Methanol gequencht. Der Ansatz wird auf Raumtemperatur erwärmt und mit 1000 ml tert-Butyl-methylether verdünnt. Man wäscht sukzessive mit 1 N HCl (3x 100 ml), gesättigter Natriumhydrogencarbonat Lösung (3x) und gesättigter NaCl Lösung (3x). Die organische Phase wird getrocknet (MgSO₄), filtriert und eingeengt. Der verbleibende Rückstand wird chromatographisch (Hexan / Essigester: 4:1 - 1:1) gereinigt. Man erhält 22,5 g (83,9 mmol, entsprechend 62% der Theorie) des Produktes.

¹H-NMR (CDCl₃): 9.6 (s,1H), 8.2 (s,1H), 6.3 (d,1H), 4.8 (m,1H), 1.5 (d,3H).

### Ib) Herstellung von (2R,3R)-3-[(5-Brom-2-chlorpyrimidin-4-yl)amino]-4-methoxybutan-2-ol

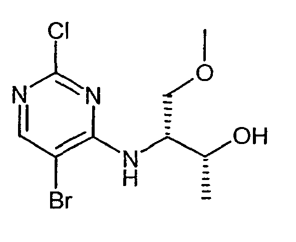

311 mg (2,6 mmol) (2*R*,3*R*)-3-Amino-4-methoxy-butan-2-ol Hydrochlorid (Herstellung nach A.I. Meyers, D. Hoyer, Tet. Lett. 1985, 26, 4687) in 2 ml Acetonitril werden mit 0,28 ml Triethylamin versetzt und geschüttelt. Man filtriert und wäscht den Filterkuchen mit 2 ml Acetonitril. Das Filtrat wird zu einer Lösung von 455 mg (2,0 mmol) 5-Brom-2,4dichlor-pyrimidin in 26 ml Acetonitril bei -30°C getropft. Durch Entfernen des Kühlbades wird langsam unter Rühren auf Raumtemperatur erwärmt. Nach 16 Stunden wird das Lösungsmittel am Rotationsverdampfer entfernt und der verbleibende Rückstand chromatographisch (Hexan / Essigester: 4:1 - 1:1) gereinigt. Man erhält 509 mg (1,6 mmol, entsprechend 80% der Theorie) des Produktes.

¹H-NMR (CDCl₃): 8.1 (s,1 H), 6.3 (d,1 H), 4.3 (m,1 H), 4.2 (m,1 H), 3.8 (d,2H), 3.4 (s,3H), 3.1 (d,1H), 1.2 (d,3H).

### Ic) Herstellung von 5-Brom-2-chlorpyrimidin-4-ol

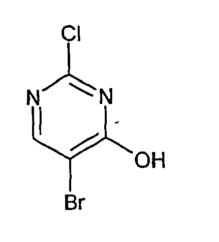

50,5 g 5-Brom-2,4-dichlorpyrimidin werden mit 133 ml 2N Natronlauge versetzt und bei 45-50 °C 50 Minuten gerührt. Nach Abkühlung wird unter Eiskühlung mit 21 ml konzentrierter Salzsäure angesäuert. Der Niederschlag wird abgesaugt, mit Wasser und wenig Methylenchlorid gewaschen und bei 25-35 °C getrocknet. Man erhält 17,12 g (36,9 % d. Th.) des Produktes vom Schmelzpunkt 136-145 °C (Zersetzung).

In analoger Verfahrensweise zu den oben jeweils beschriebenen Verfahrensvarianten werden auch die nachfolgenden Verbindungen hergestellt:

| | | | |
|---|---|---|---|
| | 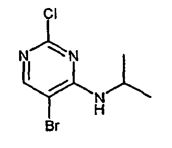 | 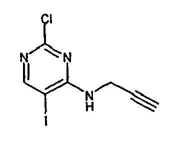 | 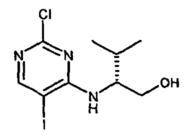 |

| Beispiel | m | ma | mb |
|---|---|---|---|
| MS | 250 (CI) | 293 (EI) | 341 (EI) |

### n) Herstellung von Herstellung von 5-Brom-2-[4-(methylsulfanyl)phenylamino]pyrimidin-4-ol

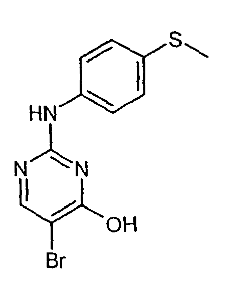

9,8 g 5-Brom-2-chlorpyrimidin-4-ol werden in 200 ml Acetonitril suspendiert. Nach Zugabe von 7,2 g 4-Methylsulfanyl-phenylamin werden unter kräftigem Rühren 12 ml einer 4N Lösung von HCl in Dioxan zugetropft. Nach tropfenweiser Zugabe von 5 ml Wasser wird die Mischung 3 Stunden bei 78 °C und 2 Tage bei Raumtemperatur gerührt. Die Mischung wird im Eisbad gekühlt und abgesaugt. Der Filterkuchen wird zweimal mit Acetonitril gewaschen und getrocknet. Man erhält 15,2 g (92,7 % d. Th.) des Produktes vom Schmelzpunkt 238 °C (Zersetzung).

### o) Herstellung von (RS)-5-Brom-2-[4-(methylsulfinyl)phenylamino]pyrimidin-4-ol

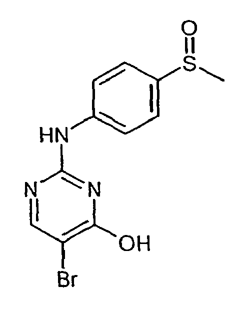

11 g 5-Brom-2-[4-(methylsulfanyl)phenylamino]pyrimidin-4-ol werden in 110 ml Eisessig suspendiert. Unter Kühlung mit Eiswasser werden 4,6 ml einer 30%igen Lösung von Wasserstoffsuperoxid zugetropft. Die Mischung wird 18 Stunden bei Raumtemperatur gerührt und anschliessend abgesaugt. Der Filterkuchen wird zweimal mit Wasser und einmal mit Ethanol gewaschen und bei 60 °C im Vakuum getrocknet. Man erhält 8,75 g (75,7 % d. Th.) des Produktes vom Schmelzpunkt 240 °C (Zersetzung).

### p) Herstellung von (RS)-S-{4-[(5-Brom-4-hydroxypyrimidin-2-yl)amino]-phenyl}-S-methylsulfoximid

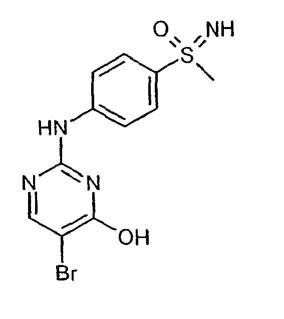

324 mg (*RS*)-5-Brom-2-[4-(methylsulfinyl)phenylamino]pyrimidin-4-ol und 128 mg Natriumazid werden in 6 ml Methylenchlorid suspendiert und unter Eiskühlung tropfenweise mit 0,3 ml konzentrierter Schwefelsäure versetzt. Die Mischung wird 36 Stunden bei 40 °C gerührt. Die organische Phase wird abdekantiert und der Rückstand mit Eiswasser verrührt. Der Feststoff wird abgesaugt, zweimal mit Wasser und einmal mit Ethanol gewaschen und getrocknet. Man erhält 266 mg (78,2 % d. Th.) des Produktes vom Schmelzpunkt 230 °C (Zersetzung).

### q) Herstellung von (RS)-S-{4-[(5-Brom-4-chlorpyrimidin-2-yl)amino]phenyl}-S-methylsulfoximid

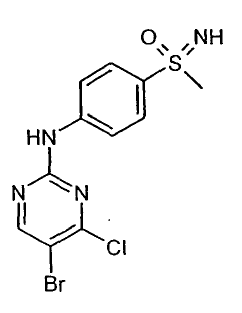

255 mg (*RS*)-*S*-{4-[(5-Brom-4-hydroxypyrimidin-2-yl)amino]phenyl}-*S-*methylsulfoximid werden in 1,5 ml Phosphoroxychlorid suspendiert und 3 Stunden bei 106 °C und 16 Stunden bei Raumtemperatur gerührt. Die Mischung wird auf Eiswasser gegossen, unter starker Kühlung (Temperatur < 5 °C) mit 25%iger Ammoniaklösung alkalisch gestellt und 1 Stunde im Eisbad gerührt. Der Niederschlag wird abgesaugt, mit Wasser gewaschen und bei 60 °C getrocknet. Man erhält 220 mg (81,8 % d. Th.) des Produktes vom Schmelzpunkt 170-173 °C.

### r) Herstellung von (RS)-S-(4-Aminophenyl)-S-cyclopropyl-N-[2-(trimethylsilyl)ethylsulfonyl]sulfoximid

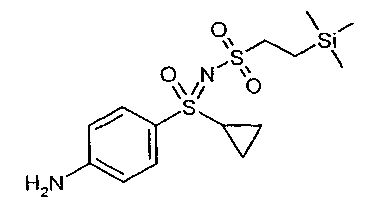

320 mg (*RS*)-*S*-Cyclopropyl-*S*-(4-nitrophenyl)-*N*-[2-(trimethylsilyl)ethylsulfonyl]sulfoximid werden in 5 ml Tetrahydrofuran gelöst. Unter Eiskühlung werden 7,2 ml einer ca. 10 Gewichts-%igen Lösung von Titan(III)-chlorid in 20-30 Gewichts-%iger Salzsäure zugetropft. Die Lösung wird 16 Stunden bei Raumtemperatur gerührt und auf Eis gegossen. Mit 15%iger Natronlauge wird der pH-Wert auf 8-9 eingestellt. Nach Zugabe von Ethylacetat wird die Mischung kräftig gerührt. Der Niederschlag wird abgesaugt und mit 100 ml Ethylacetat gewaschen. Die Filtrate werden vereinigt, getrocknet und eingeengt. Nach Reinigung durch Flash-Chromatographie erhält man 215 mg (RS)-S-(4-Aminophenyl)-S-cyclopropyl-N-[2-(trimethylsilyl)ethylsulfonyl]sulfoximid.
Schmelzpunkt: 137-138 °C

In analoger Weise werden auch **(RS)-S-(4-Aminophenyl)-S-cyclopropylmethyl-N-[2-(trimethylsilyl)ethylsulfonyl]sulfoximid** (Schmelzpunkt: 138-140 °C) und **(RS)-S-(4-Aminophenyl)-S-cyclopentyl-N-[2-(trimethylsilyl)ethylsulfonyl]sulfoximid** (Schmelzpunkt: 146-147 °C) hergestellt.

### s) Herstellung von (RS)-S-Cyclopropyl-S-(4-nitrophenyl)-N-[2-(trimethylsilyl)ethylsulfonyl]sulfoximid

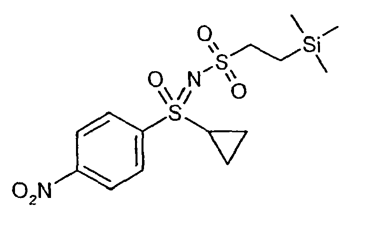

260 mg (*RS*)-1-(Cyclopropylsulfinyl)-4-nitrobenzol werden in 10 ml Acetonitril gelöst, mit 100 mg Tetrakis-(acetonitril)-kupfer(I)-hexafluorophosphat versetzt und 45 Minuten bei Raumtemperatur gerührt. Die Lösung wird im Eisbad gekühlt und mit 613 mg [N-(2-(Trimethylsilyl)ethansulfonyl)imino]phenyliodinan (Phl=NSes: J. Org. Chem., 64(14), 5304-5307 (1999)) versetzt. Nach 30 Minuten Rühren bei 0 °C werden weitere 232 mg Phl=NSes zugegeben. Nach 2 Stunden Rühren bei 0 °C werden weitere 60 mg Phl=NSes und 10 mg Tetrakis-(acetonitril)-kupfer(I)-hexafluorophosphat hinzugefügt. Nach 30 Minuten Rühren bei 0 °C wird die Mischung eingeengt. Der ölige Rückstand wird mit Hexan versetzt, wobei das Produkt kristallisiert. Die Lösung wird abdekantiert und der Feststoff durch Flash-Chromatographie (Hexan/Ethylacetat) gereinigt. Man erhält 325 mg (*RS*)-*S*-Cyclopropyl-*S*-(4-nitrophenyl)-*N*-[2-(trimethylsilyl)ethylsulfonyl]sulfoximid.
Schmelzpunkt: 111-114 °C

### t) Herstellung von (RS)-1-(Cyclopropylsulfinyl)-4-nitrobenzol

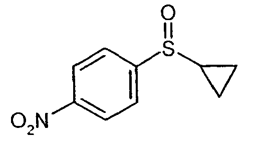

350 mg 1-(Cyclopropylsulfanyl)-4-nitrobenzol werden in 5 ml Acetonitril gelöst und mit 10 mg Eisen(III)-chlorid Hexahydrat versetzt. Nach 10 Minuten Rühren bei Raumtemperatur werden 450 mg Perjodsäure unter Kühlung dazugegeben. Die Mischung wird 30 Minuten bei Raumtemperatur gerührt, im Eisbad abgekühlt und tropfenweise mit halbgesättigter Natriumdisulfit-Lösung versetzt. Es wird mit Methylenchlorid verdünnt, mit Wasser, NatriumhydrogencarbonatLösung und gesättigter Natriumchlorid-Lösung gewaschen und eingeengt. Nach Reinigung durch Flash-Chromatographie erhält man 270 mg (*RS*)-1-(Cyclopropylsulfinyl)-4-nitrobenzol.
Schmelzpunkt: 104-106 °C

In analoger Weise werden hergestellt:

### ta) Herstellung von (RS)-1-(Ethylsulfinyl)-4-nitrobenzol

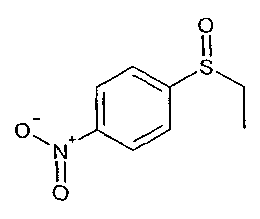

¹H-NMR (DMSO): 8.39 (m, 2H), 7.91 (m, 2H), 3.18 (m, 1 H), 2.88 (m, 1 H), 1.06 (tr, 3H).

### tb) Herstellung von (RS)-2-[(4-Nitrophenyl)sulfinyl]ethanol

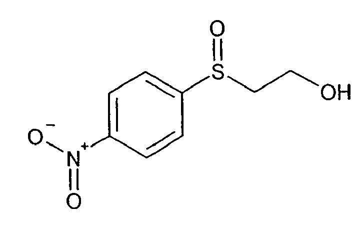

¹H-NMR (DMSO): 8.41 (m, 2H), 7.93 (m, 2H), 5.13 (tr, 1 H), 3.84 (m, 1H), 3.78 (m, 1 H), 3.16 (m, 1 H), 2.95 (m, 1 H).
MS: 216 (ES).

### tc) Herstellung von (RS)-1-(Isopropylsulfinyl)-4-nitrobenzol

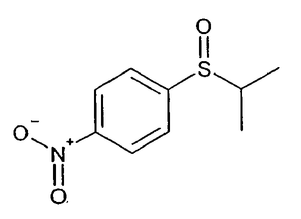

¹H-NMR (DMSO): 8.39 (m, 2H), 7.88 (m, 2H), 3.10 (m, 1H), 1.25 (d, 3H), 0.88 (d, 3H).

### td) Herstellung von (RS)-2-Methyl-1-(methylsulfinyl)-4-nitrobenzol

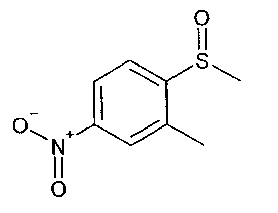

¹H-NMR (DMSO): 8.31 (m, 1 H), 8.19 (m, 1 H), 8.04 (m, 1 H), 2.78 (s, 3H), 2.45 (s, 3H).
MS: 200 (ES).

### te) Herstellung von (RS)-1-(Methylsulfinyl)-4-nitro-2-(trifluormethyl)benzol

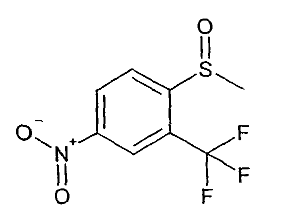

¹H-NMR (DMSO): 8.78 (m, 1H), 8.50 (m, 2H), 2.83 (s, 3H).
MS: 270 (ES).

### tf) Herstellung von (RS)-2-Fluor-1-(methylsulfinyl)-4-nitrobenzol

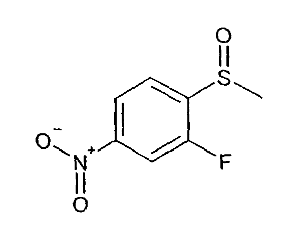

¹H-NMR (DMSO): 8.33 (m, 2H), 7.99 (m, 1 H), 2.90 (s, 3H).
MS: 204 (ES).

### u) Herstellung von 1-(Cyclopropylsulfanyl)-4-nitrobenzol

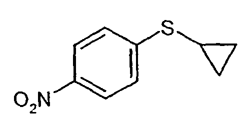

Die Cyclisierung von1-(3-Chlor-propylsulfanyl)-4-nitro-benzol wurde wie in J. Org. Chem., 33(1), 43-47 (1968) beschrieben durchgeführt.
¹H-NMR (DMSO): 8.18 (d, 2H), 7.60 (d, 2H), 2.40 (m, 1H), 1.21 (m, 2H), 0.66 (m, 2H).
MS (CI): 195 (M⁺, 12 %), 213 (M⁺+1+NH₃, 100 %), 230 (M⁺+1+2 NH₃, 44 %).

### v) Herstellung von 1-[(3-Chlorpropyl)sulfanyl]-4-nitrobenzol

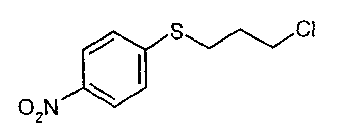

1 g Kaliumhydroxid werden in 40 ml Methanol gelöst und mit 2,3 g 4-Nitrothiophenol versetzt. Die Suspension wird 1 Stunde bei Raumtemperatur gerührt und tropfenweise mit 1,48 ml 1-Brom-3-chlorpropan versetzt. Nach 4 Stunden Rühren bei Raumtemperatur werden weitere 0,15 ml 1-Brom-3-chlorpropan zugetropft. Die Mischung wird 65 Stunden bei Raumtemperatur gerührt, im Vakuum eingeengt und in Ethylacetat aufgenommen. Es wird mit Wasser und gesättigter Kochsalz-Lösung extrahiert, über Natriumsulfat getrocknet und eingeengt. Nach Reinigung durch Flash-Chromatographie erhält man 2,54 g 1-(3-Chlor-propylsulfanyl)-4-nitro-benzol.
¹H-NMR (DMSO): 8.16 (d, 2H), 7.55 (d, 2H), 3.77 (t, 2H), 3.25 (t, 2H), 2.08 (q, 2H).
MS (ES): 232 (100 %), 234 (38 %).

In analoger Weise werden auch 1-Cyclopropylmethylsulfanyl-4-nitro-benzol (aus (Chlormethyl)-cyclopropan) und 1-Cyclopentylsulfanyl-4-nitro-benzol (aus Bromcyclopentan) hergestellt.

### w) Herstellung von (RS)-S-(2-Hydroxyethyl)-S-(4-nitrophenyl)-N-[2-(trimethylsilyl)ethylsulfonyl]sulfoximid

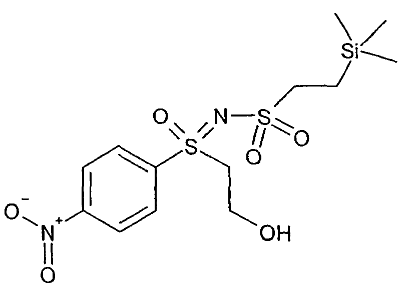

¹H-NMR (DMSO): 8.48 (m, 2H), 8.24 (m, 2H) , 4.97 (tr, 1 H), 3.99 (tr, 2H), 3.79 (m, 2H), 3.00 (dd, 2H), 0.96 (m, 2H), 0.05 (s, 9H).

### x) Herstellung von (RS)-S-(4-Amino-2-methoxyphenyl)-S-methylsulfoximid

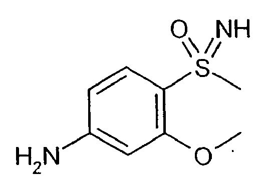

1,5 g (6,5 mmol) (*RS*)-*S*-(2-Methoxy-4-nitrophenyl)-S-methylsulfoximid in 100ml Ethanol werden mit 300 mg Palladium auf Kohle (10% x 50 % H₂O) versetzt und 45 Minuten bei Raumtemperatur und Normaldruck hydriert. Der Ansatz wird filtriert und eingeengt. Man erhält 1,0 g (5,1 mmol, entsprechend 79 % d. Theorie) des Produktes.

¹H-NMR (DMSO-D6): 7.10 (m, 1 H), 6.92 (m, 1 H), 6.73 (m, 1 H), 4.70 (br, 3H), 3.76 (s, 3H), 3.13 (s, 3H).
MS: 201 (ES).

### y) Herstellung von (RS)-S-(2-Methoxy-4-nitrophenyl)-S-methylsulfoximid

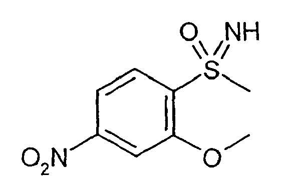

7,5 g rauchende Salpetersäure werden auf -10 °C gekühlt und langsam mit 5,0 g (32, 4 mmol) 1-Methoxy-2-methylsulfanyl-benzol versetzt. Der Ansatz wird langsam unter Rühren auf Raumtemperatur erwärmt, mit 100 ml Wasser verdünnt und mit Natriumhydrogencarbonat neutralisiert. Man extrahiert mit Diethylether und Ethylacetat. Die vereinten organischen Phasen werden getrocknet (Na₂SO₄), filtriert und eingeengt.
5,3 g des erhaltenen Zwischenproduktes werden mit 1,8 g (27,7 mmol) Natriumazid und 25 ml CHCl₃ versetzt. Der Ansatz wird auf 0 °C gekühlt und vorsichtig mit 6,3 ml konzentrierter Schwefelsäure versetzt. Es wird zunächst auf Raumtemperatur und anschließend auf 45 °C erwärmt. Der Ansatz wird über Nacht bei dieser Temperatur gerührt. Nach dem Abkühlen wird mit 75 ml Eiswasser und 20 ml CHCl₃ versetzt. Die organische Phase wird abgetrennt und die wässrige Phase erneut mit 100 ml CHCl₃ extrahiert. Die wässrige Phase wird mit 1 N NaOH Lösung basisch gestellt und anschließend gegen CHCl₃ extrahiert (2x). Die organischen Phasen der letzten Extraktion werden vereint, getrocknet (Na₂SO₄), filtriert und eingeengt. Man erhält 3,8 g (16,5 mmol) des Produktes.

¹H-NMR (DMSO-D6): 8.66 (m, 1H), 8.48 (m, 1 H), 7.45 (m, 1 H), 4.70 (s, 1H), 4.08 (s, 3H), 3.21 (s, 3H).
MS: 231 (ES).

### z) Herstellung von (RS)-S-(2-Methyl-4-nitrophenyl)-S-methylsulfoximid

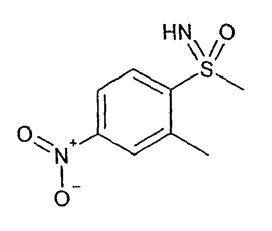

1,5 g (7,5 mmol) (RS)-2-Methyl-1-(methylsulfinyl)-4-nitrobenzol und 1,1 g (17,1 mmol) Natriumazid in 10,0 ml CHCl₃ werden bei 0 °C vorsichtig mit 2,2 ml konz. Schwefelsäure versetzt. Der Ansatz wird zunächst auf Raumtemperatur und anschließend unter starkem Rühren auf 45 °C erwärmt. Man rührt 116 Stunden bei dieser Temperatur. Nach dem Abkühlen wird mit Wasser versetzt und gegen DCM extrahiert (2x). Die wässrige Phase wird mit 2 N NaOH Lösung basisch gestellt und gegen DCM extrahiert. Die vereinten organischen Phasen werden über einen Whatman Filter filtriert und eingeengt. Das erhaltene Rohprodukt wird aus Essigester umkristallisiert. Man erhält 1,3 g (6,1 mmol, entsprechend 81 % d. Theorie) des Produktes.
¹H-NMR (DMSO): 8.28 (m, 1 H), 8.22 (m, 2H), 4.67 (s, 1 H), 3.17 (s, 3 H), 2.81 (s, 3H).
MS: 215 (ES).

### za) Herstellung von (RS)-S-Methyl-S-[4-nitro-2-(trifluormethyl)phenyl]sulfoximid

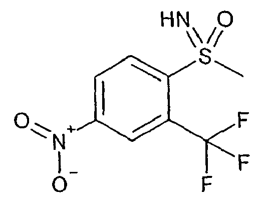

¹H-NMR (DMSO): 8.73 (m, 1 H), 8.52 (m, 2H), 5.00 (s, 1 H), 3.17 (s, 3H).
MS: 269 (ES).

### zb) Herstellung von (RS)-S-(2-Fluor-4-nitrophenyl)-S-methylsulfoximid

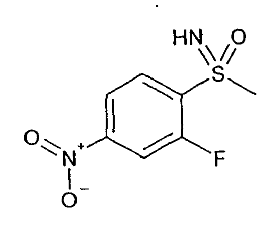

¹H-NMR (DMSO): 8.34 (m, 1 H), 8.24 (m, 1 H), 8.10 (m, 1H), 5.08 (s, 1 H), 3.21 (s, 3H).
MS: 219 (ES).

### zc) Herstellung von (RS)-N,S-Dimethyl-S-(4-nitrophenyl)sulfoximid

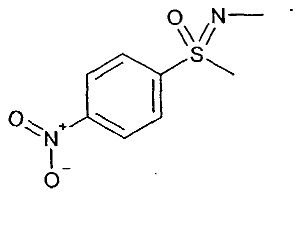

500 mg (2,5 mmol) (*RS*)-*S*-(4-Nitrophenyl)-*S*-methylsulfoximid in 4 ml Formaldehyd (aqueous, 37 %) und 20 ml Ameisensäure (98-100%) werden bei 100 °C im offenen Kolben gerührt. Nach 22 Stunden ist das Lösungmittel abgedampft, man versetzt erneut mit 4 ml Formaldehyd (aqueous, 37 %) und 20 ml Ameisensäure (98-100%) und rührt weitere 22 Stunden bei 100 °C. Reste des Lösungsmittels werden am Rotationsverdampfer entfernt. Der verbleibende Rückstand wird mit 2N HCl gelöst und gegen DCM extrahiert. Die wässrige Phase wird mit NaHCO₃ basisch gestellt und gegen DCM extrahiert. Die vereinten organischen Phasen werden getrocknet (Na₂SO₄), filtriert und eingeengt. Man erhält 448 mg (2,1 mmol, entsprechend 85 % d. Theorie) des Produktes.
¹H-NMR (DMSO-D6): 8.43 (m, 2H), 8.08 (m, 2H), 3.24 (s, 3H), 2.48 (s,3H).
MS: 214 (ES).

### zd) Herstellung von (RS)-N-(Ethoxycarbonyl)-S-methyl-S-(4-nitrophenyl)sulfoximid

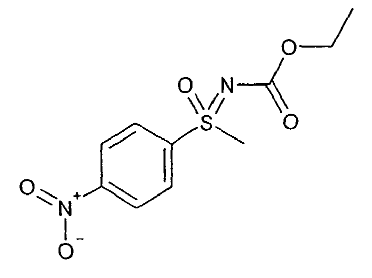

8,50 g (42,5 mmol) (*RS*)-*S*-(4-Nitrophenyl)-*S*-methylsulfoximid in 400 ml Pyridin werden bei Raumtemperatur tropfenweise mit 18,8 ml (197,2 mmol) Chlorameisensäureethylester versetzt. Der Ansatz wird 4 Stunden bei Raumtemperatur gerührt und anschließend in verdünnte NaCl Lösung gegeben. Es wird gegen Essigester extrahiert. Die vereinten organischen Phasen werden getrocknet (Na₂SO₄), filtriert und eingeengt. Der verbleibende Rückstand wird chromatographisch (Hexan / Essigester 1 : 1) gereinigt. Man erhält 8,94 g (32,8 mmol, entsprechend 77 % d. Theorie) des Produktes.

¹H-NMR (DMSO-D6): 8.49 (m, 2H), 8.22 (m, 2H), 3.90 (m, 2H), 3.56 (s, 3H), 1.10 (tr, 3H).

### ze) Herstellung von (RS)-S-Ethyl-N-({[(1R,2S,5R)-2-isopropyl-5-methylcyclohexyl]oxy}carbonyl)-S-(4-nitrophenyl)sulfoximid

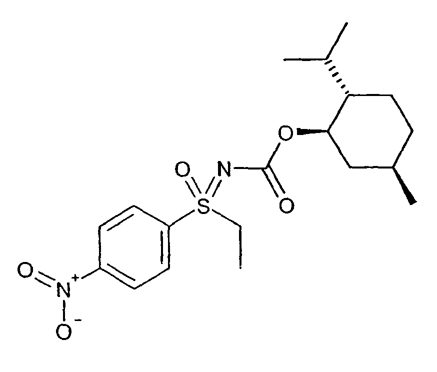

100 mg (0,47 mmol) (RS)-S-(4-Nitrophenyl)-S-ethylsulfoximid in 4,40 ml Pyridin werden bei Raumtemperatur tropfenweise mit 0,46 ml (2,17 mmol) (+) Menthylchloroformat versetzt. Der Ansatz wird 4 Stunden bei Raumtemperatur gerührt und anschließend in verdünnte NaCl Lösung gegeben. Es wird gegen Essigester extrahiert. Die vereinten organischen Phasen werden getrocknet (Na₂SO₄), filtriert und eingeengt. Der verbleibende Rückstand wird chromatographisch (Hexan / Essigester 1 : 1) gereinigt. Man erhält 161 mg (0,41 mmol, entsprechend 87 % d. Theorie) des Produktes.

¹H-NMR (DMSO-D6): 8.49 (m, 2H), 8.13 (m, 2H), 4.28 (m, 1H), 3.67 (m, 2H), 1.77 (m, 1H), 1.55 (m, 2H), 1.25 (m, 6H), 0.75 (m, 12H).

### zf) Herstellung von (RS)-N-(Ethoxycarbonyl)-S-ethyl-S-(4-nitrophenyl)sulfoximid

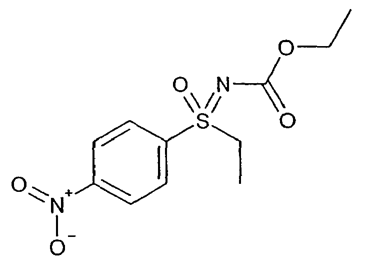

¹H-NMR (DMSO-D6): 8.48 (m, 2H), 8.15 (m, 2H), 3.92 (m, 2H), 3.69 (m, 2H), 1.12 (m, 6H).

### zg) Herstellung von (RS)-N-(Ethoxycarbonyl)-S-methyl-S-(2-methyl-4-nitrophenyl)sulfoximid

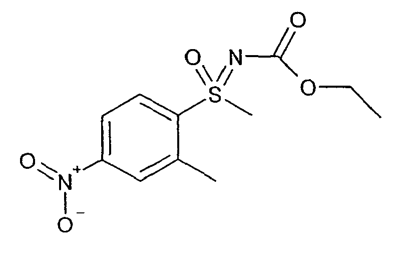

¹H-NMR (DMSO): 8.33 (m, 2H), 8.17 (m, 1H), 3.90 (q, 2H), 3.55 (s, 3H), 2.73 (s, 3H), 1.08 (tr, 3H).
MS: 287 (ES).

### zh) Herstellung von (RS)-N-(Ethoxycarbonyl)-S-(2-fluor-4-nitrophenyl)-S-methylsulfoximid

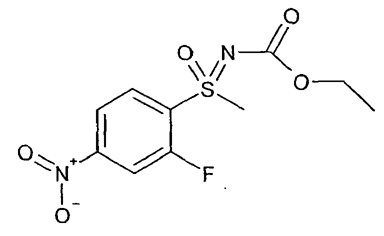

¹H-NMR (DMSO): 8.45 (m, 1 H), 8.33 (m, 1 H), 8.19 (m, 1 H), 3.40 (m, 2H), 3.60 (s, 3H), 1.04 (tr, 3H).

### zi) Herstellung von (RS)-N-(Ethoxycarbonyl)- S-methyl-S-[4-nitro-2-(trifluormethyl)phenyl]sulfoximid

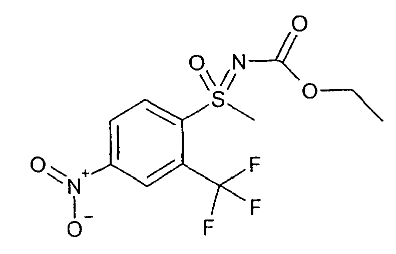

¹H-NMR (DMSO): 8.78 (m, 1 H), 8.65 (m, 1 H), 8.49 (m, 1 H), 3.90 (q, 2H), 3.58 (s, 3H), 1.07 (tr, 3H).

### zj) Herstellung von (RS)-S-(4-Aminophenyl)-N-(ethoxycarbonyl)-S-methylsulfoximid

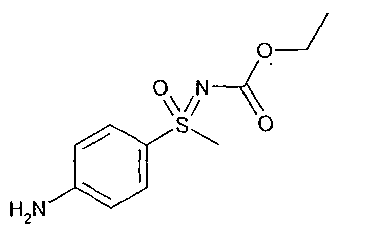

Eine Lösung von 8,70 g (32,0 mmol) (*RS)*-*N*-(Ethoxycarbonyl)-*S*-methyl-*S*-(4-nitrophenyl)sulfoximid in 650 ml THF wird bei Raumtemperatur langsam mit 435 ml einer 10%igen Lösung von Ti(III)Cl in etwa 10%iger Salzsäure (Aldrich) versetzt. Der Ansatz wird 4 Stunden bei Raumtemperatur gerührt und anschliessend auf 0 °C abgekühlt. Es werden 450 ml einer 32%igen NaOH Lösung zugetropft. Dabei wird das Reaktionsgemisch zwischenzeitlich durch die Zugabe von Wasser und Essigester verdünnt. Man versetzt mit 500 ml Essigester und trennt die organische Phase ab. Die breiige wässrige Phase wird gegen Essigester extrahiert. Die vereinten organischen Phasen werden mit verdünnter NaCl Lösung gewaschen, getrocknet (Na₂SO₄), filtriert und eingeengt. Man erhält 8,05 g (ca. 32,0 mmol) des Produktes, das ohne weitere Reinigung verwendet wird.

¹H-NMR (DMSO-D6): 7.52 (m, 2H), 6.66 (m, 2H), 6.17 (m, 2H), 3.91 (q, 2H), 3.30 (s, 3H), 1.12 (tr, 3H).

### zk) Herstellung von (RS)-S-(4-Aminophenyl)-N-(ethoxycarbonyl)-S-ethylsulfoximid

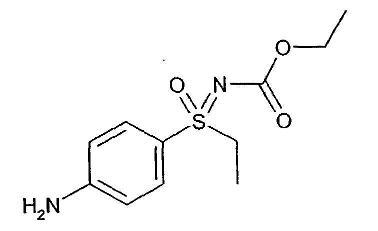

¹H-NMR (DMSO-D6): 7.47 (m, 2H), 6.67 (m, 2H), 6.20 (s, 2H), 3.90 (m, 2H), 3.42 (q, 2H), 1.10 (m, 6H).

### zl) Herstellung von (RS)-S-(4-Aminophenyl)-S-(2-hydroxyethyl)-N-[2-(trimethylsilyl)ethylsulfonyl]sulfoximid

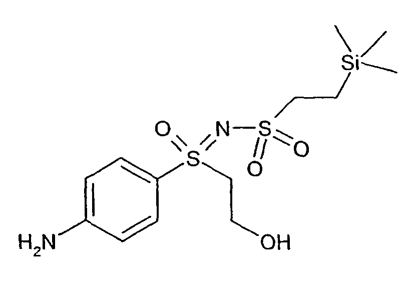

¹H-NMR (DMSO-D6): 7.54 (m, 2H), 6.68 (m, 2H), 6.30 (s, 2H), 4.90 (tr, 1 H), 3.68 (m, 4H), 2.95 (m, 2H), 0.95 (m, 2H), 0.01 (s, 9H).

### zm) Herstellung von (RS)-S-(4-Amino-2-methylphenyl)-N-(ethoxycarbonyl)-S-methylsulfoximid

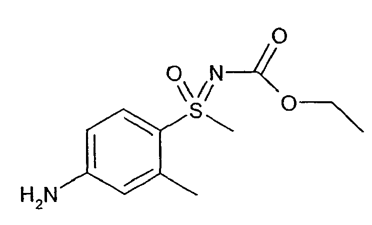

¹H-NMR (DMSO-D6): 7.53 (m, 1 H), 6.48 (m, 2H), 6.04 (s, 2H), 3.90 (q, 2H), 3.30 (s, 3H), 2.42 (s, 3H), 1.13 (tr, 3H).

### zn) Herstellung von (RS)-S-(4-Aminophenyl)-N,S-dimethylsulfoximid

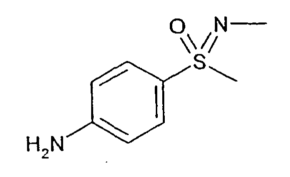

¹H-NMR (DMSO-D6): 7.48 (d, 2H), 6.62 (d, 2H), 5.95 (s, 2H), 2.95 (s, 3H), 2.41 (s, 3H).

### zo) Herstellung von (RS)-S-(4-Amino-2-fluorphenyl)-N-(ethoxycarbonyl)-S-methylsulfoximid

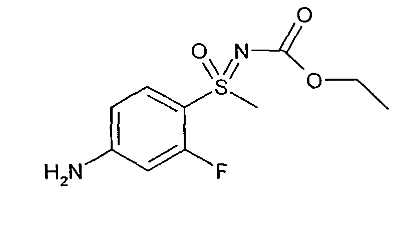

¹H-NMR (DMSO): 7.45 (m, 1H), 6.48 (m, 4H), 3.88 (m, 2H), 3.30 (s, 3H), 1.10 (tr, 3H).

### zp) Herstellung von (RS)-S-[4-Amino-2-(trifluormethyl)phenyl]-N-(ethoxycarbonyl)-S-methylsulfoximid

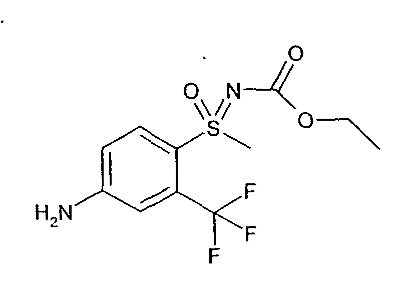

¹H-NMR (DMSO): 7.78 (m, 1 H), 7.12 (m, 1 H), 6.84 (m, 1H), 6.63 (s, 2H), 3.89 (q, 2H), 3.30 (s, 3H), 1.08 (tr, 3H).
MS: 311 (ES).

Die nachfolgenden Beispiele beschreiben die biologische Wirkung der erfindungsgemäßen Verbindungen.

### Beispiel 1

### CDK1/CycB Kinase Assay

Rekombinante CDK1- und CycB-GST-Fusionsproteine, gereinigt aus Bakulovirus-infizierten Insektenzellen (Sf9), wurden von ProQinase GmbH, Freiburg, gekauft. Das als Kinase-Substrat verwendet Histon IIIS ist über die Fa. Sigma käuflich zu erwerben.

CDK1/CycB (200 ng/Meßpunkt) wurde für 15 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl2, 0,1 mM Na ortho-Vanadat, 1,0 mM Dithiothreitol, 0,5 µM Adenosintrisphosphat (ATP), 10 µg/Meßpunkt Histon IIIS, 0,2 µCi/Messpunkt 33P-gamma ATP, 0,05% NP40, 12,5% Dimethylsulfoxid] inkubiert. Die Reaktion wurde durch Zugabe von EDTA-Lösung (250 mM, pH8,0, 14 µl/Meßpunkt) gestoppt.

Von jedem Reaktionsansatz wurden 10 µl auf P30 Filterstreifen (Fa. Wallac) aufgetragen, und nicht-eingebautes 33P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLexTM A, Fa. Wallac) bedeckt und für 1 Stunde bei 90°C eingebrannt. Die Menge an eingebautem 33P (Substratphosphorylierung) wurde durch Szintillationsmessung in einem Gamma-Strahlungsmessgerät (Wallac) bestimmt.

### Beispiel 2

### CDK2/CycE Kinase Assay

Rekombinante CDK2- und CycE-GST-Fusionsproteine, gereinigt aus Bakulovirus-infizierten Insektenzellen (Sf9), wurden von ProQinase GmbH, Freiburg, gekauft. Histon IIIS, das als Kinase-Substrat verwendet wurde, wurde bei der Fa. Sigma gekauft.

CDK2/CycE (50 ng/Meßpunkt) wurde für 15 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM Adenosintrisphosphat (ATP), 10 µg/Messpunkt Histon IIIS, 0,2 µCi/Messpunkt ³³P-gamma ATP, 0,05% NP40, 12,5% Dimethylsulfoxid] inkubiert. Die Reaktion wurde durch Zugabe von EDTA-Lösung (250 mM, pH8,0, 14 µl/Meßpunkt) gestoppt.

Von jedem Reaktionsansatz wurden 10 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex^{™} A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt.

### Beispiel 3

### VEGF Rezeptor-2 Kinase Assay

Rekombinante VEGF Rezeptortyrosinkinase-2 wurde als GST-Fusionsprotein aus Bakulovirus-infizierten Insektenzellen (Sf9) gereinigt. Poly-(Glu4Tyr), das als Kinase-Substrat verwendet wurde, wurde bei der Fa. Sigma gekauft. VEGF Rezeptortyrosinkinase (90 ng/Meßpunkt) wurde für 10 min bei 22°C in Anwesenheit verschiedener Konzentrationen an Testsubstanzen (0 µM, sowie innerhalb des Bereiches 0,001 - 30 µM) in 30 µl Assaypuffer [40 mM Tris/HCl pH5,5, 10 mM MgCl2, 1 mM MnCl2, 3 µM Na ortho-Vanadat, 1,0 mM Dithiothreitol, 8 µM Adenosintrisphosphat (ATP), 27 µg/Meßpunkt poly-(Glu4Tyr), 0,2 µCi/Meßpunkt 33P-gamma ATP, 1% Dimethylsulfoxid] inkubiert. Die Reaktion wurde durch Zugabe von EDTA-Lösung (250 mM, pH7,0, 10 µl/Meßpunkt) gestoppt.

Von jedem Reaktionsansatz wurden 10 µl auf P30 Filterstreifen (Fa. Wallac) aufgetragen, und nicht-eingebautes 33P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLexTM A, Fa. Wallac) bedeckt und für 1 Stunde bei 90°C eingebrannt. Die Menge an eingebautem 33P (Substratphosphorylierung) wurde durch Szintillationsmessung in einem gamma-Strahlungsmeßgerät (Wallac) bestimmt. Die IC50-Werte bestimmen sich aus der Inhibitorkonzentration, die notwendig ist, um den Phosphateinbau auf 50% des ungehemmten Einbaus nach Abzug des Leerwertes (EDTAgestoppte Reaktion) zu hemmen.

### Beispiel 4

### Proliferationsassay

Kultivierte humane Tumorzellen (MCF7, hormonunabhängige menschliche Mammakarzinomazellen, bezogen von ATCC HTB22; NCl-H460, menschliche nicht kleinzellige Lungenkarzinomazellen, ATCC HTB-177, HCT 116, menschliche Kolonkarzinomazellen, ATCC CCL-247; DU 145, hormonunabhängige menschliche Prostatakarzinomzellen, ATCC HTB-81; MaTu-MDR, hormonunabhängige, multiple Arzneimittel resistente menschliche Mammakarzinomzellen, EPO-GmbH, Berlin) wurden in einer Dichte von ca. 5000 Zellen/Messpunkt, je nach Wachstumsgeschwindigkeit der jeweiligen Zellen in einer 96-Loch Multititerplatte in 200 µl des entsprechenden Wachstumsmediums ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Messpunkt einer 11 %igen Glutaraldehyd-Lösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Messpunkt einer 0,1 %igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Messpunkt einer 10%igen Essigsäure-Lösung gelöst. Die Extinktion wurde photometrisch bei einer Wellenlänge von 595 nm bestimmt. Die prozentuale Änderung des Zellwachstums wurde durch Normalisierung der Messwerte auf die Extinktionwerte der Nullpunktplatte (=0%) und die Extinktion der unbehandelten (0 µM) Zellen (=100%) berechnet.

### Beispiel 5

### Carboanhydraseassay

Das Prinzip des Assays beruht auf der Hydrolyse von 4-Nitrophenyl Acetat durch Carbonanhydrasen (Pocker & Stone, Biochemistry, 1967, 6, 668.), mit anschließender photometrischer Bestimmung des entstandenen Farbstoffs 4-Nitrophenolat bei 400 nm mittels eines 96-Kanal Spektralphotometers.

2µl der Testverbindungen, gelöst in DMSO (100x der finalen Konzentration), in einem Konzentrationsbereich von 0.03 - 10 µM (final) wurden als Vierfachbestimmungen in die Löcher einer 96-Loch Mikrotiter-Platte pipettiert. Löcher, die Lösungsmittel ohne Testverbindungen enthielten dienten al Referenzwerte (1. Löcher ohne Carboanhydrase zur Korrektur der nichtenzymatischen Hydrolyse des Substrates, und 2. Löcher mit Carboanhydrase zur Bestimmung der Aktivität des nicht-inhibierten Enzyms).

188 µl Assaypuffer (10 mM Tris/HCl pH7.4, 80 mM NaCl) ohne oder mit 3 units/Loch an Carboanhydrase I oder II wurdenin die Löcher der Mikrotiterplatte pipettiert. Die enzymatische Reaktion wurde durch die Zugabe von 10 µl der Substratlösung (1 mM 4-Nitrophenyl Acetat (Fluka #4602), gelöst in Wasserfreiem Acetonitril, gestartet (finale Substratkonzentration: 50 µM). The Platte wurde bei Raumtemperatur 15 min inkubiert. Die Extinktionen wurden photometrisch bei einer Wellenlänge von 400 nm gemessen. Die Enzyminhibition wurde nach Normalisation der Meßwerte auf die Extinktion der Reaktionen in den Löchern ohne Enzym (=100% Inhibition) und auf die Extinktion der Reaktionen in den Löchern mit nicht-inhibiertem Enzym (=0% Inhibition) berechnet.

Die Ergebnisse aus den Beispielen und die Vergleichsdaten sind in den nachfolgenden Tabellen 1 bis 3 angegeben. Zum Nachweis der Überlegenheit der erfindungsgemäßen Verbindungen gegenüber den bekannten Verbindungen wurden die erfindungsgemäßen Verbindungen mit bekannten Referenzverbindungen und einer strukturähnlichen bekannten Verbindung Bsp. 10 aus WO 00/096888 in Enzymtests verglichen. Das Ergebnis ist in den folgenden Tabellen 1 und 2 aufgeführt. In Tabelle 3 werden die verbesserten Daten der erfindungsgemäßen Verbindungen im Vergleich zu Verbindung Bsp. 10 aus WO 00/12486 und Acetazolamid dargestellt

**Tabelle 1**

| **Beispiel-Nr.** | **Proliferation IC₅₀ [**µ**M]** | | | | |
|---|---|---|---|---|---|
| | **MCF7** | **H460** | **HCT116** | **DU145** | **MaTu-ADR** |
| 1.0 | 0.3 | 1.2 | 0.4 | 1.5 | 1.6 |
| 2.0 | 1.5 | 0.3 | 0.3 | 1.7 | 0.4 |
| 1.3 | <0.1 | 0.14 | 0.10 | 0.2 | 0.17 |
| 1.4 | 0.06 | 0.06 | 0.05 | 0.10 | 0.08 |
| 1.2 | 0.11 | 0.03 | 0.02 | 0.04 | 0.04 |
| 2.1 | 0.9 | | | | |
| 2.3 | 0.3 | 0.4 | | 0.19 | 0.12 |
| 1.23 | 0.13 | <0.1 | | 0.1 | <0.1 |
| 1.24 | <0.1 | 0.07 | | 0.13 | 0.08 |
| 1.25 | <0.1 | | | | |
| 1.31 | 0.2 | 0.18 | | 0.3 | 0.7 |
| 1.41 | 0.08 | 0.07 | | 0.09 | 0.07 |
| 1.42 | 0.15 | <0.1 | | 0.17 | <0.1 |
| 1.7 | 1.1 | | | | |
| 1.26 | 0.06 | 0.03 | | 0.07 | 0.04 |
| 1.27 | 0.06 | 0.02 | | 0.13 | 0.03 |
| 1.10 | 0.5 | 0.7 | | 0.8 | 0.8 |
| 1.39 | 0.3 | 0.3 | | 0.3 | 0.9 |
| 1.33 | 1 | | | | |
| 1.35 | 0.11 | 0.12 | | 0.12 | 0.3 |
| 1.34 | 0.8 | | | | |
| 1.40 | 0.11 | 0.17 | | 0.18 | 0.3 |
| 1.63 | 0.9 | | | | |
| 1.48 | 0.3 | 0.3 | | 0.4 | 0.6 |
| 1.54 | 0.11 | 0.12 | | 0.19 | 0.07 |
| 1.11 | 0.1 | <0.1 | | <0.1 | 0.1 |
| 1.9 | 0.1 | 0.11 | | 0.1 | 0.1 |
| 1.12 | 0.2 | 0.4 | | 0.3 | 2.8 |
| 1.6 | 0.14 | <0.1 | | <0.1 | <0.1 |
| 1.37 | 0.17 | <0.1 | | 0.16 | 0.3 |
| 1.57 | <0.1 | 0.12 | | 0.11 | 0.09 |
| 1.49 | 0.3 | 0.4 | | 0.3 | 0.6 |
| 1.50 | 1.2 | | | | |
| 1.55 | 0.3 | 0.3 | | 0.3 | 0.15 |
| 1.56 | 0.02 | 0.1 | | 0.07 | 0.05 |
| 1.46 | 0.2 | 0.11 | | 0.17 | 0.2 |
| 1.47 | 0.6 | 0.6 | | 0.6 | 0.8 |
| 1.16 | 0.18 | 0.2 | | 0.19 | 4.0 |
| 1.20 | 0.2 | 0.4 | | 0.4 | 2.1 |
| 1.38 | 0.12 | 0.06 | | 0.13 | 0.4 |
| 1.36 | 0.14 | 0.12 | | 0.17 | 1.2 |
| 1.51 | 0.08 | 0.05 | | 0.05 | 0.06 |
| 1.60 | 0.06 | 0.04 | | 0.04 | 0.04 |
| 1.14 | 0.09 | 0.10 | | 0.09 | 0.11 |
| 1.15 | 0.18 | 0.2 | | 0.3 | 0.3 |
| 1.32 | 0.19 | 0.18 | | 0.3 | 0.6 |
| 1.28 | 0.17 | 0.12 | | 0.2 | 0.2 |
| 3.4 | 1.0 | | | | |
| 3.5 | 0.12 | 0.05 | | 0.06 | 0.03 |
| 1.58 | 0.06 | 0.03 | | 0.03 | 0.04 |
| 1.59 | 0.11 | <0.1 | | <0.1 | <0.1 |
| 3.0 | 0.5 | | | | |
| 3.6 | 0.08 | 0.02 | | 0.02 | 0.02 |
| 3.7 | 0.1 | <0.1 | | <0.1 | <0.1 |
| 3.8 | 0.4 | 0.3 | | 0.3 | 0.19 |
| 3.1 | 0.4 | | | | |
| 1.29 | 0.17 | | | | |
| 1.30 | 0.17 | | | | |
| 3.10 | 0.4 | | | | |
| 3.9 | 1.0 | | | | |
| 1.18 | <0.1 | | | | |
| 1.21 | <0.1 | | | | |
| 1.52 | <0.1 | | | | |
| 1.53 | 0.3 | | | | |
| 1.19 | <0.1 | | | | |
| 1.43 | <0.1 | | | | |
| 1.44 | 0.13 | | | | |
| Bsp. 10 aus WO 02/096888 | 0.4 | 0.6 | 0.4 | 0.7 | 0.8 |

**Tabelle 2**

| **Beispiel-Nr.** | **CDK2/CycE** **IC₅₀ [nM]** | **CDK1/CycB** **IC₅₀ [nM]** | **VEGF-R2** **IC₅₀ [nM]** |
|---|---|---|---|
| 2.0 | 16 | 110 | 70 |
| 1.0 | <10 | 79 | 40 |
| 1.3 | 6 | 10 | 140 |
| 1.4 | 10 | 13 | 340 |
| 1.2 | 20 | 130 | 48 |
| 2.1 | 390 | >1000 | 74 |
| 2.3 | 33 | 160 | 61 |
| 1.23 | 6 | 8 | 75 |
| 1.24 | 8 | 5 | 150 |
| 1.25 | 3 | 2 | 70 |
| 1.31 | 9 | 27 | 140 |
| 1.41 | 2 | 2 | 76 |
| 1.42 | 2 | 5 | 64 |
| 1.7 | >1000 | >1000 | 240 |
| 1.26 | 4 | 2 | 31 |
| 1.27 | 4 | 3 | 97 |
| 1.10 | >1000 | >1000 | 910 |
| 1.39 | 19 | 49 | 150 |
| 1.33 | 51 | 200 | 450 |
| 1.35 | 42 | 96 | 94 |
| 1.34 | 28 | 110 | 530 |
| 1.40 | 14 | 21 | 110 |
| 1.63 | 63 | 200 | 89 |
| 1.48 | 7 | 16 | 270 |
| 1.54 | 5 | 8 | 69 |
| 1.11 | 25 | 44 | 83 |
| 1.9 | 4 | 5 | 49 |
| 1.12 | 49 | 160 | 160 |
| 1.6 | 8 | 14 | 29 |
| 1.37 | 48 | 63 | 57 |
| 1.57 | 4 | 8 | 66 |
| 1.49 | 9 | 15 | 470 |
| 1.50 | 9 | 44 | 230 |
| 1.55 | 27 | 45 | 79 |
| 1.56 | 24 | 68 | 32 |
| 1.46 | 4 | 11 | 340 |
| 1.47 | 6 | 27 | 300 |
| 1.16 | 130 | 170 | 130 |
| 1.20 | 54 | 160 | 820 |
| 1.38 | 78 | 75 | 59 |
| 1.36 | 11 | 43 | 92 |
| 1.51 | 4 | 5 | 26 |
| 1.60 | 4 | 4 | 39 |
| 1.14 | 4 | 7 | 69 |
| 1.15 | 4 | 25 | 59 |
| 1.32 | 12 | 16 | 56 |
| 1.28 | 7 | 14 | 37 |
| 3.4 | 41 | 72 | 250 |
| 3.5 | 8 | 17 | 150 |
| 1.58 | 7 | 4 | 45 |
| 1.59 | 7 | 9 | 48 |
| 3.0 | 16 | 49 | 170 |
| 3.6 | 18 | 22 | 200 |
| 3.7 | 11 | 19 | 110 |
| 3.8 | 27 | 91 | >1000 |
| 3.1 | 33 | 97 | 120 |
| 1.29 | 4 | 7 | 16 |
| 1.30 | 6 | 15 | 29 |
| 3.10 | 4 | 18 | |
| 3.9 | 8 | 55 | |
| 1.18 | 3 | 3 | |
| 1.21 | 6 | 5 | |
| 1.53 | 4 | 11 | |
| 1.19 | 3 | 7 | |
| 1.44 | 2 | 5 | |
| Bsp. 10 aus WO 02/096888 | <10 | 90 | 200 |

**Tabelle 3**

| **Beispiel-Nr.** | **Inhibition der humanen** **Carboanhydrase-2** **IC₅₀ [nM]** |
|---|---|
| Bsp. 1.0 | >10000 |
| Bsp. 2.0 | >10000 |
| Acetazolamid | 51 |
| Bsp. 10 aus WO 02/096888 | 190 |

Tabellen 1 und 2 zeigen, dass die erfindungsgemäßen Verbindungen zyklin-abhängige Kinasen und/oder VEGF-Rezeptortyrosinkinasen im nanomolaren Bereich inhibieren und somit die Proliferation der Tumorzellen und/oder die Tumorangiogenese inhibieren können.

Tabelle 3 zeigt, dass erfindungsgemäße Substanzen im Gegensatz zu Verbindungen aus dem Stand der Technik, wie z.B. Acetazolamid oder Bsp. 10 aus WO02/096888, das den engsten Stand der Technik darstesllt, keine meßbare Carboanhydrase Inihbition aufweisen und somit eine möglichen Nebenwirkung, die auf die Carboanhydraseinhibierung zurückzuführen sein könnte, nicht mehr aufweisen.

Insofern belegen die vorgenannten Tabellen, dass die erfindungsgemäßen Substanzen im Vergleich zum Stand überlegen sind.

## Patentansprüche

1. Verbindungen der allgemeinen Formel (I) 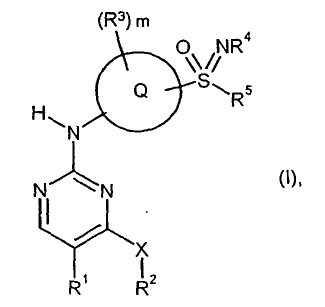 in der
Q für die Gruppe 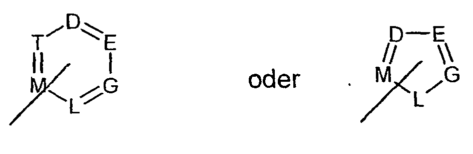
D, E, G, L, M und T jeweils unabhängig voneinander für Kohlenstoff, Sauerstoff, Stickstoff oder Schwefel stehen,
R¹ für Wasserstoff, Halogen, C₁-C₆-Alkyl, CF₃, CN, Nitro oder für die Gruppe -COR⁸ oder -O-C₁-C₆-Alkyl steht,
R² für Wasserstoff oder gegebenenfalls ein- oder mehrfach, gleich oder verschieden mit Hydroxy, Halogen, C₁-C₆-Alkoxy, Amino, Cyano, C₁-C₆-Alkyl, -NH-(CH₂)ₙ-C₃-C₁₀-Cycloalkyl, - C₃-C₁₀-Cycloalkyl, C₁-C₆-Hydroxyalkyl, C₂-C₆-Alkenyl, C₂-C₆- Alkinyl, C₁-C₆-Alkoxy-C₁-C₆-Alkyl, C₁-C₆-Alkoxy-C₁-C₆-Alkoxy- C₁-C₆-Alkyl, -NHC₁-C₆-Alkyl, -N(C₁-C₆-Alkyl)₂, C₁-C₆-Alkanoyl, -CONR⁹R¹⁰, -COR⁸, C₁-C₆-AlkylOAc, Carboxy, Aryl, Heteroaryl, -(CH₂)ₙ-Aryl, -(CH₂)ₙ-Heteroaryl, Phenyl-(CH₂)ₙ- R⁸, -(CH₂)ₙPO₃(R⁸)₂ oder mit der Gruppe -R⁶ oder -NR⁹R¹⁰ substituiertes C₁-C₁₀-Alkyl, C₂-C₁₀-Alkenyl, C₂-C₁₀-Alkinyl, C₃- C₁₀-Cycloalkyl, Aryl oder Heteroaryl steht und das Phenyl, C₃- C₁₀-Cycloalkyl, Aryl, Heteroaryl, -(CH₂)ₙ-Aryl und -(CH₂)ₙ- Heteroaryl selbst gegebenenfalls ein- oder mehrfach, gleich oder verschieden mit Halogen, Hydroxy, C₁-C₆-Alkyl, C₁-C₆- Alkoxy, oder mit der Gruppe -CF₃ oder -OCF₃ substituiert sein kann, und der Ring des C₃-C₁₀-Cycloalkyls und das C₁-C₁₀- Alkyl gegebenenfalls durch ein- oder mehrere Stickstoff, Sauerstoff und/ oder Schwefel-Atome unterbrochen sein kann und / oder durch ein oder mehrere -C(O)- Gruppen im Ring unterbrochen sein kann und / oder gegebenenfalls ein oder mehrere mögliche Doppelbindungen im Ring enthalten sein können,
X für Sauerstoff, Schwefel oder für die Gruppe -NH- oder -N(C₁- C₃-Alkyl)- steht,
oder X und R² gemeinsam einen C₃-C₁₀ -Cycloalkyl-Ring bilden, der gegebenenfalls ein oder mehrere Heteroatome enthalten kann, und gegebenenfalls ein- oder mehrfach, gleich oder verschieden mit Hydroxy, C₁-C₆-Alkyl, C₁-C₆-Alkoxy, Halogen oder der Gruppe -NR⁹R¹⁰ substituiert sein kann,
R³ für Wasserstoff, Hydroxy, Halogen, CF₃, OCF₃ oder für die Gruppe -NR⁹R¹⁰ oder für gegebenenfalls ein- oder mehrfach, gleich oder verschieden mit Halogen, Hydroxy, C₁-C₆-Alkoxy oder der Gruppe -NR⁹R¹⁰ substituiertes C₁-C₆-Alkyl, C₃-C₆- Cycloalkyl oder C₁-C₆-Alkoxy steht,
m für 0 - 4 steht,
R⁴ für Wasserstoff oder für die Gruppe -COR⁸, NO₂, Trimethylsilanyl (TMS), tert.-Butyl-dimethylsilanyl (TBDMS), tert.-Butyl-diphenylsilanyl (TBDPS), Triethylsilanyl (TES) oder -SO₂R⁷ steht oder für gegebenenfalls ein- oder mehrfach, gleich oder verschieden mit Hydroxy, Halogen, C₁-C₆-Alkoxy, C₁-C₆-Alkylthio, Cyano, C₃-C₁₀-Cycloalkyl, C₁-C₆- Hydroxyalkyl, C₂-C₆-Alkenyl, C₂-C₆-Alkinyl, C₁-C₆-Alkoxy-C₁- C₆-Alkyl, C₁-C₆-Alkoxy-C₁-C₆-Alkoxy-C₁-C₆-Alkyl oder mit der Gruppe -CONR⁹R¹⁰, -COR⁸, -CF₃, -OCF₃ oder -NR⁹R¹⁰ substituiertes C₁-C₁₀-Alkyl oder C₃-C₁₀-Cycloalkyl, steht,
R⁵ für gegebenenfalls ein- oder mehrfach, gleich oder verschieden mit Hydroxy, C₁-C₆-Alkoxy, C₃-C₁₀-Cycloalkyl, Halogen oder der Gruppe -NR⁹R¹⁰ substituiertes C₁-C₁₀-Alkyl, C₂-C₆-Alkenyl, C₂-C₆-Alkinyl oder C₃-C₁₀-Cycloalkyl steht,
oder R⁴ und R⁵ gemeinsam einen C₅-C₁₀-Cycloalkylring der Gruppe 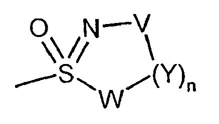 bilden kann, wobei
V, W und Y jeweils unabhängig voneinander für gegebenenfalls ein- oder mehrfach, gleich oder verschieden mit Hydroxy, C₁-C₁₀-Alkyl, C₁-C₁₀-Alkoxy oder -NR⁹R¹⁰ substituiertes -CH₂- steht, wobei C₁-C₁₀-Alkyl oder C₁-C₁₀-Alkoxy ebenfalls ein oder mehrfach, gleich oder verschieden mit Hydroxy, -NR⁹R¹⁰ oder C₁-C₁₀- Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
R⁶ für einen Heteroaryl oder einen C₃-C₁₀-Cycloalkyl-Ring, der gegebenenfalls ein oder mehrere Heteroatome enthalten kann und gegebenenfalls ein- oder mehrfach, gleich oder verschieden mit Hydroxy, C₁-C₆-Alkyl, C₁-C₆-Alkoxy oder Halogen substituiert sein kann, steht,
R⁷ für gegebenenfalls ein- oder mehrfach, gleich oder verschieden mit Halogen, Hydroxy, C₁-C₆-Alkyl, C₁-C₆-Alkoxy oder mit der Gruppe Trimethylsilanyl (TMS) oder -NR⁹R¹⁰ substituiertes C₁-C₁₀-Alkyl oder Aryl steht,
R⁸ für Wasserstoff, C₁-C₆-Alkyl , Hydroxy, C₁-C₆-Alkoxy, C₁-C₆- Alkylthio, Benzoxy oder -NR⁹R¹⁰ steht,
R⁹ und R¹⁰ jeweils unabhängig voneinander für Wasserstoff, C₁-C₆-Alkyl, C₁-C₆-Alkoxy, Hydroxy, Hydroxy-C₁-C₆-Alkyl, Dihydroxy-C₁- C₆-Alkyl, Phenyl, Heteroaryl oder für die Gruppe - (CH₂)ₙNR⁹R¹⁰, -CNHNH₂ oder -NR⁹R¹⁰ stehen,
oder R⁹ und R¹⁰ gemeinsam einen C₃-C₁₀-Cycloalkyl-Ring bilden, der gegebenenfalls durch ein- oder mehrere Stickstoff, Sauerstoff und/ oder Schwefel-Atome unterbrochen sein kann und/ oder durch ein oder mehrere -C(O)- Gruppen im Ring unterbrochen sein kann und/ oder gegebenenfalls ein oder mehrere mögliche Doppelbindungen im Ring enthalten sein können, steht und
n für 1 - 6 steht,
bedeuten, sowie deren Isomeren, Diastereomeren, Enantiomeren und/oder Salze.

2. Verbindungen der allgemeinen Formel (I) gemäß Anspruch 1, in der
Q für Aryl steht,
R¹ für Wasserstoff, Halogen, C₁-C₆-Alkyl, CF₃, CN, Nitro oder für die Gruppe -COR⁸ oder -O-C₁-C₆-Alkyl steht,
R² für Wasserstoff oder gegebenenfalls ein- oder mehrfach, gleich oder verschieden mit Hydroxy, Halogen, C₁-C₆-Alkoxy, Amino, Cyano, C₁-C₆-Alkyl, -NH-(CH₂)ₙ-C₃-C₁₀-Cycloalkyl, - C₃-C₁₀-Cycloalkyl, C₁-C₆-Hydroxyalkyl, C₂-C₆-Alkenyl, C₂-C₆- Alkinyl, C₁-C₆-Alkoxy-C₁-C₆-Alkyl, C₁-C₆-Alkoxy-C₁-C₆-Alkoxy- C₁-C₆-Alkyl, -NHC₁-C₆-Alkyl, -N(C₁-C₆-Alkyl)₂, C₁-C₆-Alkanoyl, -CONR⁹R¹⁰, -COR⁸ , C₁-C₆-AlkylOAc, Carboxy, Aryl, Heteroaryl, -(CH₂)ₙ-Aryl, -(CH₂)ₙ-Heteroaryl, Phenyl-(CH₂)ₙ- R⁸, -(CH₂)ₙPO₃(R⁸)₂ oder mit der Gruppe -R⁶ oder -NR⁹R¹⁰ substituiertes C₁-C₁₀-Alkyl, C₂-C₁₀-Alkenyl, C₂-C₁₀-Alkinyl, C₃- C₁₀-Cycloalkyl, Aryl oder Heteroaryl steht und das Phenyl, C₃- C₁₀-Cycloalkyl, Aryl, Heteroaryl, -(CH₂)ₙ-Aryl und -(CH₂)ₙ- Heteroaryl selbst gegebenenfalls ein- oder mehrfach, gleich oder verschieden mit Halogen, Hydroxy, C₁-C₆-Alkyl, C₁-C₆- Alkoxy, oder mit der Gruppe -CF₃ oder -OCF₃ substituiert sein kann, und der Ring des C₃-C₁₀-Cycloalkyls und das C₁-C₁₀- Alkyl gegebenenfalls durch ein- oder mehrere Stickstoff, Sauerstoff und/ oder Schwefel-Atome unterbrochen sein kann und / oder durch ein oder mehrere -C(O)- Gruppen im Ring unterbrochen sein kann und / oder gegebenenfalls ein oder mehrere mögliche Doppelbindungen im Ring enthalten sein können,
X für Sauerstoff, Schwefel oder für die Gruppe -NH-, -N(C₁-C₃- Alkyl)- steht,
oder X und R² gemeinsam einen C₃-C₁₀ -Cycloalkyl-Ring bilden, der gegebenenfalls ein oder mehrere Heteroatome enthalten kann, und gegebenenfalls ein- oder mehrfach, gleich oder verschieden mit Hydroxy, C₁-C₆-Alkyl, C₁-C₆-Alkoxy, Halogen oder der Gruppe -NR⁹R¹⁰ substituiert sein kann,
R³ für Wasserstoff, Hydroxy, Halogen, CF₃, OCF₃ oder für die Gruppe -NR⁹R¹⁰ oder für gegebenenfalls ein- oder mehrfach, gleich oder verschieden mit Halogen, Hydroxy, C₁-C₆-Alkoxy oder der Gruppe -NR⁹R¹⁰ substituiertes C₁-C₆-Alkyl, C₃-C₆- Cycloalkyl oder C₁-C₆-Alkoxy steht,
m für 0 - 4 steht,
R⁴ für Wasserstoff oder für die Gruppe -COR⁸, NO₂, Trimethylsilanyl (TMS), tert.-Butyl-dimethylsilanyl (TBDMS), tert.-Butyl-diphenylsilanyl (TBDPS), Triethylsilanyl (TES) oder -SO₂R⁷ steht oder für gegebenenfalls ein- oder mehrfach, gleich oder verschieden mit Hydroxy, Halogen, C₁-C₆-Alkoxy, C₁-C₆-Alkylthio, Cyano, C₃-C₁₀-Cycloalkyl, C₁-C₆- Hydroxyalkyl, C₂-C₆-Alkenyl, C₂-C₆-Alkinyl, C₁-C₆-Alkoxy-C₁- C₆-Alkyl, C₁-C₆-Alkoxy-C₁-C₆-Alkoxy-C₁-C₆-Alkyl oder mit der Gruppe -CONR⁹R¹⁰, -COR⁸, -CF₃, -OCF₃ oder -NR⁹R¹⁰ substituiertes C₁-C₁₀-Alkyl, C₃-C₁₀-Cycloalkyl, steht,
R⁵ für gegebenenfalls ein- oder mehrfach, gleich oder verschieden mit Hydroxy, C₁-C₆-Alkoxy, C₃-C₁₀-Cycloalkyl, Halogen oder der Gruppe -NR⁹R¹⁰ substituiertes C₁-C₁₀-Alkyl, C₂-C₆-Alkenyl, C₂-C₆-Alkinyl oder C₃-C₁₀-Cycloalkyl steht,
oder R⁴ und R⁵ gemeinsam einen C₅-C₁₀-Cycloalkylring der Gruppe 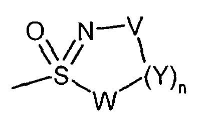 bilden kann, wobei
V, W und Y jeweils unabhängig voneinander für gegebenenfalls ein- oder mehrfach, gleich oder verschieden mit Hydroxy, C₁-C₁₀-Alkyl, C₁-C₁₀-Alkoxy oder -NR⁹R¹⁰ substituiertes -CH₂- steht, wobei C₁-C₁₀-Alkyl oder C₁-C₁₀-Alkoxy ebenfalls ein oder mehrfach, gleich oder verschieden mit Hydroxy, -NR⁹R¹⁰ oder C₁-C₁₀- Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
R⁶ für einen Heteroaryl oder einen C₃-C₁₀-Cycloalkyl-Ring, der gegebenenfalls ein oder mehrere Heteroatome enthalten kann und gegebenenfalls ein- oder mehrfach, gleich oder verschieden mit Hydroxy, C₁-C₆-Alkyl, C₁-C₆-Alkoxy oder Halogen substituiert sein kann, steht,
R⁷ für gegebenenfalls ein- oder mehrfach, gleich oder verschieden mit Halogen, Hydroxy, C₁-C₆-Alkyl, C₁-C₆-Alkoxy oder mit der Gruppe Trimethylsilanyl (TMS) oder -NR⁹R¹⁰ substituiertes C₁-C₁₀-Alkyl oder Aryl steht,
R⁸ für Wasserstoff, C₁-C₆-Alkyl , Hydroxy, C₁-C₆-Alkoxy, C₁-C₆- Alkylthio, Benzoxy oder -NR⁹R¹⁰ steht,
R⁹ und R¹⁰ jeweils unabhängig voneinander für Wasserstoff, C₁-C₆-Alkyl, C₁-C₆-Alkoxy, Hydroxy, Hydroxy-C₁-C₆-Alkyl, Dihydroxy-C₁- C₆-Alkyl, Phenyl, Heteroaryl oder für die Gruppe - (CH₂)ₙNR⁹R¹⁰, -CNHNH₂ oder -NR⁹R¹⁰ stehen,
oder R⁹ und R¹⁰ gemeinsam einen C₃-C₁₀-Cycloalkyl-Ring bilden, der gegebenenfalls durch ein- oder mehrere Stickstoff, Sauerstoff und/ oder Schwefel-Atome unterbrochen sein kann und/ oder durch ein oder mehrere -C(O)- Gruppen im Ring unterbrochen sein kann und/ oder gegebenenfalls ein oder mehrere mögliche Doppelbindungen im Ring enthalten sein können, steht und
n für 1 - 6 steht,
bedeuten, sowie deren Isomeren, Diastereomeren, Enantiomeren und/oder Salze.

3. Verbindungen der allgemeinen Formel (I) gemäß Anspruch 1 oder 2, in der
Q für Phenyl steht,
R¹ für Wasserstoff, Halogen, C₁-C₆-Alkyl, CF₃, CN, Nitro oder für die Gruppe -COR⁸ oder -O-C₁-C₆-Alkyl steht,
R² für Wasserstoff oder gegebenenfalls ein- oder mehrfach, gleich oder verschieden mit Hydroxy, Halogen, C₁-C₆-Alkoxy, Amino, Cyano, C₁-C₆-Alkyl, -NH-(CH₂)ₙ-C₃-C₁₀-Cycloalkyl, - C₃-C₁₀-Cycloalkyl, C₁-C₆-Hydroxyalkyl, C₂-C₆-Alkenyl, C₂-C₆- Alkinyl, C₁-C₆-Alkoxy-C₁-C₆-Alkyl, C₁-C₆-Alkoxy-C₁-C₆-Alkoxy- C₁-C₆-Alkyl, -NHC₁-C₆-Alkyl, -N(C₁-C₆-Alkyl)₂, C₁-C₆-Alkanoyl, -CONR⁹R¹⁰, -COR⁸ , C₁-C₆-AlkylOAc, Carboxy, Aryl, Heteroaryl, -(CH₂)ₙ-Aryl, -(CH₂)ₙ-Heteroaryl, Phenyl-(CH₂)ₙ- R⁸, -(CH₂)ₙPO₃(R⁸)₂ oder mit der Gruppe -R⁶ oder -NR⁹R¹⁰ substituiertes C₁-C₁₀-Alkyl, C₂-C₁₀-Alkenyl, C₂-C₁₀-Alkinyl, C₃- C₁₀-Cycloalkyl, Aryl oder Heteroaryl steht und das Phenyl, C₃- C₁₀-Cycloalkyl, Aryl, Heteroaryl, -(CH₂)ₙ-Aryl und -(CH₂)ₙ- Heteroaryl selbst gegebenenfalls ein- oder mehrfach, gleich oder verschieden mit Halogen, Hydroxy, C₁-C₆-Alkyl, C₁-C₆- Alkoxy, oder mit der Gruppe -CF₃ oder -OCF₃ substituiert sein .kann, und der Ring des C₃-C₁₀-Cycloalkyls und das C₁-C₁₀- Alkyl gegebenenfalls durch ein- oder mehrere Stickstoff, Sauerstoff und/ oder Schwefel-Atome unterbrochen sein kann und / oder durch ein oder mehrere -C(O)- Gruppen im Ring unterbrochen sein kann und / oder gegebenenfalls ein oder mehrere mögliche Doppelbindungen im Ring enthalten sein können,
X für Sauerstoff, Schwefel oder für die Gruppe -NH-, -N(C₁-C₃- Alkyl)- steht,
oder X und R² gemeinsam einen C₃-C₁₀ -Cycloalkyl-Ring bilden, der gegebenenfalls ein oder mehrere Heteroatome enthalten kann, und gegebenenfalls ein- oder mehrfach mit Hydroxy, C₁-C₆-Alkyl, C₁-C₆-Alkoxy, Halogen oder der Gruppe -NR⁹R¹⁰ substituiert sein kann,
R³ für Wasserstoff, Hydroxy, Halogen, CF₃, OCF₃ oder für die Gruppe -NR⁹R¹⁰ oder für gegebenenfalls ein- oder mehrfach, gleich oder verschieden mit Halogen, Hydroxy, C₁-C₆-Alkoxy oder der Gruppe -NR⁹R¹⁰ substituiertes C₁-C₆-Alkyl, C₃-C₆- Cycloalkyl oder C₁-C₆-Alkoxy steht,
m für 0 - 2 steht,
R⁴ für Wasserstoff oder für die Gruppe -COR⁸, NO₂, Trimethylsilanyl (TMS), tert.-Butyl-dimethylsilanyl (TBDMS), tert.-Butyl-diphenylsilanyl (TBDPS), Triethylsilanyl (TES) oder -SO₂R⁷ steht oder für gegebenenfalls ein- oder mehrfach, gleich oder verschieden mit Hydroxy, Halogen, C₁-C₆-Alkoxy, C₁-C₆-Alkylthio, Cyano, C₃-C₁₀-Cycloalkyl, C₁-C₆- Hydroxyalkyl, C₂-C₆-Alkenyl, C₂-C₆-Alkinyl, C₁-C₆-Alkoxy-C₁- C₆-Alkyl, C₁-C₆-Alkoxy-C₁-C₆-Alkoxy-C₁-C₆-Alkyl oder mit der Gruppe -CONR⁹R¹⁰, -COR⁸, -CF₃, -OCF₃ oder -NR⁹R¹⁰ substituiertes C₁-C₁₀-Alkyl, C₃-C₁₀-Cycloalkyl, steht,
R⁵ für gegebenenfalls ein- oder mehrfach, gleich oder verschieden mit Hydroxy, C₁-C₆-Alkoxy, C₃-C₁₀-Cycloalkyl, Halogen oder der Gruppe -NR⁹R¹⁰ substituiertes C₁-C₁₀-Alkyl, C₂-C₆-Alkenyl, C₂-C₆-Alkinyl oder C₃-C₁₀-Cycloalkyl steht,
oder R⁴ und R⁵ gemeinsam einen C₅-C₁₀-Cycloalkylring der Gruppe 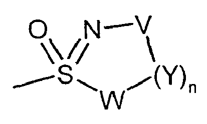 bilden kann, wobei
V, W und Y jeweils unabhängig voneinander für gegebenenfalls ein- oder mehrfach, gleich oder verschieden mit Hydroxy, C₁-C₁₀-Alkyl, C₁-C₁₀-Alkoxy oder -NR⁹R¹⁰ substituiertes -CH₂- steht, wobei C₁-C₁₀-Alkyl oder C₁-C₁₀-Alkoxy ebenfalls ein oder mehrfach, gleich oder verschieden mit Hydroxy, -NR⁹R¹⁰ oder C₁-C₁₀- Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
R⁶ für einen Heteroaryl oder einen C₃-C₁₀-Cycloalkyl-Ring, der gegebenenfalls ein oder mehrere Heteroatome enthalten kann und gegebenenfalls ein- oder mehrfach, gleich oder verschieden mit Hydroxy, C₁-C₆-Alkyl, C₁-C₆-Alkoxy oder Halogen substituiert sein kann, steht,
R⁷ für gegebenenfalls ein- oder mehrfach, gleich oder verschieden mit Halogen, Hydroxy, C₁-C₆-Alkyl, C₁-C₆-Alkoxy oder mit der Gruppe Trimethylsilanyl (TMS) oder -NR⁹R¹⁰ substituiertes C₁-C₁₀-Alkyl oder Aryl steht,
R⁸ für Wasserstoff, C₁-C₆-Alkyl , Hydroxy, C₁-C₆-Alkoxy, C₁-C₆- Alkylthio, Benzoxy oder -NR⁹R¹⁰ steht,
R⁹ und R¹⁰ jeweils unabhängig voneinander für Wasserstoff, C₁-C₆-Alkyl, C₁-C₆-Alkoxy, Hydroxy, Hydroxy-C₁-C₆-Alkyl, Dihydroxy-C₁- C₆-Alkyl, Phenyl, Heteroaryl oder für die Gruppe - (CH₂)ₙNR⁹R¹⁰, -CNHNH₂ oder -NR⁹R¹⁰ stehen,
oder R⁹ und R¹⁰ gemeinsam einen C₃-C₁₀-Cycloalkyl-Ring bilden, der gegebenenfalls durch ein- oder mehrere Stickstoff, Sauerstoff und/ oder Schwefel-Atome unterbrochen sein kann und/ oder durch ein oder mehrere -C(O)- Gruppen im Ring unterbrochen sein kann und/ oder gegebenenfalls ein oder mehrere mögliche Doppelbindungen im Ring enthalten sein können, steht und
n für 1 - 6 steht,
bedeuten, sowie deren Isomeren, Diastereomeren, Enantiomeren und/oder Salze.

4. Verbindungen der allgemeinen Formel (I) gemäß einem der vorgehenden Ansprüche, in der
Q für Phenyl steht,
R¹ für Wasserstoff, Halogen, CN, NO₂ oder CF₃ steht,
R² für gegebenenfalls ein- oder mehrfach, gleich oder verschieden mit Hydroxy, Halogen, C₁-C₆-Alkyl, C₁-C₆-Alkoxy, C₂-C₆-Alkinyl oder mit der Gruppe -COR⁸ substituiertes C₁- C₁₀-Alkyl, C₂-C₁₀-Alkinyl, Aryl oder Heteroaryl steht,
X für Sauerstoff, Schwefel oder für die Gruppe -NH- steht,
R³ für Wasserstoff, Halogen, Hydroxy oder gegebenenfalls ein- oder mehrfach mit Halogen oder Hydroxy substituiertes C₁- C₆-Alkyl oder C₁-C₆-Alkoxy steht,
m für 0 - 2 steht,
R⁴ für Wasserstoff oder für die Gruppe NO₂, -CO-R⁸, -SO₂R⁷ oder für gegebenenfalls ein- oder mehrfach, gleich oder verschieden mit Halogen oder Hydroxy substituiertes C₁-C₁₀- Alkyl steht,
R⁵ für gegebenenfalls ein- oder mehrfach, gleich oder verschieden mit Hydroxy oder C₃-C₁₀-Cycloalkyl substituiertes C₁-C₁₀-Alkyl oder C₃-C₁₀-Cycloalkyl steht,
oder R⁴ und R⁵ gemeinsam einen C₅-C₁₀-Cycloalkylring der Gruppe 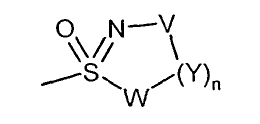 bilden kann, wobei
V, W und Y jeweils unabhängig voneinander für gegebenenfalls ein- oder mehrfach, gleich oder verschieden mit Hydroxy, C₁-C₁₀-Alkyl, C₁-C₁₀-Alkoxy oder -NR⁹R¹⁰ substituiertes -CH₂- steht, wobei C₁-C₁₀-Alkyl oder C₁-C₁₀-Alkoxy ebenfalls ein oder mehrfach, gleich oder verschieden mit Hydroxy, -NR⁹R¹⁰ oder C₁-C₁₀- Alkoxy substituiert sein kann und/ oder durch ein oder mehrere -C(O)- Gruppen im Ring unterbrochen sein kann und/ oder gegebenenfalls ein oder mehrere Doppelbindungen im Ring enthalten sein können, steht,
R⁷ für gegebenenfalls ein- oder mehrfach, gleich oder verschieden mit der Gruppe Trimethylsilanyl (TMS) substituiertes C₁-C₁₀-Alkyl oder steht,
R⁸ für Wasserstoff, C₁-C₆-Alkyl , C₁-C₆-Alkoxy oder C₃-C₆- Cycloalkyl, das gegebenenfalls ein- oder mehrfach mit C₁-C₆- Alkyl substituiert sein kann, steht,
n für 1 steht,
bedeuten, sowie deren Isomeren, Diastereomeren, Enantiomeren und/oder Salze.

5. Verbindungen der allgemeinen Formel (I) gemäß einem der vorgehenden Ansprüche, in der
Q für Phenyl steht,
R¹ für Wasserstoff oder Halogen steht,
R² für gegebenenfalls ein- oder mehrfach, gleich oder verschieden mit Hydroxy, Halogen, C₁-C₆-Alkyl, C₁-C₆-Alkoxy, C₂-C₆-Alkinyl oder mit der Gruppe -COR⁸ substituiertes C₁- C₁₀-Alkyl, C₂-C₁₀-Alkinyl oder Aryl steht,
X für Sauerstoff, Schwefel oder für die Gruppe -NH- steht,
R³ für Wasserstoff, Halogen oder gegebenenfalls ein- oder mehrfach mit Halogen substituiertes C₁-C₆-Alkyl oder C₁-C₆- Alkoxy steht,
m für 0 - 2 steht,
R⁴ für Wasserstoff oder für die Gruppe NO₂, -CO-R⁸, -SO₂R⁷ oder für C₁-C₁₀-Alkyl steht,
R⁵ für gegebenenfalls ein- oder mehrfach, gleich oder verschieden mit Hydroxy oder C₃-C₁₀-Cycloalkyl substituiertes C₁-C₁₀-Alkyl oder C₃-C₁₀-Cycloalkyl steht,
R⁷ für gegebenenfalls ein- oder mehrfach, gleich oder verschieden mit der Gruppe Trimethylsilanyl (TMS) substituiertes C₁-C₁₀-Alkyl oder steht,
R⁸ für Wasserstoff, C₁-C₆-Alkyl , C₁-C₆-Alkoxy oder C₃-C₆- Cycloalkyl, das gegebenenfalls ein- oder mehrfach mit C₁-C₆- Alkyl substituiert sein kann, steht,
bedeuten, sowie deren Isomeren, Diastereomeren, Enantiomeren und/oder Salze.

6. Verbindung der allgeimeinen Formel (I) gemäß einem der vorgehenden Ansprüchen, in der
Q für Phenyl steht,
R¹ für Wasserstoff oder Halogen steht ,
R² für gegebenenfalls ein- oder mehrfach, gleich oder verschieden mit Hydroxy, Halogen, Methyl, Methoxy, Ethinyl oder mit der Gruppe -COH oder -COCH₃ substituiertes C₁- C₁₀-Alkyl, C₂-C₁₀-Alkinyl oder Aryl steht,
X für Sauerstoff, Schwefel oder für die Gruppe -NH- steht,
R³ für Wasserstoff, Halogen, Methyl, Methoxy oder -CF₃ steht,
m für 0 - 2 steht,
R⁴ für Wasserstoff, Methyl oder für die Gruppe NO₂, -COOC₂H₅ oder -SO₂-C₂H₄-Si(CH₃) steht,
R⁵ für Methyl, Ethyl, Cyclopropyl, Cyclopentyl, -(CH₂)- Cyclopropyl oder Hydroxyethyl steht,
bedeuten, sowie deren Isomeren, Diastereomeren, Enantiomeren und/oder Salze.

7. Verwendung der Verbindung der allgemeinen Formel (IIa) oder (IIb) 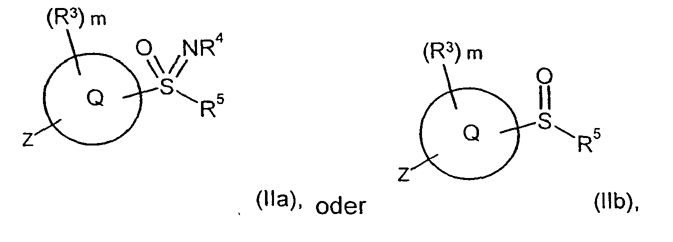 in der Z für -NH₂ oder NO₂ steht und m, R³, R⁴ und R⁵ die in der allgemeinen Formel (I) angegebenen Bedeutungen haben, sowie deren Isomeren, Diastereomeren, Enantiomeren und/oder Salze als Zwischenprodukte zur Herstellung der Verbindung der allgemeinen Formel (I).

8. Verwendung der Verbindung der allgemeinen Formel (IIa) oder (IIb), gemäß Anspruch 7, **dadurch gekennzeichnet, dass**
m für 0 -2 steht,
R³ für Halogen oder gegebenenfalls ein- oder mehrfach mit Halogen substituiertem C₁-C₁₀-Alkyl oder C₁-C₁₀-Alkoxy steht,
R⁴ für Wasserstoff oder für die Gruppe NO₂, -SO₂-R⁷, -CO- R⁸ oder für C₁-C₁₀-Alkyl steht, wobei R⁷ und R⁸ die in der allgemeinen Formel (I) angegebene Bedeutung hat, und
R⁵ für gegebenenfalls ein- oder mehrfach mit Halogen, Hydroxy substituiertem C₁-C₁₀-Alkyl oder C₃-C₆-Cycloalkyl steht.

9. Verwendung der Verbindung der allgemeinen Formel (IIIa), (IIIb) oder (IIIc) 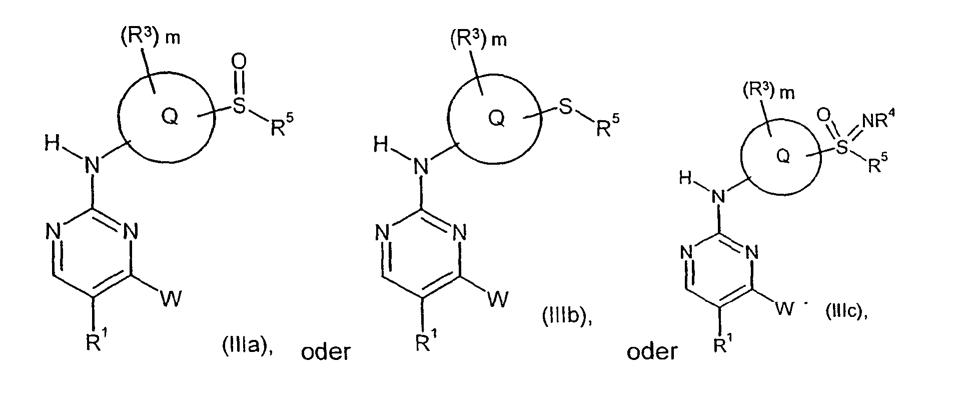 in der W für Halogen, Hydroxy oder X-R² steht und R¹, R², R³, R⁵, m und X die in der allgemeinen Formel (I) angegebenen Bedeutungen haben, sowie deren Isomeren, Diastereomeren, Enantiomeren und/oder Salze als Zwischenprodukte zur Herstellung der Verbindung der allgemeinen Formel (I).

10. Verwendung der Verbindung der allgemeinen Formel (IIIa), (IIIb) oder (IIIc), gemäß Anspruch 9, **dadurch gekennzeichnet, dass**
R¹ für Halogen steht,
X für -NH- steht,
R² für gegebenenfalls ein oder mehrfach mit Hydroxy substituiertes C₁-C₁₀-Alkyl steht,
m für 0 steht und
R⁵ für C₁-C₁₀-Alkyl steht.

11. Verwendung der Verbdindungen der allgemeinen Formel (IV), 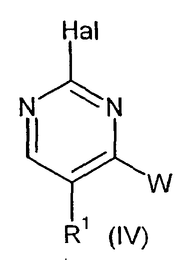 in der
Hal für Halogen, Y für Halogen, Hydroxy oder X-R² steht und R¹, R² und X die in der allgemeinen Formel (I) angegebenen Bedeutungen haben, sowie deren Isomeren, Diastereomeren, Enantiomeren und/oder Salze als Zwischenprodukte zur Herstellung der Verbindung der allgemeinen Formel (I).

12. Verwendung der Verbindung der allgemeinen Formel (IV) gemäß Anspruch 11, in der
X für Sauerstoff, Schwefel oder -NH- steht,
R¹ für Halogen steht,
R² für gegebenenfalls mit Hydroxy, C₁-C₆-Alkoxy oder mit der Gruppe -CO- R⁸ substituiertes C₁-C₁₀-Alkyl oder C₂-C₁₀- Alkinyl steht, wobei R⁸ die in der allgemeinen Formel (I) angegebene Bedeutung hat.

13. Pharmazeutisches Mittel umfassend eine Verbindung der allgemeinen Formel I gemäß mindestens einem der Ansprüche 1 bis 6.

14. Verbindung gemäß einem der Ansprüche 1 bis 6 zur Verwendung als Arzneimittel.

15. Verwendung der Verbindungen der allgemeinen Formel I, gemäß den Ansprüchen 1 bis 6, zur Herstellung eines Arzneimittels zur Behandlung von Krebs, Angiofribroma, Arthritis, Augenerkrankungen, Autoimmunerkrankungen, Chemotherapeutika-induzierter Alopezie und Mukositis, Crohn-Krankheit, Endometriose, fibrotische Erkrankungen, Hämangioma, kardiovaskulären Erkrankungen, infektiösen Erkrankungen, nephrologischen Erkrankungen, chronischen und akuten neurodegenerativen Erkrankungen, sowie von Verletzungen des Nervengewebes, viralen Infektionen, zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, zur Unterstützung der narbenfreien Wundheilung, bei Altersflecken und bei Kontaktdermatitis.

16. Verwendung gemäß Anspruch 15 , **dadurch gekennzeichnet, dass**
unter Krebs solide Tumoren, Tumor- oder Metastasenwachstum, Kaposis Sarkom, Morbus Hodgkin und Leukämie,
unter Arthritis, rheumatoide Arthritis, unter Augenerkrankungen, diabetische Retinopathie, Neovaskulares Glaukom,
unter Autoimmunerkrankungen Psoriasis, Alopezie und Multiple Sklerose,
unter fibrotische Erkrankungen, Leberzirrhose, mesangialzellproliferative Erkrankungen, Arteriosklerose,
unter infektiösen Erkrankungen durch unizelluläre Parasiten hervorgerufene Erkrankungen,
unter kardiovaskulären Erkrankungen Stenosen, wie z. B. Stent-induzierte Restenose, Arteriosklerosen und Restenosen,
unter nephrologischen Erkrankungen Glomerulonephritis, diabetische Nephropatie, maligne Nephrosklerose, thrombische mikroangiopatische Syndrome, Transplantationsabstoßungen und Glomerulopathie,
unter chronisch neurodegenerativen Erkrankungen Huntington's Erkrankung, amyotrophe Lateralsklerose, Parkinsonsche Erkrankung, AIDS Dementia und Alzheimer'sche Erkrankung,
unter akut neurodegenerativen Erkrankungen Ischämien des Gehirns und Neurotraumata,
und unter viralen Infektionen Cytomegalus-Infektionen, Herpes, Hepatitis B oder C, und HIV Erkrankungen zu verstehen sind.

17. Arzneimittel enthaltend mindestens eine Verbindung gemäß mindestens einem der Ansprüche 1 bis 6 enthalten.

18. Arzneimittel gemäß Anspruch 17 für die enterale, parenterale oder orale Applikation.

## Claims

1. Compounds of general formula (I) 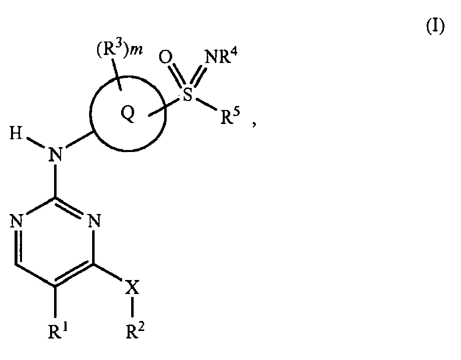 in which
Q stands for the group 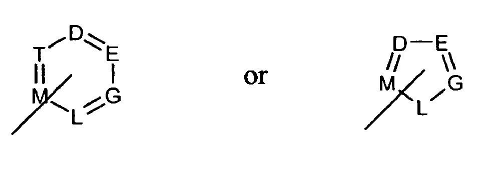
D, E, G, L, M and T, in each case independently of one another, stand for carbon, oxygen, nitrogen or sulfur,
R¹ stands for hydrogen, halogen, C₁-C₆-alkyl, CF₃, CN, nitro, or for the group -COR⁸ or -O-C₁-C₆-alkyl,
R² stands for hydrogen, or C₁-C₁₀-alkyl, C₂-C₁₀- alkenyl, C₂-C₁₀-alkinyl, C₃-C₁₀-cycloalkyl, aryl or heteroaryl that is optionally substituted in one or more places, in the same way or differently, with hydroxy, halogen, C₁-C₆-alkoxy, amino, cyano, C₁-C₆-alkyl, -NH-(CH₂)ₙ-C₃-C₁₀-cycloalkyl, -C₃-C₁₀- cycloalkyl, C₁-C₆-hydroxyalkyl, C₂-C₆-alkenyl, C₂- C₆-alkinyl, C₁-C₆-alkoxy-C₁-C₆-alkyl, C₁-C₆-alkoxy- C₁-C₆-alkoxy-C₁-C₆-alkyl, -NHC₁-C₆-alkyl, -N(C₁-C₆- alkyl)₂, C₁-C₆-alkanoyl, -CONR⁹R¹⁰, -COR⁸, C₁-C₆- alkylOAc, carboxy, aryl, heteroaryl, -(CH₂)ₙ-aryl, -(CH₂)ₙ-heteroaryl, phenyl-(CH₂)ₙ-R⁸, -(CH₂)ₙPO₃(R⁸)₂ or with the group -R⁶ or -NR⁹R¹⁰, and the phenyl, C₃-C₁₀-cycloalkyl, aryl, heteroaryl, -(CH₂)ₙ-aryl and (CH₂)ₙ-heteroaryl itself optionally can be substituted in one or more places, in the same way or differently, with halogen, hydroxy, C₁-C₆-alkyl, C₁-C₆-alkoxy, or with the group -CF₃ or -OCF₃, and the ring of C₃-C₁₀-cycloalkyl and the C₁-C₁₀-alkyl optionally can be interrupted by one or more nitrogen, oxygen and/or sulfur atoms and/or can be interrupted by one or more -C(O) groups in the ring and/or optionally one or more possible double bonds can be contained in the ring,
X stands for oxygen, sulfur, or for the group -NH- or -N(C₁-C₃-alkyl),
or X and R² together form a C₃-C₁₀-cycloalkyl ring, which optionally can contain one or more heteroatoms and optionally can be substituted in one or more places, in the same way or differently, with hydroxy, C₁-C₆-alkyl, C₁-C₆-alkoxy, halogen or the group -NR⁹R¹⁰,
R³ stands for hydrogen, hydroxy, halogen, CF₃, OCF₃ or for the group -NR⁹R¹⁰, or for C₁-C₆-alkyl, C₃-C₆- cycloalkyl or C₁-C₆-alkoxy that is optionally substituted in one or more places, in the same way or differently, with halogen, hydroxy, C₁-C₆-alkoxy or the group -NR⁹R¹⁰,
m stands for 0 - 4,
R⁴ stands for hydrogen or for the group -COR⁸, NO₂, trimethylsilanyl (TMS), tert-butyl-dimethylsilanyl (TBDMS), tert-butyl-diphenylsilanyl (TBDPS), triethylsilanyl (TES) or -SO₂R⁷ or for C₁-C₁₀-alkyl or C₃-C₁₀-cycloalkyl that is optionally substituted in one or more places, in the same way or differently, with hydroxy, halogen, C₁-C₆-alkoxy, C₁-C₆-alkylthio, cyano, C₃-C₁₀-cycloalkyl, C₁-C₆- hydroxyalkyl, C₂-C₆-alkenyl, C₂-C₆-alkinyl, C₁-C₆- alkoxy-C₁-C₆-alkyl, C₁-C₆-alkoxy-C₁-C₆-alkoxy-C₁-C₆- alkyl or with the group -CONR⁹R¹⁰, -COR⁸, -CF₃, -OCF₃ or -NR⁹R¹⁰,
R⁵ stands for C₁-C₁₀-alkyl, C₂-C₆-alkenyl, C₂-C₆-alkinyl or C₃-C₁₀-cycloalkyl that is optionally substituted in one or more places, in the same way or differently, with hydroxy, C₁-C₆-alkoxy, C₃-C₁₀- cycloalkyl, halogen or the group -NR⁹R¹⁰,
or R⁴ and R⁵ together can form a C₅-C₁₀-cycloalkyl ring of the group 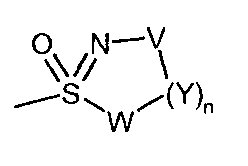
whereby V, W and Y, in each case independently of one another, stand for -CH₂- that is optionally substituted in one or more places, in the same way or differently, with hydroxy, C₁-C₁₀-alkyl, C₁-C₁₀- alkoxy or -NR⁹R¹⁰, whereby C₁-C₁₀-alkyl or C₁-C₁₀- alkoxy also can be substituted in one or more places, in the same way or differently, with hydroxy, -NR⁹R¹⁰ or C₁-C₁₀-alkoxy and/or can be interrupted by one or more -C(O)- groups in the ring, and/or optionally one or more double bonds can be contained in the ring,
R⁶ stands for a heteroaryl or a C₃-C₁₀-cycloalkyl ring, which optionally can contain one or more heteroatoms and optionally can be substituted in one or more places, in the same way or differently, with hydroxy, C₁-C₆-alkyl, C₁-C₆-alkoxy or halogen,
R⁷ stands for C₁-C₁₀-alkyl or aryl that is optionally substituted in one or more places, in the same way or differently, with halogen, hydroxy, C₁-C₆-alkyl, C₁-C₆-alkoxy or with the group trimethylsilanyl (TMS) or -NR⁹R¹⁰,
R⁸ stands for hydrogen, C₁-C₆-alkyl, hydroxy, C₁-C₆- alkoxy, C₁-C₆-alkylthio, benzoxy or -NR⁹R¹⁰,
R⁹ and R¹⁰, in each case independently of one another, stand for hydrogen, C₁-C₆-alkyl, C₁-C₆-alkoxy, hydroxy, hydroxy-C₁-C₆-alkyl, dihydroxy-C₁-C₆-alkyl, phenyl, heteroaryl or for the group -(CH₂)ₙNR⁹R¹⁰, -CNHNH₂ or -NR⁹R¹⁰,
or R⁹ and R¹⁰ together form a C₃-C₁₀-cycloalkyl ring that optionally can be interrupted by one or more nitrogen, oxygen and/or sulfur atoms and/or can be interrupted by one or more -C(O)- groups in the ring and/or optionally one or more possible double bonds can be contained in the ring, and
n stands for 1 - 6,
as well as their isomers, diastereomers, enantiomers and/or salts.

2. Compounds of general formula (I) according to claim 1, in which
Q stands for aryl,
R¹ stands for hydrogen, halogen, C₁-C₆-alkyl, CF₃, CN, nitro, or for the group -COR⁸ or -O-C₁-C₆-alkyl,
R² stands for hydrogen or C₁-C₁₀-alkyl, C₂-C₁₀-alkenyl, C₂-C₁₀-alkinyl, C₃-C₁₀-cycloalkyl, aryl or heteroaryl that is optionally substituted in one or more places, in the same way or differently, with hydroxy, halogen, C₁-C₆-alkoxy, amino, cyano, C₁-C₆-alkyl, -NH-(CH₂)ₙ-C₃-C₁₀-cycloalkyl, -C₃-C₁₀- cycloalkyl, C₁-C₆-hydroxyalkyl, C₂-C₆-alkenyl, C₂- C₆-alkinyl, C₁-C₆-alkoxy-C₁-C₆-alkyl, C₁-C₆-alkoxy- C₁-C₆-alkoxy-C₁-C₆-alkyl, -NHC₁-C₆-alkyl, -N (C₁-C₆- alkyl)₂, C₁-C₆-alkanoyl, -CONR⁹R¹⁰, -COR⁸, C₁- C₆alkylOAc, carboxy, aryl, heteroaryl, -(CH₂)ₙ- aryl, -(CH₂)ₙ-heteroaryl, phenyl-(CH₂)ₙ-R⁸, -(CH₂)ₙ- PO₃(R⁸)₂ or with the group -R⁶ or -NR⁹R¹⁰, and the phenyl, C₃-C₁₀-cycloalkyl, aryl, heteroaryl, - (CH₂)ₙ- aryl and -(CH₂)ₙ-heteroaryl itself optionally can be substituted in one or more places, in the same way or differently, with halogen, hydroxy, C₁-C₆- alkyl, C₁-C₆-alkoxy or with the group -CF₃ or -OCF₃, and the ring of the C₃-C₁₀-cycloalkyl and the C₁- C₁₀-alkyl optionally can be interrupted by one or more nitrogen, oxygen and/or sulfur atoms and/or can be interrupted by one or more -C(O)- groups in the ring and/or optionally one or more possible double bonds can be contained in the ring,
X stands for oxygen, sulfur, or for the group -NH-, or -N(C₁-C₃-alkyl),
or X and R² together form a C₃-C₁₀-cycloalkyl ring, which optionally can contain one or more heteroatoms and optionally can be substituted in one or more places, in the same way or differently, with hydroxy, C₁-C₆-alkyl, C₁-C₆-alkoxy, halogen or the group -NR⁹R¹⁰,
R³ stands for hydrogen, hydroxy, halogen, CF₃, OCF₃ or for the group -NR⁹R¹⁰, or for C₁-C₆-alkyl, C₃-C₆- cycloalkyl or C₁-C₆-alkoxy that is optionally substituted in one or more places, in the same way or differently, with halogen, hydroxy, C₁-C₆-alkoxy or the group -NR⁹R¹⁰,
m stands for 0 - 4,
R⁴ stands for hydrogen or for the group -COR⁸, NO₂, trimethylsilanyl (TMS), tert-butyl-dimethylsilanyl (TBDMS), tert-butyl-diphenylsilanyl (TBDPS), triethylsilanyl (TES) or for -SO₂R⁷ or for C₁-C₁₀- alkyl or C₃-C₁₀cycloalkyl that is optionally substituted in one or more places, in the same way or differently, with hydroxy, halogen, C₁-C₆- alkoxy, C₁-C₆-alkylthio, cyano, C₃-C₁₀-cycloalkyl, C₁-C₆-hydroxyalkyl, C₂-C₆-alkenyl, C₁-C₆-alkinyl, C₁- C₆-alkoxy-C₁-C₆-alkyl, C₁-C₆-alkoxy-C₁-C₆-alkoxy-C₁- C₆-alkyl or with the group -CONR⁹R¹⁰, -COR⁸, -CF₃, -OCF₃ or -NR⁹R¹⁰
R⁵ stands for C₁-C₁₀-alkyl, C₂-C₆-alkenyl, C₂-C₆-alkinyl or C₃-C₁₀-cycloalkyl that is optionally substituted in one or more places, in the same way or differently, with hydroxy, C₁-C₆-alkoxy, C₃-C₁₀- cycloalkyl, halogen, or the group -NR⁹R¹⁰,
or R⁴ and R⁵ together can form a C₅-C₁₀-cycloalkyl ring of the group 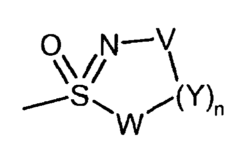
whereby V, W and Y, in each case independently of one another, stands for -CH2-, which is optionally substituted in one or more places, in the same way or differently, with hydroxy, C₁-C₁₀-alkyl, C₁-C₁₀- alkoxy or -NR⁹R¹⁰, whereby C₁-C₁₀-alkyl or C₁-C₁₀- alkoxy also can be substituted in one or more places, in the same way or differently, with hydroxy, -NR⁹R¹⁰ or C₁-C₁₀-alkoxy and/or can be interrupted by one or more -C(O)- groups in the ring, and/or optionally one or more double bonds can be contained in the ring,
R⁶ stands for a heteroaryl or a C₃-C₁₀-cycloalkyl ring, which optionally can contain one or more heteroatoms and optionally can be substituted in one or more places, in the same way or differently, with hydroxy, C₁-C₆-alkyl, C₁-C₆-alkoxy or halogen,
R⁷ stands for C₁-C₁₀-alkyl or aryl that is optionally substituted in one or more places, in the same way or differently, with halogen, hydroxy, C₁-C₆-alkyl, or C₁-C₆-alkoxy or with the group trimethylsilanyl (TMS) or -NR⁹R¹⁰,
R⁸ stands for hydrogen, C₁-C₆-alkyl, hydroxy, C₁-C₆- alkoxy, C₁-C₆-alkylthio, benzoxy or -NR⁹R¹⁰,
R⁹ and R¹⁰, in each case independently of one another, stand for hydrogen, C₁-C₆-alkyl, C₁-C₆-alkoxy, hydroxy, hydroxy-C₁-C₆-alkyl, dihydroxy-C₁-C₆-alkyl, phenyl, heteroaryl, or for the group -(CH₂)ₙNR⁹R¹⁰, -CNHNH₂ or -NR⁹R¹⁰,
or R⁹ and R¹⁰ together form a C₃-C₁₀-cycloalkyl ring that optionally can be interrupted by one or more nitrogen, oxygen and/or sulfur atoms and/or can be interrupted by one or more -C(O)- groups in the ring and/or optionally one or more possible double bonds can be contained in the ring, and
n stands for 1 - 6,
as well as their isomers, diastereomers, enantiomers and/or salts.

3. Compounds of general formula (I) according to claim 1 or 2, in which
Q stands for phenyl,
R¹ stands for hydrogen, halogen, C₁-C₆-alkyl, CF₃, CN, nitro or for the group -COR⁸ or -O-C₁-C₆-alkyl,
R² stands for hydrogen or for C₁-C₁₀-alkyl, C₂-C₁₀- alkenyl, C₂-C₁₀-alkinyl, C₃-C₁₀-cycloalkyl, aryl or heteroaryl that is optionally substituted in one or more places, in the same way or differently, with hydroxy, halogen, C₁-C₆-alkoxy, amino, cyano, C₁-C₆-alkyl, -NH-(CH₂)ₙC₃-C₁₀-cycloalkyl, -C₃-C₁₀- cycloalkyl, C₁-C₆-hydroxyalkyl, C₂-C₆-alkenyl, C₂- C₆-alkinyl, C₁-C₆-alkoxy-C₁-C₆-alkyl, C₁-C₆-alkoxy- C₁-C₆-alkoxy-C₁-C₆-alkyl, -NHC₁-C₆-alkyl, -N(C₁-C₆- alkyl)₂, C₁-C₆-alkanoyl, -CONR⁹R¹⁰, -COR⁸, C₁-C₆- alkylOAc, carboxy, aryl, heteroaryl, -(CH₂)ₙ-aryl, -(CH₂)ₙ-heteroaryl, phenyl-(CH₂)ₙ-R⁸, -(CH₂)PO₃(R⁸)₂ or with the group -R⁶ or -NR⁹R¹⁰, and phenyl, C₃-C₁₀- cycloalkyl, aryl, heteroaryl, -(CH₂)ₙ-aryl and -(CH₂)ₙ-heteroaryl itself optionally can be substituted in one or more places, in the same way or differently, with halogen, hydroxy, C₁-C₆-alkyl, C₁-C₆-alkoxy, or with the group -CF₃ or -OCF₃, and the ring of C₃-C₁₀-cycloalkyl and the C₁-C₁₀-alkyl optionally can be interrupted by one or more nitrogen, oxygen and/or sulfur atoms, and/or can be interrupted by one or more -C(O)- groups in the ring, and/or optionally one or more possible double bonds can be contained in the ring,
X stands for oxygen, sulfur, or for the group -NH- or -N(C₁-C₃-alkyl)-,
or X and R² together form a C₃-C₁₀-cycloalkyl ring, which optionally can contain one or more heteroatoms, and optionally can be substituted in one or more places with hydroxy, C₁-C₆-alkyl, C₁-C₆-alkoxy, halogen or the group -NR⁹R¹⁰,
R³ stands for hydrogen, hydroxy, halogen, CF₃, OCF₃ or for the group -NR⁹R¹⁰ or for C₁-C₆-alkyl, C₃-C₆- cycloalkyl or C₁-C₆-alkoxy that is optionally substituted in one or more places, in the same way or differently, with halogen, hydroxy, C₁-C₆-alkoxy or the group -NR⁹R¹⁰,
m stands for 0 - 2,
R⁴ stands for hydrogen or for the group -COR⁸, NO₂, trimethylsilanyl (TMS), tert-butyl-dimethylsilanyl (TBDMS), tert-butyl-diphenylsilanyl (TBDPS), triethylsilanyl (TES) or -SO₂R⁷, or for C₁-C₁₀-alkyl or C₃-C₁₀-cycloalkyl that is optionally substituted in one or more places, in the same way or differently, with hydroxy, halogen, C₁-C₆-alkoxy, C₁-C₆-alkylthio, cyano, C₃-C₁₀-cycloalkyl, C₁-C₆- hydroxyalkyl, C₂-C₆-alkenyl, C₂-C₆-alkinyl, C₁-C₆- alkoxy-C₁-C₆-alkyl, C₁-C₆-alkoxy-C₁-C₆-alkoxy-C₁-C₆- alkyl or with the group -CONR⁹R¹⁰, -COR⁸, -CF₃, -OCF₃ or -NR⁹R¹⁰,
R⁵ stands for C₁-C₁₀-alkyl, C₂-C₆-alkenyl, C₂-C₆-alkinyl or C₃-C₁₀-cycloalkyl that is optionally substituted in one or more places, in the same way or differently, with hydroxy, C₁-C₆-alkoxy, C₃-C₁₀- cycloalkyl, halogen or the group -NR⁹R¹⁰,
or R₄ and R⁵ together can form a C₅-C₁₀-cycloalkyl ring of the group 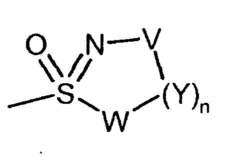
whereby V, W, and Y, in each case independently of one another, stand for -CH2- that is optionally substituted in one or more places, in the same way or differently, with hydroxy, C₁-C₁₀-alkyl, C₁-C₁₀- alkoxy or -NR⁹R¹⁰, whereby C₁-C₁₀-alkyl or C₁-C₁₀- alkoxy also can be substituted in one or more places, in the same way or differently, with hydroxy, -NR⁹R¹⁰ or C₁-C₁₀-alkoxy, and/or can be interrupted by one or more -C(O)- groups in the ring, and/or optionally one or more double bonds can be contained in the ring,
R⁶ stands for a heteroaryl or a C₃-C₁₀-cycloalkyl ring, which optionally can contain one or more heteroatoms, and optionally can be substituted in one or more places, in the same way or differently, with hydroxy, C₁-C₆-alkyl, C₁-C₆-alkoxy or halogen,
R⁷ stands for C₁-C₁₀-alkyl or aryl that is optionally substituted in one or more places, in the same way or differently, with halogen, hydroxy, C₁-C₆-alkyl, C₁-C₆-alkoxy or with the group trimethylsilanyl (TMS) or -NR⁹R¹⁰,
R⁸ stands for hydrogen, C₁-C₆-alkyl, hydroxy, C₁-C₆- alkoxy, C₁-C₆-alkylthio, benzoxy or -NR⁹R¹⁰,
R⁹ and R¹⁰, in each case independently of one another, stand for hydrogen, C₁-C₆-alkyl, C₁-C₆-alkoxy, hydroxy, hydroxy-C₁-C₆-alkyl, dihydroxy-C₁-C₆-alkyl, phenyl, heteroaryl or for the group -(CH₂)ₙNR⁹R¹⁰, -CNHNH₂ or -NR⁹R¹⁰,
or R⁹ and R¹⁰ together form a C₃-C₁₀-cycloalkyl ring, which optionally can be interrupted by one or more nitrogen, oxygen and/or sulfur atoms and/or can be interrupted by one or more -C(O)- groups in the ring and/or optionally one or more possible double bonds can be contained in the ring, and
n stands for 1 - 6,
as well as their isomers, diastereomers, enantiomers and/or salts.

4. Compounds of general formula (I) according to one of the preceding claims, in which
Q stands for phenyl,
R¹ stands for hydrogen, halogen, CN, NO₂ or CF₃,
R² stands for C₁-C₁₀-alkyl, C₂-C₁₀-alkinyl, aryl or heteroaryl that is optionally substituted in one or more places, in the same way or differently, with hydroxy, halogen, C₁-C₆-alkyl, C₁-C₆-alkoxy, C₂-C₆-alkinyl or with the group -COR⁸,
X stands for oxygen, sulfur or for the group -NH-,
R³ stands for hydrogen, halogen, hydroxy or C₁-C₆- alkyl or C₁-C₆-alkoxy that is optionally substituted in one or more places with halogen or hydroxy,
m stands for 0 - 2,
R⁴ stands for hydrogen or for the group NO₂, -CO-R⁸, -SO₂R⁷ or for C₁-C₁₀-alkyl that is optionally substituted in one or more places, in the same way or differently, with halogen or hydroxy,
R⁵ stands for C₁-C₁₀-alkyl or C₃-C₁₀-cycloalkyl that is optionally substituted in one or more places, in the same way or differently, with hydroxy or C₃- C₁₀-cycloalkyl,
or R⁴ and R⁵ together can form a C₅-C₁₀-cycloalkyl ring of the group 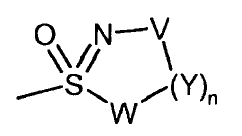
whereby V, W and Y, in each case independently of one another, stand for -CH₂- that is optionally substituted in one or more places, in the same way or differently, with hydroxy, C₁-C₁₀-alkyl, C₁-C₁₀- alkoxy or -NR⁹R¹⁰, whereby C₁-C₁₀-alkyl or C₁-C₁₀- alkoxy also can be substituted in one or more places, in the same way or differently, with hydroxy, -NR⁹R¹⁰ or C₁-C₁₀-alkoxy and/or can be interrupted by one or more -C(O)- groups in the ring and/or optionally one or more double bonds can be contained in the ring,
R⁷ stands for C₁-C₁₀-alkyl that is optionally substituted in one or more places in the same way or differently, with the group trimethylsilanyl (TMS),
R⁸ stands for hydrogen, C₁-C₆-alkyl, C₁-C₆-alkoxy or C₃-C₆-cycloalkyl, which optionally can be substituted in one or more places with C₁-C₆-alkyl,
n stands for 1,
as well as their isomers, diastereomers, enantiomers and/or salts.

5. Compounds of general formula (I) according to one of the preceding claims, in which
Q stands for phenyl,
R¹ stands for hydrogen or halogen,
R² stands for C₁-C₁₀-alkyl, C₂-C₁₀-alkinyl or aryl that is optionally substituted in one or more places, in the same way or differently, with hydroxy, halogen, C₁-C₆-alkyl, C₁-C₆-alkoxy, C₂-C₆-alkinyl or with the group -COR⁸,
X stands for oxygen, sulfur or for the group -NH-,
R³ stands for hydrogen, halogen or C₁-C₆-alkyl or C₁- C₆-alkoxy that is optionally substituted in one or more places with halogen,
m stands for 0 - 2,
R⁴ stands for hydrogen or for the group NO₂, -CO-R⁸, -SO₂R⁷ or for C₁-C₁₀-alkyl,
R⁵ stands for C₁-C₁₀-alkyl or C₃-C₁₀-cycloalkyl that is optionally substituted in one or more places, in the same way or differently, with hydroxy or C₃- C₁₀-cycloalkyl,
R⁷ stands for C₁-C₁₀-alkyl that is optionally substituted in one or more places in the same way or differently, with the group trimethylsilanyl (TMS),
R⁸ stands for hydrogen, C₁-C₆-alkyl, C₁-C₆-alkoxy or C₃-C₆-cycloalkyl, which optionally can be substituted in one or more places with C₁-C₆-alkyl,
as well as their isomers, diastereomers, enantiomers and/or salts.

6. Compound of general formula (I) according to one of the preceding claims, in which
Q stands for phenyl,
R¹ stands for hydrogen or halogen,
R² stands for C₁-C₁₀-alkyl, C₂-C₁₀-alkinyl or aryl that is optionally substituted in one or more places, in the same way or differently, with hydroxy, halogen, methyl, methoxy, ethinyl or with the group -COH or -COCH₃,
X stands for oxygen, sulfur or for the group -NH-,
R³ stands for hydrogen, halogen, methyl, methoxy or -CF₃,
m stands for 0 - 2,
R⁴ stands for hydrogen, methyl or for the group NO₂, -COOC₂H₅ or -SO₂C₂H₄-Si (CH₃)₃,
R⁵ stands for methyl, ethyl, cyclopropyl, cyclopentyl, -(CH₂)-cyclopropyl or hydroxyethyl,
as well as their isomers, diastereomers, enantiomers and/or salts.

7. Use of the compound of general formula (IIa) or (IIb) 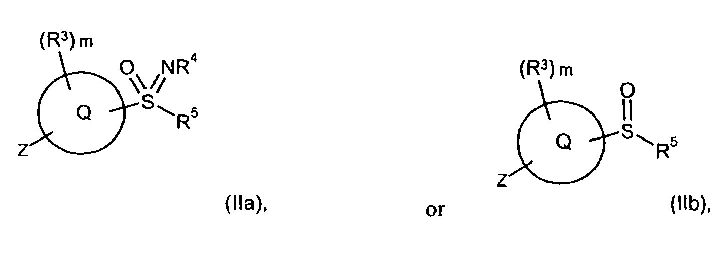 in which Z stands for -NH₂ or NO₂, and m, R³, R⁴ and R⁵ have the meanings that are indicated in general formula (I), as well as their isomers, diastereomers, enantiomers and/or salts as intermediate products for the production of the compound of general formula (I).

8. Use of the compound of general formula (IIa) or (IIb), according to claim 7, **characterized in that**
m stands for 0 - 2,
R³ stands for halogen, or for C₁-C₁₀-alkyl or C₁-C₁₀- alkoxy that is optionally substituted in one or more places with halogen,
R⁴ stands for hydrogen or for the group NO₂, -SO₂-R⁷, -CO-R⁸ or for C₁-C₁₀-alkyl, whereby R⁷ and R⁸ have the meaning that is indicated in general formula (I), and
R⁵ stands for C₁-C₁₀-alkyl or C₃-C₆-cycloalkyl that is optionally substituted in one or more places with halogen or hydroxy.

9. Use of the compound of general formula (IIIa), (IIIb) or (IIIc), 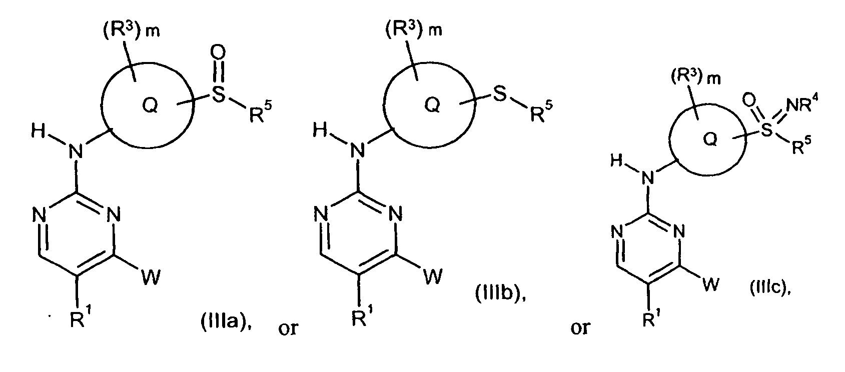 in which W stands for halogen, hydroxy or X-R², and R¹, R², R³, R⁵, m and X have the meanings that are indicated in general formula (I), as well as their isomers, diastereomers, enantiomers, and/or salts as intermediate products for the production of the compound of general formula (I).

10. Use of the compound of general formula (IIIa), (IIIb) or (IIIc), according to claim 9, **characterized in that**
R¹ stands for halogen,
X stands for -NH-,
R² stands for C₁-C₁₀-alkyl that is optionally substituted in one or more places with hydroxy,
m stands for 0, and
R⁵ stands for C₁-C₁₀-alkyl.

11. Use of the compounds of general formula (IV), 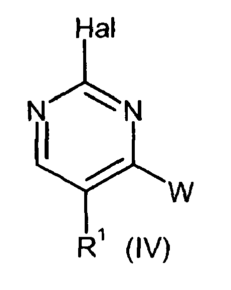 in which
Hal stands for halogen, Y stands for halogen, hydroxy or X-R², and R¹, R² and X have the meanings that are indicated in general formula (I), as well as their isomers, diastereomers, enantiomers and/or salts as intermediate products for the production of the compound of general formula (I).

12. Use of the compound of general formula (IV) according to claim 11, in which
X stands for oxygen, sulfur or -NH-,
R¹ stands for halogen,
R² stands for C₁-C₁₀-alkyl or C₂-C₁₀-alkinyl that is optionally substituted with hydroxy, C₁-C₆-alkoxy or with the group -CO-R⁸, whereby R⁸ has the meaning that is indicated in general formula (I).

13. Pharmaceutical agent that comprises a compound of general formula I according to at least one of claims 1 to 6.

14. Compound according to one of claims 1 to 6 used as a pharmaceutical agent.

15. Use of the compounds of general formula I, according to claims 1 to 6, for the production of a pharmaceutical agent for treating cancer, angiofibroma, arthritis, eye diseases, auto-immune diseases, chemotherapy agent-induced alopecia and mucositis, Crohn's disease, endometriosis, fibrotic diseases, hemangioma, cardiovascular diseases, infectious diseases, nephrological diseases, chronic and acute neurodegenerative diseases, as well as injuries to the nerve tissue, and viral infections, for inhibiting the reocclusion of vessels after balloon catheter treatment, in vascular prosthetics or after mechanical devices are used to keep vessels open, such as, e.g., stents, and as immunosuppressive agents, and for supporting scar-free wound healing, in senile keratosis and in contact dermatitis.

16. Use according to claim 15, **characterized in that** cancer is defined as solid tumors, tumor or metastatic growth, Kaposi's sarcoma, Hodgkin's disease, and leukemia; arthritis is defined as rheumatoid arthritis; eye diseases are defined as diabetic retinopathy, and neovascular glaucoma; auto-immune diseases are defined as psoriasis, alopecia and multiple sclerosis; fibrotic diseases are defined as cirrhosis of the liver, mesangial cell proliferative diseases, and arteriosclerosis; infectious diseases are defined as diseases that are caused by unicellular parasites; cardiovascular diseases are defined as stenoses, such as, e.g., stent-induced restenosis, arterioscleroses and restenoses; nephrological diseases are defined as glomerulonephritis, diabetic nephropathy, malignant nephrosclerosis, thrombic microangiopathic syndrome, transplant rejections and glomerulopathy; chronic neurodegenerative diseases are defined as Huntington's disease, amyotrophic lateral sclerosis, Parkinson's disease, AIDS dementia and Alzheimer's disease; acute neurodegenerative diseases are defined as ischemias of the brain and neurotraumas; and viral infections are defined as cytomegalic infections, herpes, hepatitis B or C, and HIV diseases.

17. Pharmaceutical agents that contain at least one compound according to at least one of claims 1 to 6.

18. Pharmaceutical agent according to claim 17 for enteral, parenteral or oral administration.

## Revendications

1. Composés de formule générale (I) 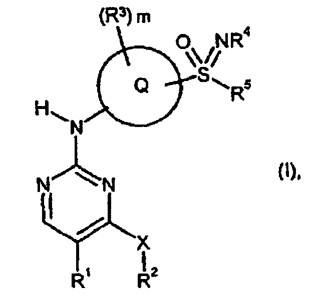 où
Q représente le groupe 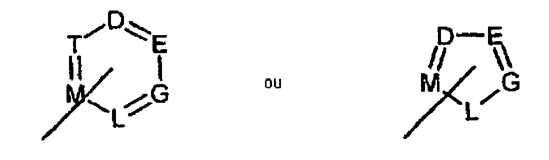
D, E, G, L, M et T représentent à chaque fois indépendamment l'un de l'autre carbone, oxygène, azote ou soufre,
R¹ représente hydrogène, halogène, C₁-C₆-alkyle, CF₃, CN, nitro ou le groupe -COR⁸ ou -O-C₁-C₆-alkyle,
R² représente hydrogène ou un groupe C₁-C₁₀-alkyle, C₂-C₁₀-alcényle, C₂-C₁₀-alcynyle, C₃-C₁₀-cycloalkyle, aryle ou hétéroaryle le cas échéant monosubstitué ou polysubstitué, de manière identique ou différente, par hydroxy, halogène, C₁-C₆-alcoxy, amino, cyano, C₁-C₆-alkyle, -NH-(CH₂)ₙ-C₃-C₁₀- cycloalkyle, -C₃-C₁₀-cycloalkyle, C₁-C₆- hydroxyalkyle, C₂-C₆-alcényle, C₂-C₆-alcynyle, C₁- C₆-alcoxy-C₁-C₆-alkyle, C₁-C₆-alcoxy-C₁-C₆-alcoxy-C₁- C₆-alkyle, -NHC₁-C₆-alkyle, -N(C₁-C₆-alkyle)₂, C₁-C₆- alcanoyle, -CONR⁹R¹⁰, -COR⁸, C₁-C₆-alkylOAc, carboxy, aryle, hétéroaryle, -(CH₂)ₙ-aryle, -(CH₂)ₙ-hétéroaryle, phényl-(CH₂)ₙ-R⁸, -(CH₂)ₙPO₃(R⁸)₂ ou par le groupe -R⁶ ou -NR⁹R¹⁰ et les groupes phényle, C₃-C₁₀-cycloalkyle, aryle, hétéroaryle, -(CH₂)ₙ-aryle et -(CH₂)ₙ-hétéroaryle eux-mêmes peuvent le cas échéant être monosubstitués ou polysubstitués, de manière identique ou différente, par halogène, hydroxy, C₁-C₆-alkyle, C₁-C₆-alcoxy, ou par le groupe -CF₃ ou -OCF₃, et le cycle du groupe C₃-C₁₀-cycloalkyle et le groupe C₁-C₁₀-alkyle peuvent le cas échéant être interrompus par un ou plusieurs atomes d'azote, d'oxygène et/ou de soufre et/ou par un ou plusieurs groupes -C(O)- dans le cycle et/ou le cas échéant une ou plusieurs doubles liaisons possibles peuvent être contenues dans le cycle,
X représente oxygène, soufre ou le groupe -NH- ou -N(C₁-C₃-alkyl)-,
ou X et R² forment ensemble un cycle C₃-C₁₀-cycloalkyle qui peut le cas échéant contenir un ou plusieurs hétéroatomes, et qui peut le cas échéant être monosubstitué ou polysubstitué, de manière identique ou différente, par hydroxy, C₁-C₆-alkyle, C₁-C₆-alcoxy, halogène ou le groupe -NR⁹R¹⁰,
R³ représente hydrogène, hydroxy, halogène, CF₃, OCF₃ ou le groupe -NR⁹R¹⁰ ou un groupe C₁-C₆-alkyle, C₃- C₆-cycloalkyle ou C₁-C₆-alcoxy le cas échéant monosubstitué ou polysubstitué, de manière identique ou différente, par halogène, hydroxy, C₁-C₆-alcoxy ou le groupe -NR⁹R¹⁰,
m vaut 0-4,
R⁴ représente hydrogène ou le groupe -COR⁸, NO₂, triméthylsilanyle (TMS), tert- butyldiméthylsilanyle (TBDMS), tert- butyldiphénylsilanyle (TBDPS), triéthylsilanyle (TES) ou -SO₂R⁷ ou un groupe C₁-C₁₀-alkyle ou C₃-C₁₀- cycloalkyle le cas échéant monosubstitué ou polysubstitué de manière identique ou différente par hydroxy, halogène, C₁-C₆-alcoxy, C₁-C₆- alkylthio, cyano, C₃-C₁₀-cycloalkyle, C₁-C₆- hydroxyalkyle, C₂-C₆-alcényle, C₂-C₆-alcynyle, C₁- C₆-alcoxy-C₁-C₆-alkyle, C₁-C₆-alcoxy-C₁-C₆-alcoxy-C₁- C₆-alkyle ou par le groupe -CONR⁹R¹⁰, -COR⁸, -CF₃, -OCF₃ ou -NR⁹R¹⁰,
R⁵ représente un groupe C₁-C₁₀-alkyle, C₂-C₆-alcényle, C₂-C₆-alcynyle ou C₃-C₁₀-cycloalkyle le cas échéant monosubstitué ou polysubstitué, de manière identique ou différente, par hydroxy, C₁-C₆-alcoxy, C₃-C₁₀-cycloalkyle, halogène ou par le groupe -NR⁹R¹⁰,
ou R⁴ et R⁵ peuvent former ensemble un cycle C₅-C₁₀- cycloalkyle de formule 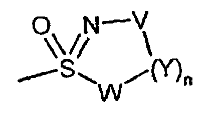
où V, W et Y représentent, à chaque fois indépendamment l'un de l'autre, -CH₂- le cas échéant monosubstitué ou polysubstitué de manière identique ou différente par hydroxy, C₁-C₁₀-alkyle, C₁-C₁₀-alcoxy ou -NR⁹R¹⁰, où le groupe C₁-C₁₀-alkyle ou C₁-C₁₀-alcoxy peut également être monosubstitué ou polysubstitué, de manière identique ou différente, par hydroxy, -NR⁹R¹⁰ ou C₁-C₁₀-alcoxy et/ou qui peut être interrompu par un ou plusieurs groupes -C(O)- dans le cycle et/ou le cas échéant une ou plusieurs doubles liaisons peuvent être contenues dans le cycle,
R⁶ représente un groupe hétéroaryle ou un cycle C₃- C₁₀-cycloalkyle qui peut le cas échéant contenir un ou plusieurs hétéroatomes, et qui peut le cas échéant être monosubstitué ou polysubstitué, de manière identique ou différente, par hydroxy, C₁- C₆-alkyle, C₁-C₆-alcoxy ou halogène,
R⁷ représente un groupe C₁-C₁₀-alkyle ou aryle le cas échéant monosubstitué ou polysubstitué, de manière identique ou différente, par halogène, hydroxy, C₁-C₆-alkyle, C₁-C₆-alcoxy ou par le groupe triméthylsilanyle (TMS) ou -NR⁹R¹⁰,
R⁸ représente hydrogène, C₁-C₆-alkyle, hydroxy, C₁-C₆- alcoxy, C₁-C₆-alkylthio, benzoxy ou -NR⁹R¹⁰,
R⁹ et R¹⁰ représentent, à chaque fois indépendamment l'un de l'autre, hydrogène, C₁-C₆-alkyle, C₁-C₆- alcoxy, hydroxy, hydroxy-C₁-C₆-alkyle, dihydroxy- C₁-C₆-alkyle, phényle, hétéroaryle ou le groupe -(CH₂)ₙNR⁹R¹⁰, -CNHNH₂ ou -NR⁹R¹⁰,
ou R⁹ et R¹⁰ forment ensemble un cycle C₃-C₁₀-cycloalkyle, qui peut le cas échéant être interrompu par un ou plusieurs atomes d'azote, d'oxygène et/ou de soufre et/ou être interrompu par un ou plusieurs groupes -C(O)- dans le cycle et/ou une ou plusieurs doubles liaisons possibles peuvent être contenues dans le cycle et
n vaut 1-6,
ainsi que leurs isomères, diastéréo-isomères, énantiomères et/ou sels.

2. Composés de formule générale (I) selon la revendication 1, où
Q représente aryle,
R¹ représente hydrogène, halogène, C₁-C₆-alkyle, CF₃, CN, nitro ou le groupe -COR⁸ ou -O-C₁-C₆-alkyle,
R² représente hydrogène ou un groupe C₁-C₁₀-alkyle, C₂-C₁₀-alcényle, C₂-C₁₀-alcynyle, C₃-C₁₀-cycloalkyle, aryle ou hétéroaryle le cas échéant monosubstitué ou polysubstitué, de manière identique ou différente, par hydroxy, halogène, C₁-C₆-alcoxy, amino, cyano, C₁-C₆-alkyle, -NH-(CH₂)ₙ-C₃-C₁₀- cycloalkyle, -C₃-C₁₀-cycloalkyle, C₁-C₆- hydroxyalkyle, C₂-C₆-alcényle, C₂-C₆-alcynyle, C₁- C₆-alcoxy-C₁-C₆-alkyle, C₁-C₆-alcoxy-C₁-C₆-alcoxy-C₁- C₆-alkyle, -NHC₁-C₆-alkyle, -N(C₁-C₆-alkyle)₂, C₁-C₆- alcanoyle, -CONR⁹R¹⁰, -COR⁸, C₁-C₆-alkylOAc, carboxy, aryle, hétéroaryle, -(CH₂)ₙ-aryle, -(CH₂)ₙ-hétéroaryle, phényl-(CH₂)ₙ-R⁸, -(CH₂)ₙPO₃(R⁸)₂ ou par le groupe -R⁶ ou -NR⁹R¹⁰ et les groupes phényle, C₃-C₁₀-cycloalkyle, aryle, hétéroaryle, -(CH₂)ₙ-aryle et -(CH₂)ₙ-hétéroaryle eux-mêmes peuvent le cas échéant être monosubstitués ou polysubstitués, de manière identique ou différente, par halogène, hydroxy, C₁-C₆-alkyle, C₁-C₆-alcoxy, ou par le groupe -CF₃ ou -OCF₃, et le cycle du groupe C₃-C₁₀-cycloalkyle et le groupe C₁-C₁₀-alkyle peuvent le cas échéant être interrompus par un ou plusieurs atomes d'azote, d'oxygène et/ou de soufre et/ou par un ou plusieurs groupes -C(O)- dans le cycle et/ou le cas échéant une ou plusieurs doubles liaisons possibles peuvent être contenues dans le cycle,
X représente oxygène, soufre ou le groupe -NH-, -N(C₁-C₃-alkyle)-,
ou X et R² forment ensemble un cycle C₃-C₁₀-cycloalkyle qui peut le cas échéant contenir un ou plusieurs hétéroatomes, et qui peut le cas échéant être monosubstitué ou polysubstitué, de manière identique ou différente, par hydroxy, C₁-C₆-alkyle, C₁-C₆-alcoxy, halogène ou le groupe -NR⁹R¹⁰,
R³ représente hydrogène, hydroxy, halogène, CF₃, OCF₃ ou le groupe -NR⁹R¹⁰ ou un groupe C₁-C₆-alkyle, C₃- C₆-cycloalkyle ou C₁-C₆-alcoxy le cas échéant monosubstitué ou polysubstitué, de manière identique ou différente, par halogène, hydroxy, C₁-C₆-alcoxy ou le groupe -NR⁹R¹⁰,
m vaut 0-4,
R⁴ représente hydrogène ou le groupe -COR⁸, NO₂, triméthylsilanyle (TMS), tert- butyldiméthylsilanyle (TBDMS), tert- butyldiphénylsilanyle (TBDPS), triéthylsilanyle (TES) ou -SO₂R⁷ ou un groupe C₁-C₁₀-alkyle, C₃-C₁₀- cycloalkyle le cas échéant monosubstitué ou polysubstitué de manière identique ou différente par hydroxy, halogène, C₁-C₆-alcoxy, C₁-C₆- alkylthio, cyano, C₃-C₁₀-cycloalkyle, C₁-C₆- hydroxyalkyle, C₂-C₆-alcényle, C₂-C₆-alcynyle, C₁- C₆-alcoxy-C₁-C₆-alkyle, C₁-C₆-alcoxy-C₁-C₆-alcoxy-C₁- C₆-alkyle ou le groupe -CONR⁹R¹⁰, -COR⁸, -CF₃, -OCF₃ ou -NR⁹R¹⁰,
R⁵ représente un groupe C₁-C₁₀-alkyle, C₂-C₆-alcényle, C₂-C₆-alcynyle ou C₃-C₁₀-cycloalkyle le cas échéant monosubstitué ou polysubstitué, de manière identique ou différente, par hydroxy, C₁-C₆-alcoxy, C₃-C₁₀-cycloalkyle, halogène ou par le groupe -NR⁹R¹⁰,
ou R⁴ et R⁵ peuvent former ensemble un cycle C₅-C₁₀- cycloalkyle de formule 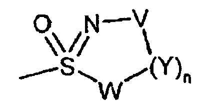
où V, W et Y représentent, à chaque fois indépendamment l'un de l'autre, -CH₂- le cas échéant monosubstitué ou polysubstitué de manière identique ou différente par hydroxy, C₁-C₁₀-alkyle, C₁-C₁₀-alcoxy ou -NR⁹R¹⁰, où le groupe C₁-C₁₀-alkyle ou C₁-C₁₀-alcoxy peut également être monosubstitué ou polysubstitué, de manière identique ou différente, par hydroxy, -NR⁹R¹⁰ ou C₁-C₁₀-alcoxy et/ou qui peut être interrompu par un ou plusieurs groupes -C(O)- dans le cycle et/ou le cas échéant une ou plusieurs doubles liaisons peuvent être contenues dans le cycle,
R⁶ représente un groupe hétéroaryle ou un cycle C₃- C₁₀-cycloalkyle qui peut le cas échéant contenir un ou plusieurs hétéroatomes, et qui peut le cas échéant être monosubstitué ou polysubstitué, de manière identique ou différente, par hydroxy, C₁- C₆-alkyle, C₁-C₆-alcoxy ou halogène,
R⁷ représente un groupe C₁-C₁₀-alkyle ou aryle le cas échéant monosubstitué ou polysubstitué, de manière identique ou différente, par halogène, hydroxy, C₁-C₆-alkyle, C₁-C₆-alcoxy ou par le groupe triméthylsilanyle (TMS) ou -NR⁹R¹⁰,
R⁸ représente hydrogène, C₁-C₆-alkyle, hydroxy, C₁-C₆- alcoxy, C₁-C₆-alkylthio, benzoxy ou -NR⁹R¹⁰,
R⁹ et R¹⁰ représentent, à chaque fois indépendamment l'un de l'autre, hydrogène, C₁-C₆-alkyle, C₁-C₆- alcoxy, hydroxy, hydroxy-C₁-C₆-alkyle, dihydroxy- C₁-C₆-alkyle, phényle, hétéroaryle ou le groupe -(CH₂)ₙNR⁹R¹⁰, -CNHNH₂ ou -NR⁹R¹⁰,
ou R⁹ et R¹⁰ forment ensemble un cycle C₃-C₁₀-cycloalkyle, qui peut le cas échéant être interrompu par un ou plusieurs atomes d'azote, d'oxygène et/ou de soufre et/ou être interrompu par un ou plusieurs groupes -C(0)- dans le cycle et/ou une ou plusieurs doubles liaisons possibles peuvent être contenues dans le cycle et
n vaut 1-6,
ainsi que leurs isomères, diastéréo-isomères, énantiomères et/ou sels.

3. Composés de formule générale (I) selon la revendication 1 ou 2, où
Q représente phényle,
R¹ représente hydrogène, halogène, C₁-C₆-alkyle, CF₃, CN, nitro ou le groupe -COR⁸ ou -O-C₁-C₆-alkyle,
R² représente hydrogène ou un groupe C₁-C₁₀-alkyle, C₂-C₁₀-alcényle, C₂-C₁₀-alcynyle, C₃-C₁₀-cycloalkyle, aryle ou hétéroaryle le cas échéant monosubstitué ou polysubstitué, de manière identique ou différente, par hydroxy, halogène, C₁-C₆-alcoxy, amino, cyano, C₁-C₆-alkyle, -NH-(CH₂)ₙ-C₃-C₁₀- cycloalkyle, -C₃-C₁₀-cycloalkyle, C₁-C₆- hydroxyalkyle, C₂-C₆-alcényle, C₂-C₆-alcynyle, C₁- C₆-alcoxy-C₁-C₆-alkyle, C₁-C₆-alcoxy-C₁-C₆-alcoxy-C₁- C₆-alkyle, -NHC₁-C₆-alkyle, -N(C₁-C₆-alkyle)₂, C₁-C₆- alcanoyle, -CONR⁹R¹⁰, -COR⁸, C₁-C₆-alkylOAc, carboxy, aryle, hétéroaryle, -(CH₂)ₙ-aryle, -(CH₂)ₙ-hétéroaryle, phényl-(CH₂)ₙ-R⁸, -(CH₂)ₙPO₃(R⁸)₂ ou par le groupe -R⁶ ou -NR⁹R¹⁰ et les groupes phényle, C₃-C₁₀-cycloalkyle, aryle, hétéroaryle, -(CH₂)ₙ-aryle et -(CH₂)ₙ-hétéroaryle eux-mêmes peuvent le cas échéant être monosubstitués ou polysubstitués, de manière identique ou différente par halogène, hydroxy, C₁- C₆-alkyle, C₁-C₆-alcoxy, ou par le groupe -CF₃ ou -OCF₃, et le cycle du groupe C₃-C₁₀-cycloalkyle et le groupe C₁-C₁₀-alkyle peuvent le cas échéant être interrompus par un ou plusieurs atomes d'azote, d'oxygène et/ou de soufre et/ou par un ou plusieurs groupes -C(O)- dans le cycle et/ou le cas échéant une ou plusieurs doubles liaisons possibles peuvent être contenues dans le cycle,
X représente oxygène, soufre ou le groupe -NH-, -N(C₁-C₃-alkyl)-,
ou X et R² forment ensemble un cycle C₃-C₁₀-cycloalkyle qui peut le cas échéant contenir un ou plusieurs hétéroatomes, et qui peut le cas échéant être monosubstitué ou polysubstitué par hydroxy, C₁-C₆- alkyle, C₁-C₆-alcoxy, halogène ou le groupe -NR⁹R¹⁰,
R³ représente hydrogène, hydroxy, halogène, CF₃, OCF₃ ou le groupe -NR⁹R¹⁰ ou un groupe C₁-C₆-alkyle, C₃- C₆-cycloalkyle ou C₁-C₆-alcoxy le cas échéant monosubstitué ou polysubstitué, de manière identique ou différente, par halogène, hydroxy, C₁-C₆-alcoxy ou le groupe -NR⁹R¹⁰,
m vaut 0-2,
R⁴ représente hydrogène ou le groupe -COR⁸, NO₂, triméthylsilanyle (TMS), tert- butyldiméthylsilanyle (TBDMS), tert- butyldiphénylsilanyle (TBDPS), triéthylsilanyle (TES) ou -SO₂R⁷ ou un groupe C₁-C₁₀-alkyle, C₃-C₁₀- cycloalkyle le cas échéant monosubstitué ou polysubstitué de manière identique ou différente par hydroxy, halogène, C₁-C₆-alcoxy, C₁-C₆- alkylthio, cyano, C₃-C₁₀-cycloalkyle, C₁-C₆- hydroxyalkyle, C₂-C₆-alcényle, C₂-C₆-alcynyle, C₁- C₆-alcoxy-C₁-C₆-alkyle, C₁-C₆-alcoxy-C₁-C₆-alcoxy-C₁- C₆-alkyle ou par le groupe -CONR⁹R¹⁰, -COR⁸, -CF₃, -OCF₃ ou -NR⁹R¹⁰,
R⁵ représente un groupe C₁-C₁₀-alkyle, C₂-C₆-alcényle, C₂-C₆-alcynyle ou C₃-C₁₀-cycloalkyle le cas échéant monosubstitué ou polysubstitué, de manière identique ou différente, par hydroxy, C₁-C₆-alcoxy, C₃-C₁₀-cycloalkyle, halogène ou par le groupe -NR⁹R¹⁰,
ou R⁴ et R⁵ peuvent former ensemble un cycle C₅-C₁₀- cycloalkyle de formule 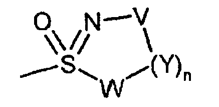
où V, W et Y représentent, à chaque fois indépendamment l'un de l'autre, -CH₂- le cas échéant monosubstitué ou polysubstitué de manière identique ou différente par hydroxy, C₁-C₁₀-alkyle, C₁-C₁₀-alcoxy ou -NR⁹R¹⁰, où le groupe C₁-C₁₀-alkyle ou C₁-C₁₀-alcoxy peut également être monosubstitué ou polysubstitué, de manière identique ou différente, par hydroxy, -NR⁹R¹⁰ ou C₁-C₁₀-alcoxy et/ou qui peut être interrompu par un ou plusieurs groupes -C(O)- dans le cycle et/ou le cas échéant une ou plusieurs doubles liaisons peuvent être contenues dans le cycle,
R⁶ représente un groupe hétéroaryle ou un cycle C₃- C₁₀-cycloalkyle qui peut le cas échéant contenir un ou plusieurs hétéroatomes, et qui peut le cas échéant être monosubstitué ou polysubstitué, de manière identique ou différente, par hydroxy, C₁- C₆-alkyle, C₁-C₆-alcoxy ou halogène,
R⁷ représente un groupe C₁-C₁₀-alkyle ou aryle le cas échéant monosubstitué ou polysubstitué, de manière identique ou différente, par halogène, hydroxy, C₁-C₆-alkyle, C₁-C₆-alcoxy ou par le groupe triméthylsilanyle (TMS) ou -NR⁹R¹⁰,
R⁸ représente hydrogène, C₁-C₆-alkyle, hydroxy, C₁-C₆- alcoxy, C₁-C₆-alkylthio, benzoxy ou -NR⁹R¹⁰,
R⁹ et R¹⁰ représentent, à chaque fois indépendamment l'un de l'autre, hydrogène, C₁-C₆-alkyle, C₁-C₆- alcoxy, hydroxy, hydroxy-C₁-C₆-alkyle, dihydroxy- C₁-C₆-alkyle, phényle, hétéroaryle ou le groupe -(CH₂)ₙNR⁹R¹⁰, -CNHNH₂ ou -NR⁹R¹⁰,
ou R⁹ et R¹⁰ forment ensemble un cycle C₃-C₁₀-cycloalkyle, qui peut le cas échéant être interrompu par un ou plusieurs atomes d'azote, d'oxygène et/ou de soufre et/ou être interrompu par un ou plusieurs groupes -C(0)- dans le cycle et/ou une ou plusieurs doubles liaisons possibles peuvent être contenues dans le cycle et
n vaut 1-6,
ainsi que leurs isomères, diastéréo-isomères, énantiomères et/ou sels.

4. Composés de formule générale (I) selon l'une quelconque des revendications précédentes, où
Q représente phényle,
R¹ représente hydrogène, halogène, CN, NO₂ ou CF₃,
R² représente un groupe C₁-C₁₀-alkyle, C₂-C₁₀-alcynyle, aryle ou hétéroaryle le cas échéant monosubstitué ou polysubstitué, de manière identique ou différente, par hydroxy, halogène, C₁-C₆-alkyle, C₁-C₆-alcoxy, C₂-C₆-alcynyle ou par le groupe -COR⁸,
X représente oxygène, soufre ou le groupe -NH-,
R³ représente hydrogène, halogène, hydroxy ou un groupe C₁-C₆-alkyle ou C₁-C₆-alcoxy le cas échéant monosubstitué ou polysubstitué par halogène ou hydroxy,
m vaut 0-2,
R⁴ représente hydrogène, le groupe NO₂, -CO-R⁸, -SO₂R⁷ ou le groupe C₁-C₁₀-alkyle le cas échéant monosubstitué ou polysubstitué, de manière identique ou différente, par halogène ou hydroxy,
R⁵ représente un groupe C₁-C₁₀-alkyle ou C₃-C₁₀- cycloalkyle le cas échéant monosubstitué ou polysubstitué, de manière identique ou différente, par hydroxy ou C₃-C₁₀-cycloalkyle,
ou R⁴ et R⁵ peuvent former ensemble un cycle C₅-C₁₀- cycloalkyle de formule 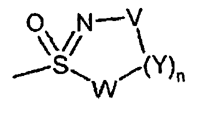
où V, W et Y représentent, à chaque fois indépendamment l'un de l'autre, -CH₂- le cas échéant monosubstitué ou polysubstitué de manière identique ou différente par hydroxy, C₁-C₁₀-alkyle, C₁-C₁₀-alcoxy ou -NR⁹R¹⁰, où le groupe C₁-C₁₀-alkyle ou C₁-C₁₀-alcoxy peut également être monosubstitué ou polysubstitué, de manière identique ou différente, par hydroxy, -NR⁹R¹⁰ ou C₁-C₁₀-alcoxy et/ou qui peut être interrompu par un ou plusieurs groupes -C(O)- dans le cycle et/ou le cas échéant une ou plusieurs doubles liaisons peuvent être contenues dans le cycle,
R⁷ représente un groupe C₁-C₁₀-alkyle le cas échéant monosubstitué ou polysubstitué, de manière identique ou différente, par le groupe triméthylsilanyle (TMS),
R⁸ représente hydrogène, C₁-C₆-alkyle, C₁-C₆-alcoxy ou C₃-C₆-cycloalkyle, qui peut le cas échéant être monosubstitué ou polysubstitué par C₁-C₆-alkyle,
n vaut 1,
ainsi que leurs isomères, diastéréo-isomères, énantiomères et/ou sels.

5. Composés de formule générale (I) selon l'une quelconque des revendications précédentes, où
Q représente phényle,
R¹ représente hydrogène ou halogène,
R² représente un groupe C₁-C₁₀-alkyle, C₂-C₁₀-alcynyle ou aryle le cas échéant monosubstitué ou polysubstitué, de manière identique ou différente, par hydroxy, halogène, C₁-C₆-alkyle, C₁-C₆-alcoxy, C₂-C₆-alcynyle ou par le groupe -COR⁸,
X représente oxygène, soufre ou le groupe -NH-,
R³ représente hydrogène, halogène ou un groupe C₁-C₆- alkyle ou C₁-C₆-alcoxy le cas échéant monosubstitué ou polysubstitué par halogène,
m vaut 0-2,
R⁴ représente hydrogène ou le groupe NO₂, -CO-R⁸, -SO₂R⁷ ou le groupe C₁-C₁₀-alkyle,
R⁵ représente un groupe C₁-C₁₀-alkyle ou C₃-C₁₀- cycloalkyle le cas échéant monosubstitué ou polysubstitué, de manière identique ou différente, par hydroxy ou C₃-C₁₀-cycloalkyle,
R⁷ représente un groupe C₁-C₁₀-alkyle le cas échéant monosubstitué ou polysubstitué, de manière identique ou différente, par le groupe triméthylsilanyle (TMS),
R⁸ représente hydrogène, C₁-C₆-alkyle, C₁-C₆-alcoxy ou C₃-C₆-cycloalkyle, qui peut le cas échéant être monosubstitué ou polysubstitué par C₁-C₆-alkyle,
ainsi que leurs isomères, diastéréo-isomères, énantiomères et/ou sels.

6. Composé de formule générale (I) selon l'une quelconque des revendications précédentes, où
Q représente phényle,
R¹ représente hydrogène ou halogène,
R² représente un groupe C₁-C₁₀-alkyle, C₂-C₁₀-alcynyle ou aryle le cas échéant monosubstitué ou polysubstitué de manière identique ou différente par hydroxy, halogène, méthyle, méthoxy, éthynyle ou par le groupe -COH ou -COCH₃,
X représente oxygène, soufre ou le groupe -NH-,
R³ représente hydrogène, halogène, méthyle, méthoxy ou CF₃,
m vaut 0-2,
R⁴ représente hydrogène, méthyle ou le groupe NO₂, -COOC₂H₅ ou -SO₂-C₂H₄-Si(CH₃)₃.
R⁵ représente méthyle, éthyle, cyclopropyle, cyclopentyle, -(CH₂)-cyclopropyle ou hydroxyéthyle,
ainsi que leurs isomères, diastéréo-isomères, énantiomères et/ou sels.

7. Utilisation du composé de formule générale (IIa) ou (IIb) 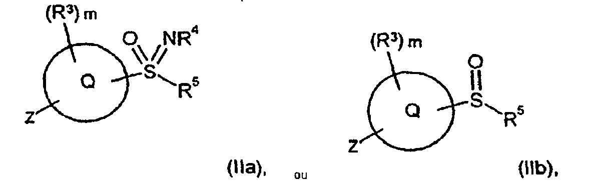 où Z représente -NH₂ ou NO₂ et m, R³, R⁴ et R⁵ présentent les significations indiquées dans la formule générale (I), ainsi que de ses isomères, diastéréo-isomères, énantiomères et/ou sels comme produits intermédiaires pour la préparation du composé de formule générale (I).

8. Utilisation du composé de formule générale (IIa) ou (IIb) selon la revendication 7, **caractérisée en ce que**
m vaut 0-2,
R³ représente halogène ou un groupe C₁-C₁₀-alkyle ou C₁-C₁₀-alcoxy le cas échéant monosubstitué ou polysubstitué par halogène,
R⁴ représente hydrogène ou le groupe NO₂, -SO₂-R⁷, -CO-R⁸ ou C₁-C₁₀-alkyle, où R⁷ et R⁸ présentent la signification indiquée dans la formule générale (I), et
R⁵ représente C₁-C₁₀-alkyle ou C₃-C₆-cycloalkyle le cas échéant monosubstitué ou polysubstitué par halogène, hydroxy.

9. Utilisation du composé de formule générale (IIIa), (IIIb) ou (IIIc) 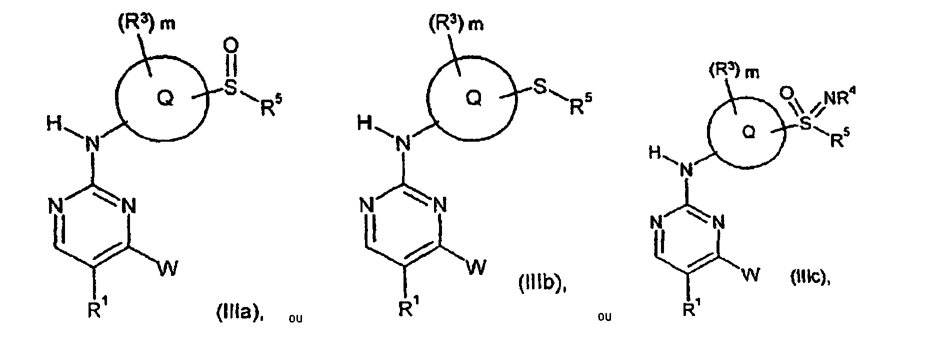 où W représente halogène, hydroxy ou X-R² et R¹, R², R³, R⁵, m et X présentent les significations indiquées dans la formule générale (I), ainsi que de ses isomères, diastéréo-isomères, énantiomères et/ou sels comme produits intermédiaires pour la préparation du composé de formule générale (I).

10. Utilisation du composé de formule générale (IIIa), (IIIb) ou (IIIc) selon la revendication 9, **caractérisée**
**en ce que**
R¹ représente halogène,
X représente -NH-,
R² représente C₁-C₁₀-alkyle le cas échéant monosubstitué ou polysubstitué par hydroxy,
m vaut 0 et
R⁵ représente alkyle en C₁-C₁₀.

11. Utilisation des composés de formule générale (IV) 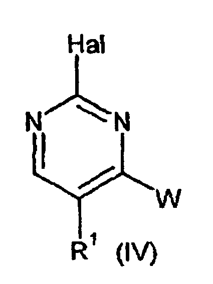 où
Hal représente halogène,
Y représente halogène, hydroxy ou X-R² et
R¹, R² et X présentent les significations indiquées dans la formule générale (I), ainsi que de leurs isomères, diastéréo-isomères, énantiomères et/ou sels comme produits intermédiaires pour la préparation du composé de formule générale (I).

12. Utilisation du composé de formule générale (IV) selon la revendication 11, où
X représente oxygène, soufre ou -NH-,
R¹ représente halogène,
R² représente un groupe C₁-C₁₀-alkyle ou C₂-C₁₀- alcynyle le cas échéant substitué par hydroxy, C₁- C₆-alcoxy ou par le groupe -CO-R⁸, où R⁸ présente la signification indiquée dans la formule générale (I).

13. Préparation pharmaceutique comprenant un composé de formule générale I selon au moins l'une quelconque des revendications 1 à 6.

14. Composé selon l'une quelconque des revendications 1 à 6 destiné à une utilisation comme médicament.

15. Utilisation des composés de formule générale I, selon les revendications 1 à 6, pour la préparation d'un médicament destiné au traitement du cancer, de l'angiofibrome, de l'arthrite, des maladies des yeux, de maladies auto-immunitaires, de l'alopécie et de la mucosite provoquée par les agents chimiothérapeutiques, de la maladie de Crohn, de l'endométriose, des maladies fibrotiques, de l'hémangiome, des maladies cardio-vasculaires, des maladies infectieuses, des maladies néphrologiques, des maladies de neurodégénérescence chroniques et aiguës, ainsi que de lésions du tissu nerveux, des infections virales, à l'inhibition de la réocclusion de vaisseaux sanguins après un traitement par un cathéter ballon, lors de la prothèse vasculaire ou après le placement de dispositifs mécaniques pour garder les vaisseaux ouverts, tels que par exemple les stents, comme agents immunosuppresseurs, pour soutenir la guérison de plaies sans cicatrices, lors de tâches de vieillissement et lors d'une dermatite de contact.

16. Utilisation selon la revendication 15, **caractérisée**
**en ce qu'**on entend
- par cancer des tumeurs solides, une croissance de tumeurs ou de métastases, le sarcome de Kaposi, la maladie de Hodgkin et la leucémie,
- par arthrite, la polyarthrite rhumatoïde, par maladies des yeux la rétinopathie diabétique, le glaucome néovasculaire,
- par maladies auto-immunitaires le psoriasis, l'alopécie et la sclérose en plaques, par maladies fibrotiques la cirrhose du foie, les maladies prolifératives de cellules mésangiales, l'artériosclérose,
- par maladies infectieuses des maladies provoquées par des parasites unicellulaires,
- par maladies cardio-vasculaires des sténoses, telles que par exemple les resténoses induites par les stents, les artérioscléroses et les resténoses,
- par maladies néphrologiques la glomérulonéphrite, la néphropathie diabétique, la néphrosclérose maligne, le syndrome de micro-angiopathique thrombique, les rejets de transplantations et la glomérulopathie,
- par maladies de neurodégénérescence chroniques la maladie d'Huntington, la sclérose latérale amyotrophique, la maladie de Parkinson, la démence associée au SIDA et la maladie d'Alzheimer,
- par maladies de neurodégénérescence aiguës les ischémies du cerveau et les neurotraumatismes,
- et par infections virales les infections par le cytomégalovirus, l'herpes, l'hépatite B ou C, et les maladies du VIH.

17. Médicament contenant au moins un composé selon au moins l'une quelconque des revendications 1 à 6.

18. Médicament selon la revendication 17 pour l'administration entérale, parentérale ou orale.
